(19) Europäisches Patentamt
European Patent Office
Office européen des brevets

(11) EP 4 596 537 A1

(12) # EUROPEAN PATENT APPLICATION
published in accordance with Art. 153(4) EPC

(43) Date of publication:
**06.08.2025 Bulletin 2025/32**

(21) Application number: **23872753.1**

(22) Date of filing: **28.07.2023**

(51) International Patent Classification (IPC):
***C07D 231/12** (2006.01)* ***A61K 31/415** (2006.01)*
***A61K 31/4439** (2006.01)* ***A61K 31/497** (2006.01)*
***A61K 31/506** (2006.01)* ***A61K 31/517** (2006.01)*
***A61K 31/4709** (2006.01)* ***A61K 31/5025** (2006.01)*
***A61K 31/427** (2006.01)* ***A61K 31/55** (2006.01)*

(52) Cooperative Patent Classification (CPC):
**A61K 31/415; A61K 31/427; A61K 31/4439; A61K 31/4709; A61K 31/497; A61K 31/5025; A61K 31/506; A61K 31/517; A61K 31/55; A61P 3/00; C07D 231/12**

(86) International application number:
**PCT/KR2023/011089**

(87) International publication number:
**WO 2024/071629 (04.04.2024 Gazette 2024/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.09.2022 KR 20220123775**

(71) Applicant: **JD Bioscience Inc.**
**Gwangju 61011 (KR)**

(72) Inventors:
- **PAGIRE, Suvarna Haushabhau**
  **Gwangju 61005 (KR)**
- **LEE, In Kyu**
  **Daegu 42107 (KR)**
- **JEON, Jae-Han**
  **Daegu 42006 (KR)**
- **PAGIRE, Suvarna Haushabhau**
  **Gwangju 61005 (KR)**
- **KIM, Pyeong Keun**
  **Gwangju 61005 (KR)**
- **OH, Chang Joo**
  **Daegu 41138 (KR)**
- **JEON, Yong Hyun**
  **Daegu 42178 (KR)**
- **SON, Kwang Hee**
  **Daegu 41069 (KR)**
- **AHN, Jin Hee**
  **Gwangju 61005 (KR)**

(74) Representative: **Regimbeau**
**20, rue de Chazelles**
**75847 Paris Cedex 17 (FR)**

(54) **NOVEL FLUORENE DERIVATIVE COMPOUND AND USE THEREOF**

(57) Provided are a novel fluorene derivative compound, and use thereof in preventing or treating metabolic diseases and/or cancer diseases through the action thereof as a PDK4 inhibitor.

EP 4 596 537 A1

[FIG. 1]

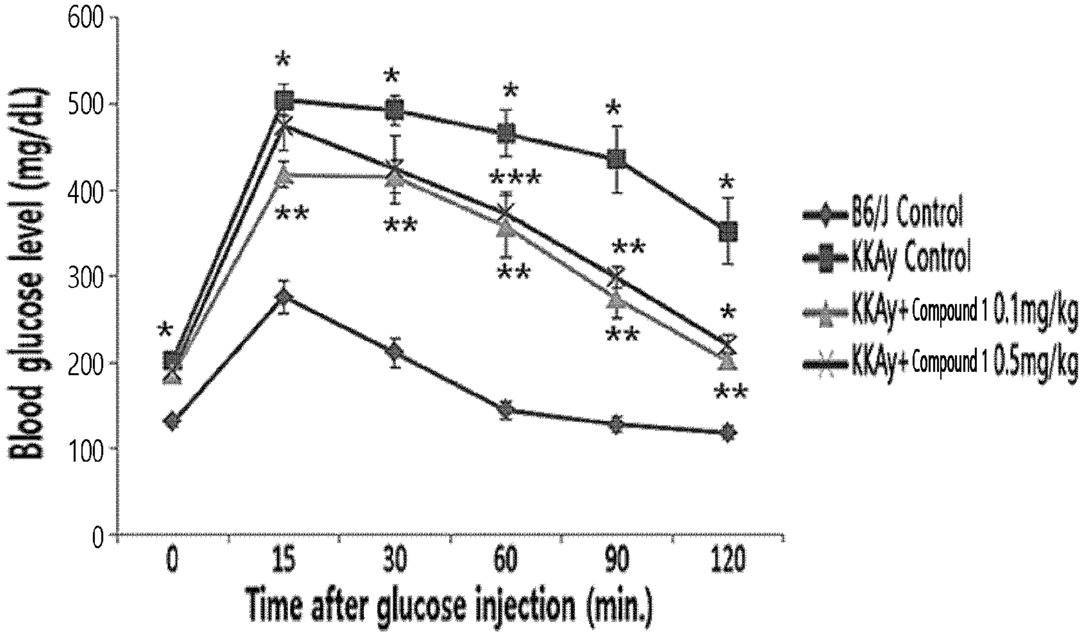
Blood glucose level (mg/dL)
Time after glucose injection (min.)
B6/J Control
KKAy Control
KKAy+Compound 1 0.1mg/kg
KKAy+Compound 1 0.5mg/kg

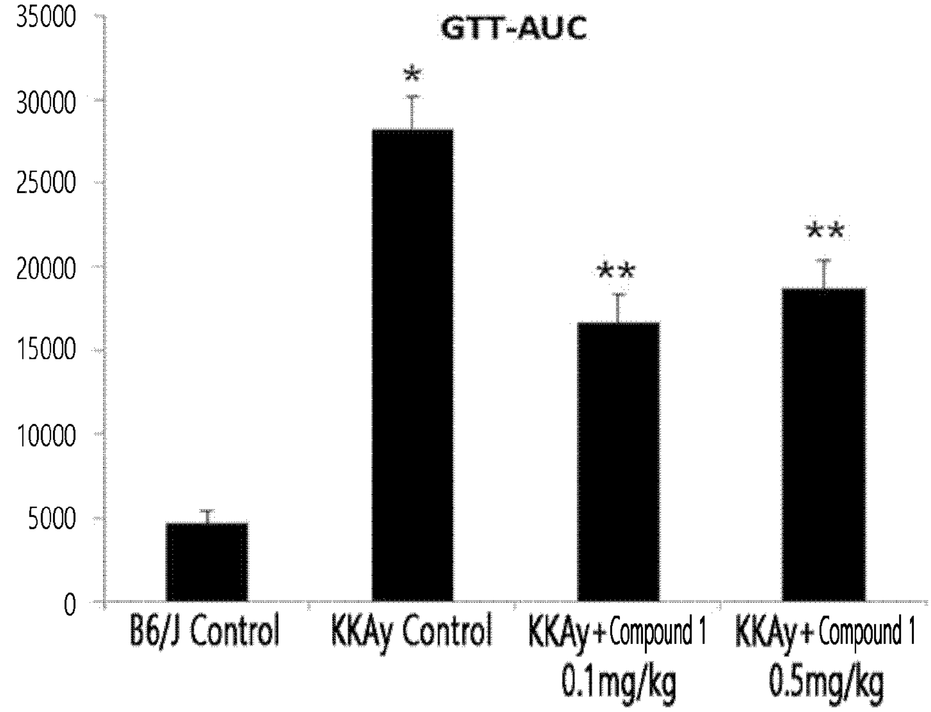
GTT-AUC
B6/J Control
KKAy Control
KKAy+Compound 1 0.1mg/kg
KKAy+Compound 1 0.5mg/kg

## Description

### [Technical Field]

The present disclosure relates to a novel fluorene derivative compound, and use thereof in preventing or treating metabolic diseases and/or cancer diseases through the action thereof as a PDK4 inhibitor.

### [Background Art]

Glycolysis, which is the initial stage of glucose metabolism, yields two ATPs, two NADHs, and two pyruvates which are 3-carbon compounds, from glucose. The pyruvate produced in glycolysis is then converted into acetyl CoA through the action of the pyruvate dehydrogenase complex (PDC) (Non-Patent Document 1). Acetyl CoA enters the citric acid cycle and ultimately produces ATP. Complete aerobic oxidation of glucose produces 36 ATP molecules. In this regard, PDC is a gatekeeping enzyme that plays an important regulatory role in cellular metabolism by linking the citric acid cycle with glycolysis, and gluconeogenesis with oxidative phosphorylation (Non-Patent Document 2). The activity of the enzyme is regulated by the site-specific phosphorylation/dephosphorylation cycle at three serine residues of the E1$\alpha$ subunits (Ser232, Ser293, and Ser300) catalyzed by pyruvate dehydrogenase kinase 1 to 4 (PDK 1 to 4). Each of these PDK1-4 has a different reactivity for the three sites, and phosphorylation at any site leads to inhibition of PDH activity. Dysregulation of the PDH/PDK system is associated with the development of various diseases such as cancer, metabolic diseases, and inflammation, implicating that PDKs are potent therapeutic targets for these diseases.

Among the four PDK isoforms, PDK4 is dramatically increased in the liver, skeletal muscle, heart, adipose tissue, and kidney of fasting or diabetic mammals. PDK4 knockout mice have been reported to have lower blood glucose levels than wild-type mice. In addition, PDK4 knockout mice fed a high-fat diet have lower blood glucose levels and greater insulin sensitivity than wild-type mice. Naturally, PDK4 deficiency increases pyruvate oxidation in the muscle, thereby limiting alanine, lactate, and pyruvate levels required for gluconeogenesis in the liver. Recently, it has been reported that PDK4 levels are elevated in patients with type 2 diabetes. These results suggest that PDK4 activation is associated with metabolic diseases including hyperglycemia and insulin resistance. In addition, PDK4 inhibition has been shown to be effective in nonalcoholic steatohepatitis, cisplatin-induced nephrotoxicity, vascular calcification, and diabetic cardiomyopathy.

Meanwhile, mast cells are considered effector cells that play a central role in acute allergic reactions (Non-Patent Document 3). Significant aggregation of mast cells and IgE receptors induces the release of preformed mediators, including histamine, proteases, and newly synthesized inflammatory mediators, upon stimulation by neuropeptides such as substance P. Activation of mast cells induces several types of diseases, such as passive cutaneous anaphylaxis, asthma, rhinitis, and atopic dermatitis. Recent findings have shown that a PDK inhibitor, dichloroacetic acid (DCA), is able to effectively reduce the degranulation of IgE/Ag-stimulated mast cells and inflammatory cytokine levels, strongly suggesting that metabolic intervention by PDK modulation is a promising therapeutic approach for mast cell-mediated allergic diseases.

Moreover, most cancer cells are characterized by a metabolic shift from mitochondrial oxidation/phosphorylation to cytosolic aerobic glycolysis even under normoxic conditions. This metabolic shift offers proliferative benefits for cancer cells by promoting glycolysis and inhibiting glucose oxidation. Disruption of this unique cancer metabolic pathway offers a therapeutic opportunity in anticancer therapy. PDK4 increases cancer cell proliferation, which implies that the inhibition of PDK4 may have a beneficial effect in cancer treatment. Recent studies have shown that PDK4 can upregulate mutant KRAS activity via posttranslational regulation. Genetic PDK4 depletion or pharmacological PDK4 inhibition reduces glucose and fatty acid oxidation, thereby inhibiting tumor growth of KRAS-mutant colorectal and lung cancer.

### [Disclosure]

### [Technical Problem]

The present inventors have conducted extensive research to discover small-molecule PDK4 inhibitors for the prevention or treatment of metabolic diseases such as obesity, diabetes, etc., and/or cancer diseases. As a result, they have synthesized a series of fluorene derivatives comprising hydrazinylene in the molecule and found that these compounds exhibit PDK4 inhibitory activity, thereby completing the present disclosure.

### [Technical Solution]

An object of the present disclosure is to provide a compound represented by the following Chemical Formula 1 or a pharmaceutically acceptable salt thereof:

[Chemical Formula 1]

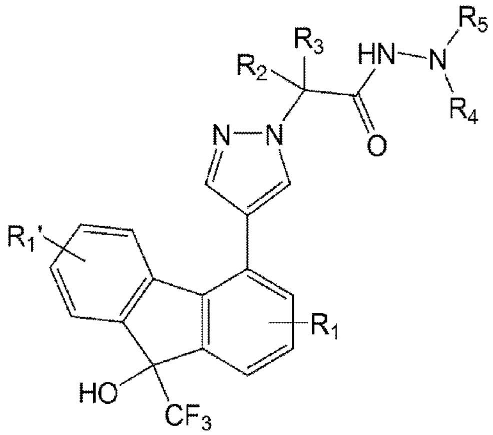

in Chemical Formula 1,

$R_1$ and $R_1$' are each independently hydrogen, $C_{1-4}$ alkoxy, or halogen;
$R_2$ and $R_3$ are each independently hydrogen, $C_{3-10}$ cycloalkyl- $C_{1-4}$ alkyl or $C_{1-4}$ alkyl;
$R_4$ is hydrogen;
$R_5$ is $C_{6-10}$ aryl, 5-10 membered heteroaryl, or 5-10 membered heterocyclyl which is unsubstituted or substituted with one or more substituents selected from the group consisting of oxo, halogen, $C_{1-4}$ haloalkyl, $C_{0-4}$ alkylsulfonyl, carboxy, and $C_{1-4}$ alkoxycarbonyl; or
$R_4$ and $R_5$ are connected to each other to form 5- to 10-membered heterocyclyl together with a nitrogen atom to which they are attached.

Another object of the present disclosure is to provide a method of preparing the compound or pharmaceutically acceptable salt thereof, comprising the step of reacting a compound represented by the following Chemical Formula 2 with a compound represented by the following Chemical Formula 3:

[Chemical Formula 2]

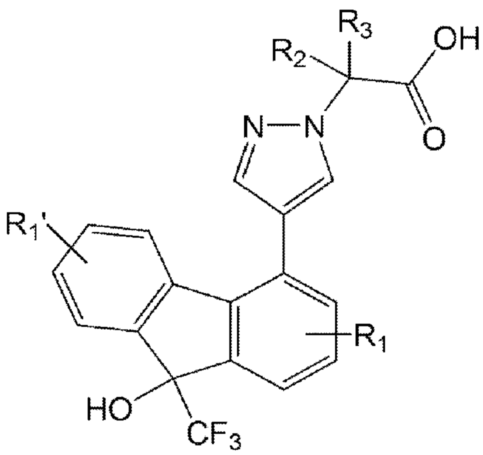

[Chemical Formula 3]

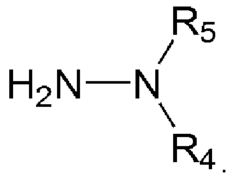

Still another object of the present disclosure is to provide a pharmaceutical composition for preventing or treating a disease related to pyruvate dehydrogenase kinase 4 (PDK4) activity, comprising the compound or pharmaceutically acceptable salt thereof as an active ingredient.

**[Advantageous Effects]**

A series of fluorene derivatives comprising hydrazinylene in the molecule which are newly synthesized according to the present disclosure exhibit the effect of inhibiting PDK4 activity, thereby being usefully applied to the prevention or treatment of diseases related thereto, for example, metabolic diseases such as obesity, diabetes, fatty liver, etc., and/or cancer diseases.

**[Brief Description of the Drawing]**

FIG. 1 shows the results of an intraperitoneal glucose tolerance test performed 10 weeks after drug administration in diabetes animal models;
FIG. 2 shows the results of H&E and TUNEL staining of kidney tissues of acute kidney ischemic reperfusion injury animal models;
FIG. 3 shows NGAL expressions and serum creatinine levels in kidney tissues of acute kidney ischemic reperfusion injury animal models;
FIG. 4 shows apoptosis and mitochondrial superoxide levels in myocardial infarction cell models;
FIG. 5 shows changes in intracellular calcium over time in myocardial infarction cell models;
FIG. 6 shows the results of colonic inflammation analysis in inflammatory bowel disease animal model, in which FIG. 6A shows the results of H&E staining, FIG. 6B shows histological scores, and FIGS. 6C and 6D show images of the recovered whole colon and colon lengths measured therefrom;
FIG. 7 shows inflammatory cytokine mRNA expressions in the colon tissues of inflammatory bowel disease animal models;
FIG. 8 shows serum amylase and lipase activities in acute pancreatitis animal models;
FIG. 9 shows viability of LPS-induced sepsis animal models;
FIG. 10 shows the inhibitory effect on inflammasome formation in bone marrow-derived dendritic cells in LPS-induced sepsis animal models;
FIG. 11 shows the weight recovery effect in graft-versus-host disease animal models;
FIG. 12 shows generation of allogeneic $CD4^+IFNg^+$ T cells in graft-versus-host disease animal models;
FIG. 13 shows mitochondrial oxygen consumption rates measured in Charcot-Marie-Tooth disease animal models;
FIG. 14 shows the inhibitory effect on cortisol production in Cushing's syndrome animal models;
FIG. 15 shows the growth inhibitory effect on normal cells;
FIG. 16 shows the growth inhibitory effect on colorectal cancer cells;
FIG. 17 shows the growth inhibitory effect on thyroid cancer cells;
FIG. 18 shows the growth inhibitory effect on prostate cancer cells and lung cancer cells;
FIG. 19 shows the growth inhibitory effect on breast cancer cells and bladder cancer cells;
FIG. 20 shows the growth inhibitory effect on liver cancer cells, brain cancer cells, and ovarian cancer cells;
FIG. 21 shows the anticancer effect and body weight change 21 days after drug administration in HCT-116 colorectal cancer models; and
FIG. 22 shows the anticancer effect, TUNEL analysis, and body weight change 21 days after drug administration in CT26 colorectal cancer models.

**[Detailed Description of Preferred Embodiments]**

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

A first aspect of the present disclosure provides a compound represented by the following Chemical Formula 1 or a pharmaceutically acceptable salt thereof:

[Chemical Formula 1]

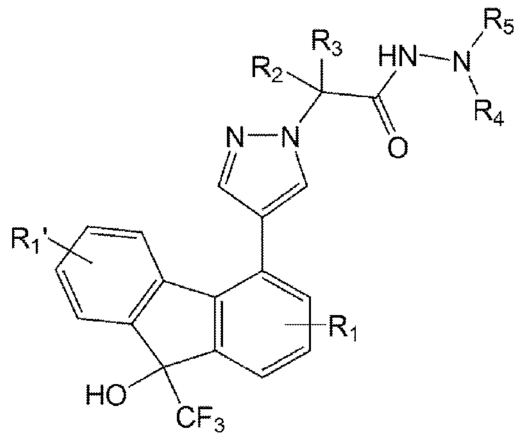

in Chemical Formula 1,

$R_1$ and $R_1$' are each independently hydrogen, $C_{1-4}$ alkoxy, or halogen;
$R_2$ and $R_3$ are each independently hydrogen, $C_{3-10}$ cycloalkyl- $C_{1-4}$ alkyl or $C_{1-4}$ alkyl;
$R_4$ is hydrogen;
$R_5$ is $C_{6-10}$ aryl, 5-10 membered heteroaryl, or 5-10 membered heterocyclyl which is unsubstituted or substituted with one or more substituents selected from the group consisting of oxo, halogen, $C_{1-4}$ haloalkyl, $C_{0-4}$ alkylsulfonyl, carboxy, and $C_{1-4}$ alkoxycarbonyl; or
$R_4$ and $R_5$ are connected to each other to form 5- to 10-membered heterocyclyl together with a nitrogen atom to which they are attached.

For example, the compound may be a compound represented by the following Chemical Formula 1-1, but is not limited thereto:

[Chemical Formula 1-1]

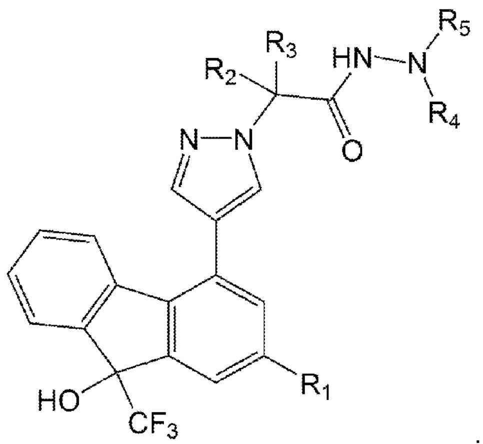

For example, in Chemical Formula 1 or Chemical Formula 1-1, $R_1$ and $R_1$' may be each independently methoxy or fluoro, but are not limited thereto.

For example, in Chemical Formula 1 or Chemical Formula 1-1, $R_2$ is hydrogen or methyl, and $R_3$ may be hydrogen, methyl, ethyl, isopropyl, isobutyl, or cyclohexylmethyl, but is not limited thereto.

For example, in Chemical Formula 1 or Chemical Formula 1-1, $R_4$ is hydrogen, $R_5$ is phenyl, naphthyl, pyridinyl, pyrimidinyl, pyrazinyl, quinoxalinyl, quinolinyl, quinazolinyl, or dihydroquinolinyl which is unsubstituted or substituted with one or more substituents selected from the group consisting of oxo, fluoro, chloro, trifluoromethyl, methylsulfonyl, carboxy, and tert-butoxycarbonyl; or $R_4$ and $R_5$ are connected to each other to form piperidinyl or azepanyl together with a nitrogen atom to which they are attached, but are not limited thereto.

Specifically the compound may be

1. 2-(4-(2-fluoro-9-hydroxy-9-(trifluoromethyl)-9H-fluoren-4-yl)-1H-pyrazol-1-yl)-N'-(4-fluorophenyl)propanehydrazide,
2. N'-(4-fluorophenyl)-2-(4-(9-hydroxy-2-methoxy-9-(trifluoromethyl)-9H-fluoren-4-yl)-1H-pyrazol-1-yl)propanehydrazide,
3. N'-(2-fluoro-4-(methylsulfonyl)phenyl)-2-(4-(2-fluoro-9-hydroxy-9-(trifluoromethyl)-9H-fluoren-4-yl)-1H-pyrazol-1-yl)propanehydrazide,
4. 2-(4-(2-fluoro-9-hydroxy-9-(trifluoromethyl)-9H-fluoren-4-yl)-1H-pyrazol-1-yl)-N'-phenylpropanehydrazide,
5. 2-(4-(2-fluoro-9-hydroxy-9-(trifluoromethyl)-9H-fluoren-4-yl)-1H-pyrazol-1-yl)-N'-(naphthalen-1-yl)propanehydrazide,
6. N'-(2-chlorophenyl)-2-(4-(2-fluoro-9-hydroxy-9-(trifluoromethyl)-9H-fluoren-4-yl)-1H-pyrazol-1-yl)propanehydrazide,
7. N'-(4-chlorophenyl)-2-(4-(2-fluoro-9-hydroxy-9-(trifluoromethyl)-9H-fluoren-4-yl)-1H-pyrazol-1-yl)propanehydrazide,
8. 2-(4-(2-fluoro-9-hydroxy-9-(trifluoromethyl)-9H-fluoren-4-yl)-1H-pyrazol-1-yl)-N'-(pyridin-2-yl)propanehydrazide,
9. N'-(2-fluoro-4-(methylsulfonyl)phenyl)-2-(4-(9-hydroxy-2-methoxy-9-(trifluoromethyl)-9H-fluoren-4-yl)-1H-pyrazol-1-yl)propanehydrazide,
10. N'-(4-chlorophenyl)-2-(4-(9-hydroxy-2-methoxy-9-(trifluoromethyl)-9H-fluoren-4-yl)-1H-pyrazol-1-yl)propanehydrazide,
11. 2-(4-(9-hydroxy-2-methoxy-9-(trifluoromethyl)-9H-fluoren-4-yl)-1H-pyrazol-1-yl)-N'-(naphthalen-1-yl)propanehydrazide,
12. N'-(2-chlorophenyl)-2-(4-(9-hydroxy-2-methoxy-9-(trifluoromethyl)-9H-fluoren-4-yl)-1H-pyrazol-1-yl)propanehydrazide,
13. 2-(4-(2-fluoro-9-hydroxy-9-(trifluoromethyl)-9H-fluoren-4-yl)-1H-pyrazol-1-yl)-N'-(2-fluorophenyl)propanehydrazide,
14. 6-(2-(2-(4-(2-fluoro-9-hydroxy-9-(trifluoromethyl)-9H-fluoren-4-yl)-1H-pyrazol-1-yl)propanoyl)hydrazinyl)nicotinic acid,
15. tert-butyl 6-(2-(2-(4-(9-hydroxy-2-methoxy-9-(trifluoromethyl)-9H-fluoren-4-yl)-1H-pyrazol-1-yl)propanoyl)hydrazinyl)nicotinate,
16. 6-(2-(2-(4-(9-hydroxy-2-methoxy-9-(trifluoromethyl)-9H-fluoren-4-yl)-1 H-pyrazol-1-yl)propanoyl)hydrazinyl)nicotinic acid,
17. 2-(4-(9-hydroxy-2-methoxy-9-(trifluoromethyl)-9H-fluoren-4-yl)-1H-pyrazol-1-yl)-N'-(pyridin-2-yl)propanehydrazide,
18. 2-(4-(2-fluoro-9-hydroxy-9-(trifluoromethyl)-9H-fluoren-4-yl)-1H-pyrazol-1-yl)-N'-(pyridin-2-yl)acetohydrazide,
19. tert-butyl 6-(2-(2-(4-(2-fluoro-9-hydroxy-9-(trifluoromethyl)-9H-fluoren-4-yl)-1H-pyrazol-1-yl)acetyl)hydrazinyl)nicotinate,
20. 6-(2-(2-(4-(2-fluoro-9-hydroxy-9-(trifluoromethyl)-9H-fluoren-4-yl)-1H-pyrazol-1-yl)acetyl)hydrazinyl)nicotinic acid,
21. 2-(4-(2-fluoro-9-hydroxy-9-(trifluoromethyl)-9H-fluoren-4-yl)-1H-pyrazol-1-yl)-N'-(4-fluorophenyl)acetohydrazide,
22. 2-(4-(2-fluoro-9-hydroxy-9-(trifluoromethyl)-9H-fluoren-4-yl)-1H-pyrazol-1-yl)-N'-(4-(methylsulfonyl)phenyl)propanehydrazide,
23. 2-(4-(9-hydroxy-2-methoxy-9-(trifluoromethyl)-9H-fluoren-4-yl)-1H-pyrazol-1-yl)-N'-(4-(methylsulfonyl)phenyl)propanehydrazide,
24. 2-(4-(2-fluoro-9-hydroxy-9-(trifluoromethyl)-9H-fluoren-4-yl)-1H-pyrazol-1-yl)-N'-(pyrazin-2-yl)propanehydrazide,
25. 2-(4-(9-hydroxy-2-methoxy-9-(trifluoromethyl)-9H-fluoren-4-yl)-1H-pyrazol-1-yl)-N'-(pyrazin-2-yl)propanehydrazide,

26. 2-(4-(2-fluoro-9-hydroxy-9-(trifluoromethyl)-9H-fluoren-4-yl)-1H-pyrazol-1-yl)-N'-(5-(trifluoromethyl)pyridin-2-yl) propanehydrazide,
27. 2-(4-(9-hydroxy-2-methoxy-9-(trifluoromethyl)-9H-fluoren-4-yl)-1 H-pyrazol-1-yl)-N'-(5-(trifluoromethyl)pyridin-2-yl)propanehydrazide,
28. 2-(4-(2-fluoro-9-hydroxy-9-(trifluoromethyl)-9H-fluoren-4-yl)-1H-pyrazol-1-yl)-N'-(pyrimidin-2-yl)propanehydrazide,
29. 2-(4-(9-hydroxy-2-methoxy-9-(trifluoromethyl)-9H-fluoren-4-yl)-1H-pyrazol-1-yl)-N'-(pyrimidin-2-yl)propanehydrazide,
30. 2-(4-(2-fluoro-9-hydroxy-9-(trifluoromethyl)-9H-fluoren-4-yl)-1H-pyrazol-1-yl)-N'-(pyridin-3-yl)propanehydrazide,
31. 2-(4-(9-hydroxy-2-methoxy-9-(trifluoromethyl)-9H-fluoren-4-yl)-1H-pyrazol-1-yl)-N'-(pyridin-3-yl)propanehydrazide,
32. N'-(3-chloro-5-(trifluoromethyl)pyridin-2-yl)-2-(4-(2-fluoro-9-hydroxy-9-(trifluoromethyl)-9H-fluoren-4-yl)-1H-pyrazol-1-yl)propanehydrazide,
33. N'-(3-chloro-5-(trifluoromethyl)pyridin-2-yl)-2-(4-(9-hydroxy-2-methoxy-9-(trifluoromethyl)-9H-fluoren-4-yl)-1H-pyrazol-1-yl)propanehydrazide,
34. 2-(4-(2-fluoro-9-hydroxy-9-(trifluoromethyl)-9H-fluoren-4-yl)-1H-pyrazol-1-yl)-N'-(pyridin-4-yl)propanehydrazide,
35. 2-(4-(2-fluoro-9-hydroxy-9-(trifluoromethyl)-9H-fluoren-4-yl)-1H-pyrazol-1-yl)-N'-(quinazolin-4-yl)propanehydrazide,
36. 2-(4-(2-fluoro-9-hydroxy-9-(trifluoromethyl)-9H-fluoren-4-yl)-1H-pyrazol-1-yl)-N'-(quinolin-4-yl)propanehydrazide,
37. 2-(4-(2-fluoro-9-hydroxy-9-(trifluoromethyl)-9H-fluoren-4-yl)-1H-pyrazol-1-yl)-N'-(quinoxalin-2-yl)propanehydrazide,
38. 2-(4-(2-fluoro-9-hydroxy-9-(trifluoromethyl)-9H-fluoren-4-yl)-1H-pyrazol-1-yl)-N'-(2-oxo-1,2-dihydroquinolin-3-yl)propanehydrazide,
39. 2-(4-(2-fluoro-9-hydroxy-9-(trifluoromethyl)-9H-fluoren-4-yl)-1H-pyrazol-1-yl)-N'-(quinolin-2-yl)propanehydrazide,
40. 2-(4-(2-fluoro-9-hydroxy-9-(trifluoromethyl)-9H-fluoren-4-yl)-1H-pyrazol-1-yl)-N'-(1-methyl-2-oxo-1,2-dihydroquinolin-4-yl)propanehydrazide,
41. 2-(4-(2-fluoro-9-hydroxy-9-(trifluoromethyl)-9H-fluoren-4-yl)-1H-pyrazol-1-yl)-N'-(imidazo[1,2-b]pyridazin-6-yl) propanehydrazide,
42. N'-(benzo[d]thiazol-2-yl)-2-(4-(2-fluoro-9-hydroxy-9-(trifluoromethyl)-9H-fluoren-4-yl)-1H-pyrazol-1-yl)propanehydrazide,
43. 2-(4-(2-fluoro-9-hydroxy-9-(trifluoromethyl)-9H-fluoren-4-yl)-1H-pyrazol-1-yl)-N'-(4-fluorophenyl)butanehydrazide,
44. 2-(4-(2-fluoro-9-hydroxy-9-(trifluoromethyl)-9H-fluoren-4-yl)-1H-pyrazol-1-yl)-N'-(4-fluorophenyl)-3-methylbutanehydrazide,
45. 2-(4-(2-fluoro-9-hydroxy-9-(trifluoromethyl)-9H-fluoren-4-yl)-1H-pyrazol-1-yl)-N'-(4-fluorophenyl)-4-methylpentanehydrazide,
46. 3-cyclohexyl-2-(4-(2-fluoro-9-hydroxy-9-(trifluoromethyl)-9H-fluoren-4-yl)-1H-pyrazol-1-yl)-N'-(4-fluorophenyl) propanehydrazide,
47. 2-(4-(2-fluoro-9-hydroxy-9-(trifluoromethyl)-9H-fluoren-4-yl)-1H-pyrazol-1-yl)-N'-(4-fluorophenyl)-2-methylpropanehydrazide,
48. (S)-2-(4-((S)-2-fluoro-9-hydroxy-9-(trifluoromethyl)-9H-fluoren-4-yl)-1H-pyrazol-1-yl)-N'-(4-fluorophenyl)propanehydrazide,
49. (R)-2-(4-((R)-2-fluoro-9-hydroxy-9-(trifluoromethyl)-9H-fluoren-4-yl)-1H-pyrazol-1-yl)-N'-(4-fluorophenyl)propanehydrazide,
50. (S)-2-(4-((R)-2-fluoro-9-hydroxy-9-(trifluoromethyl)-9H-fluoren-4-yl)-1H-pyrazol-1-yl)-N'-(4-fluorophenyl)propanehydrazide,
51. (R)-2-(4-((S)-2-fluoro-9-hydroxy-9-(trifluoromethyl)-9H-fluoren-4-yl)-1H-pyrazol-1-yl)-N'-(4-fluorophenyl)propanehydrazide,
52. 2-(4-(9-hydroxy-2-methoxy-9-(trifluoromethyl)-9H-fluoren-4-yl)-1H-pyrazol-1-yl)-N-(piperidin-1-yl)propanamide,
53. 2-(4-(2-fluoro-9-hydroxy-9-(trifluoromethyl)-9H-fluoren-4-yl)-1H-pyrazol-1-yl)-N-(piperidin-1-yl)propanamide, or
54. N-(azepan-1-yl)-2-(4-(2-fluoro-9-hydroxy-9-(trifluoromethyl)-9H-fluoren-4-yl)-1H-pyrazol-1-yl)propanamide, but is not limited thereto.

Furthermore, these compounds may be represented by Chemical Formulae disclosed in Table 1 below.

[Table 1]

| Compound | Structural formula | Compound | Structural formula |
|---|---|---|---|
| 1 | 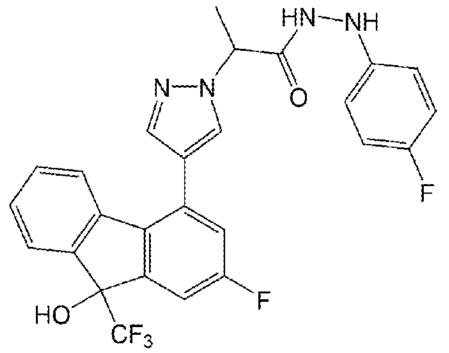 | 2 | 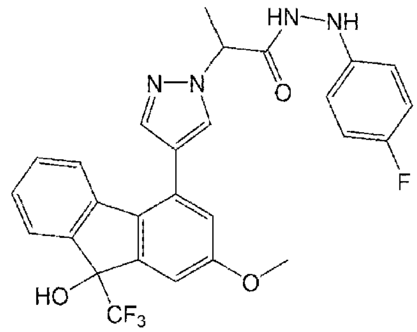 |
| 3 | 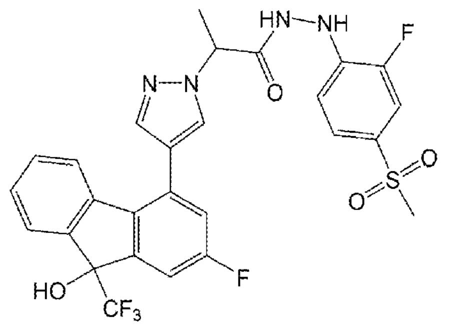 | 4 | 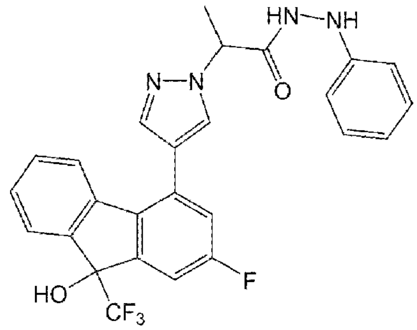 |
| 5 | 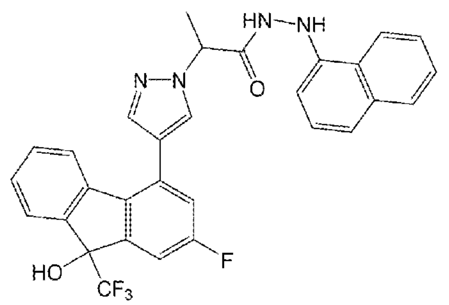 | 6 | 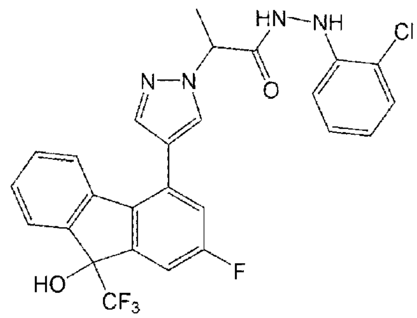 |
| 7 | 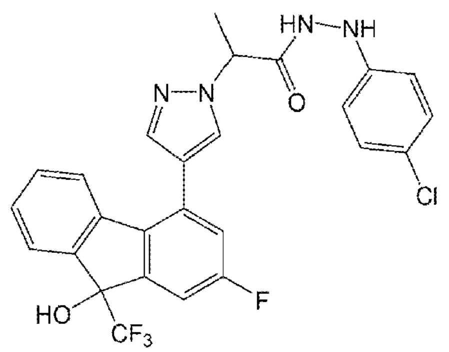 | 8 | 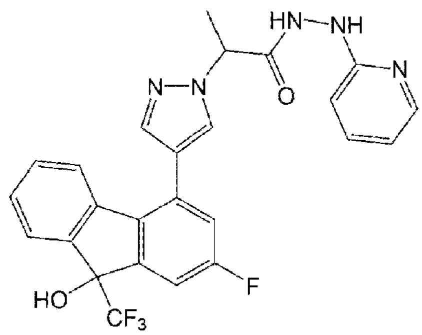 |

(continued)

| Compound | Structural formula | Compound | Structural formula |
|---|---|---|---|
| 9 | 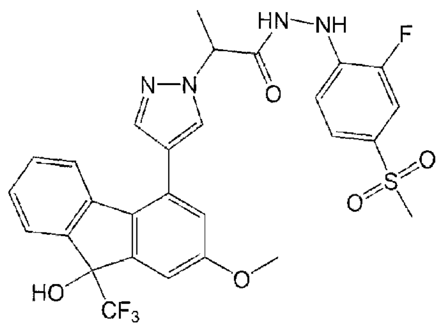 | 10 | 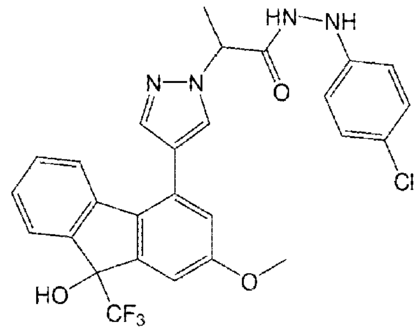 |
| 11 | 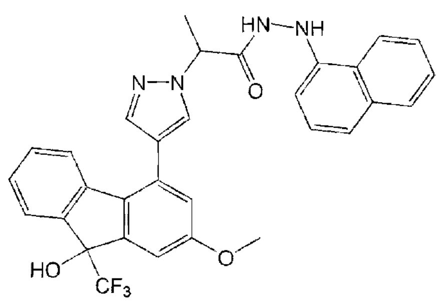 | 12 | 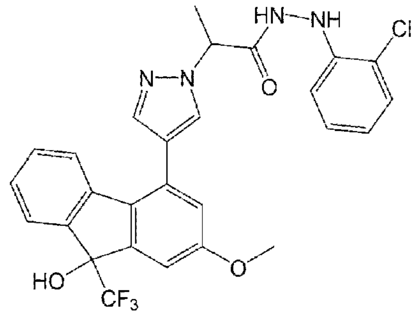 |
| 13 | 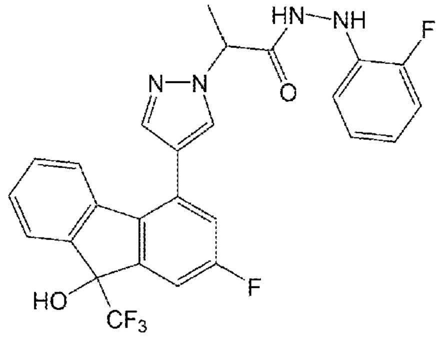 | 14 | 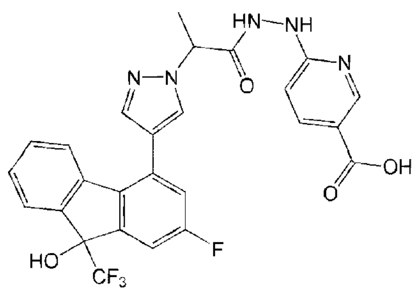 |
| 15 | 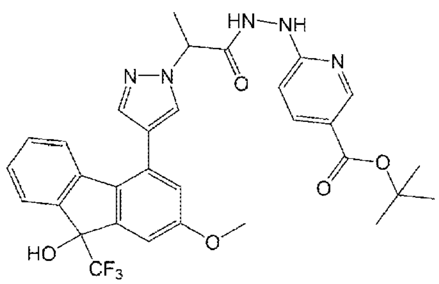 | 16 | 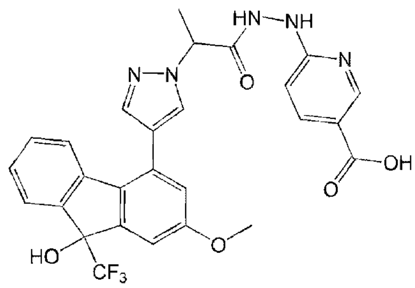 |
| 17 | 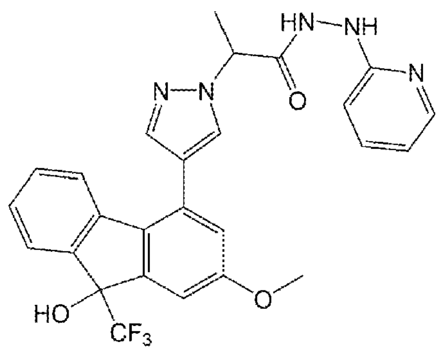 | 18 | 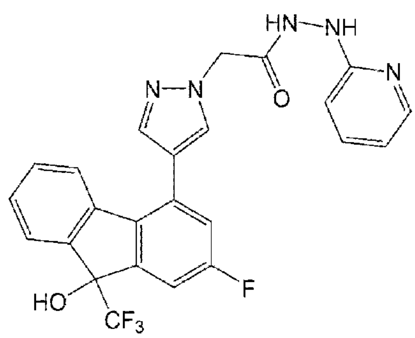 |

(continued)

| Compound | Structural formula | Compound | Structural formula |
|---|---|---|---|
| 19 | 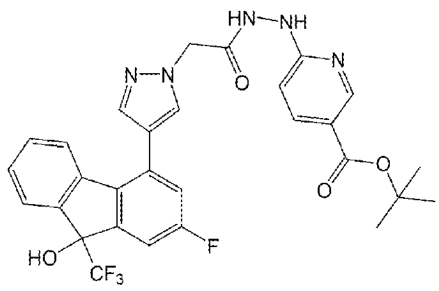 | 20 | 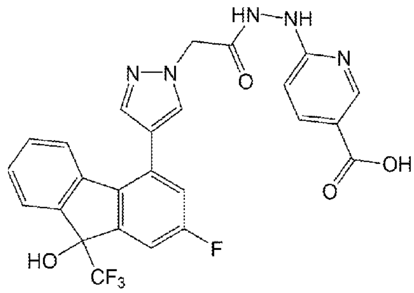 |
| 21 | 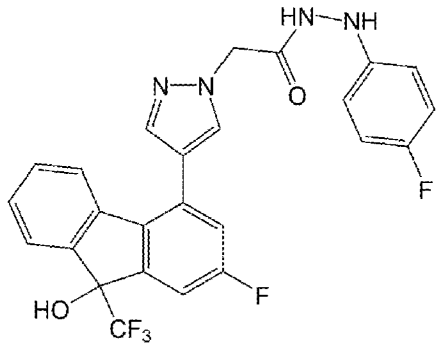 | 22 | 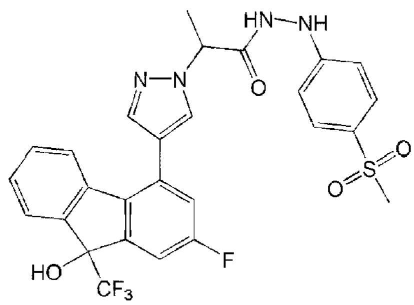 |
| 23 | 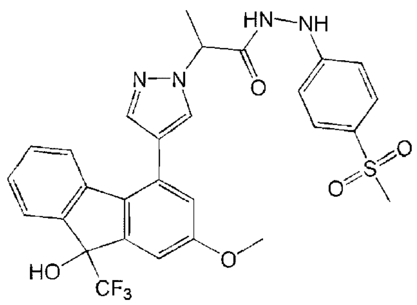 | 24 | 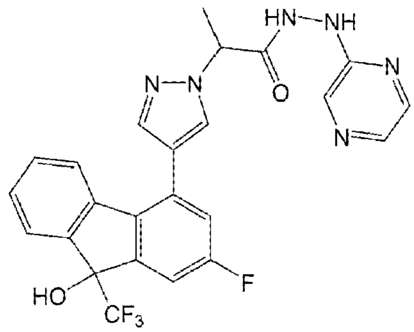 |
| 25 | 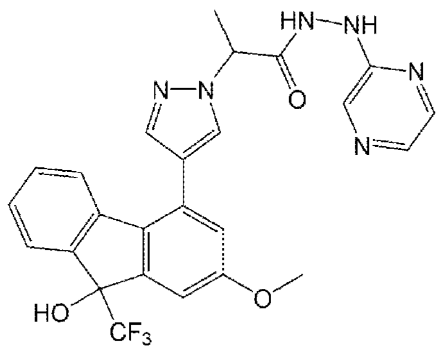 | 26 | 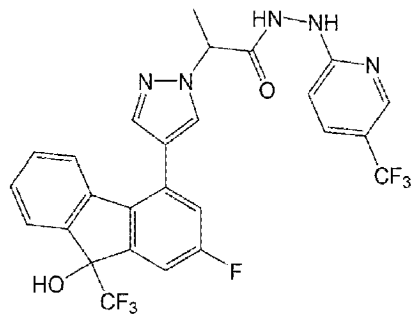 |

(continued)

| Compound | Structural formula | Compound | Structural formula |
|---|---|---|---|
| 27 | 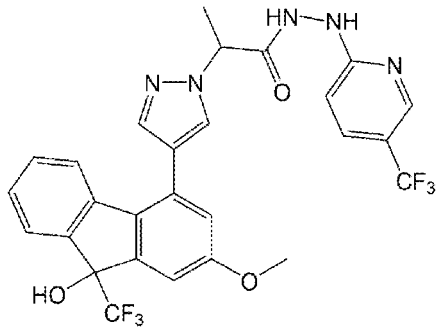 | 28 | 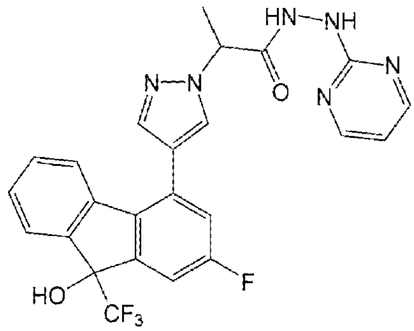 |
| 29 | 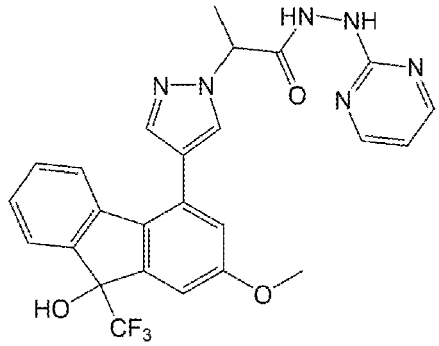 | 30 | 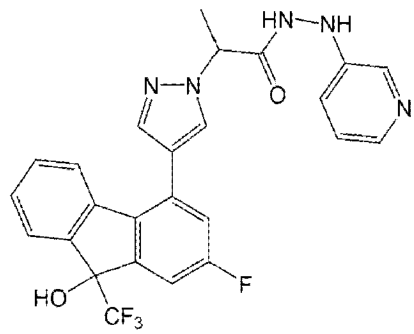 |
| 31 | 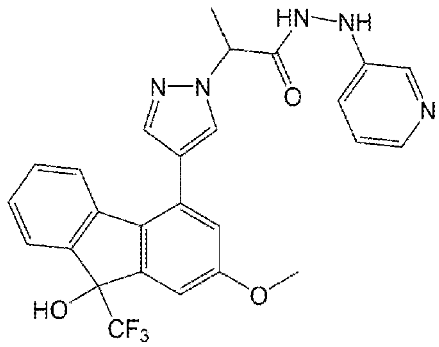 | 32 | 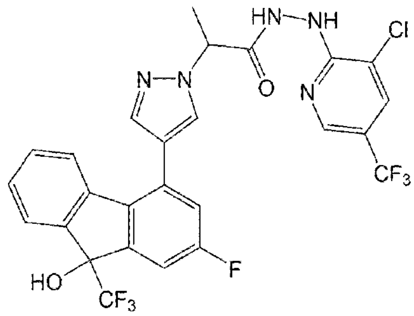 |
| 33 | 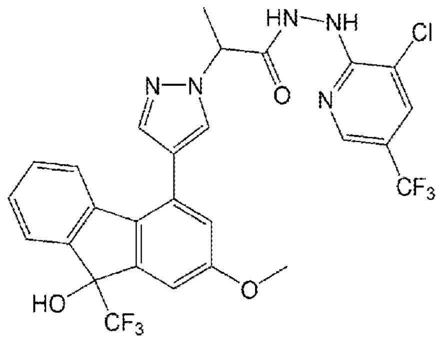 | 34 | 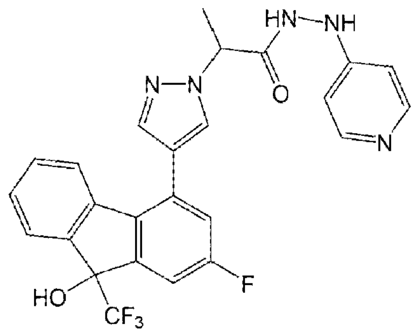 |

(continued)

| Compound | Structural formula | Compound | Structural formula |
|---|---|---|---|
| 35 | 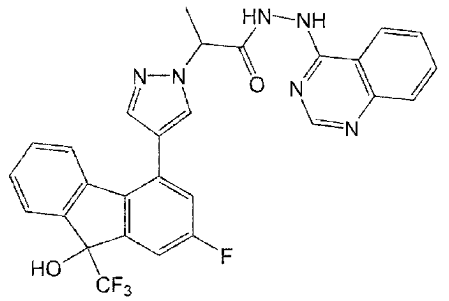 | 36 | 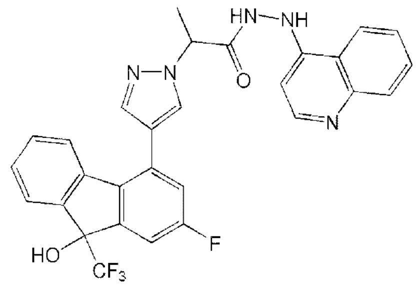 |
| 37 | 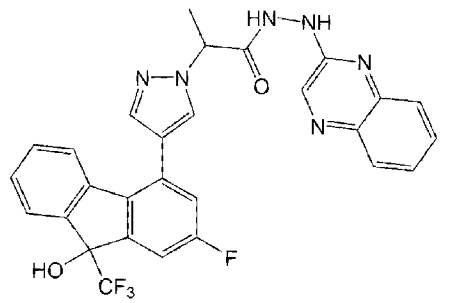 | 38 | 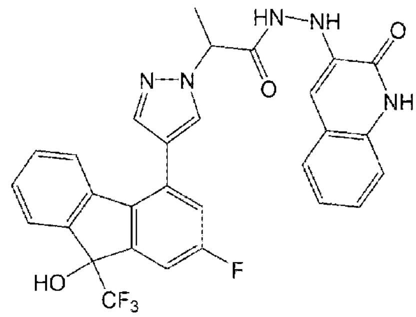 |
| 39 | 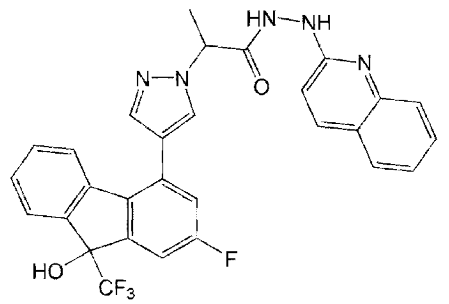 | 40 | 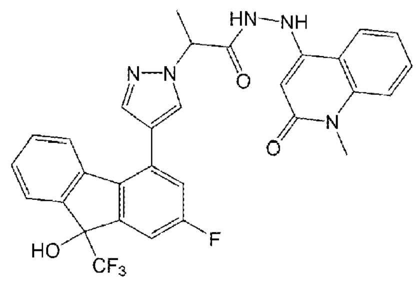 |
| 41 | 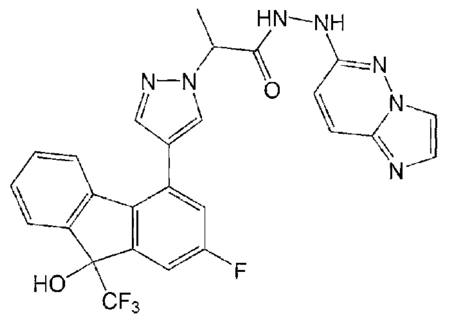 | 42 | 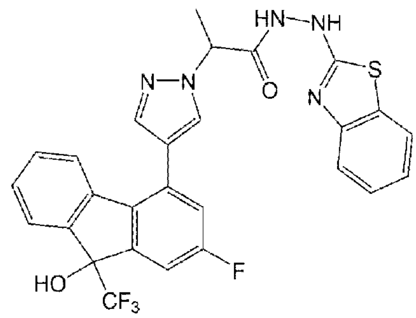 |
| 43 | 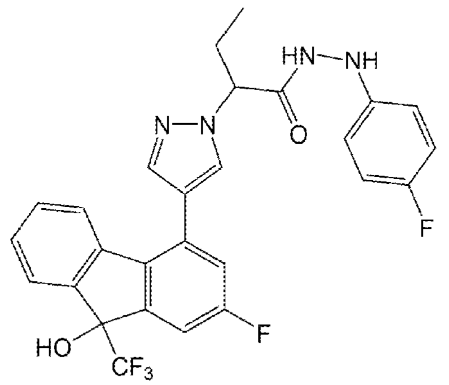 | 44 | 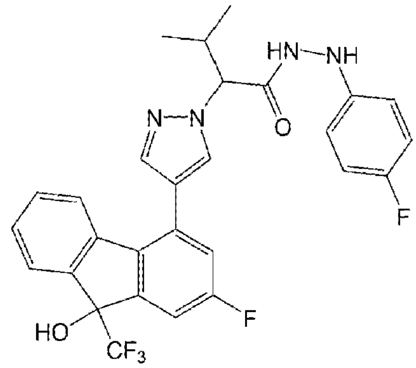 |

(continued)

| Compound | Structural formula | Compound | Structural formula |
|---|---|---|---|
| 45 | 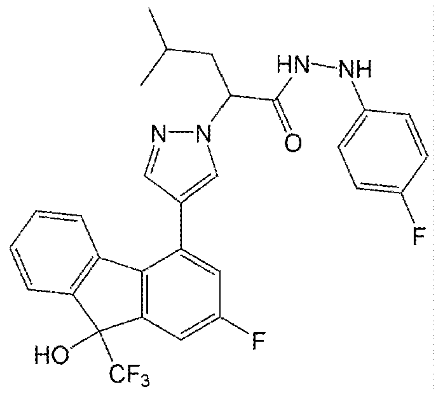 | 46 | 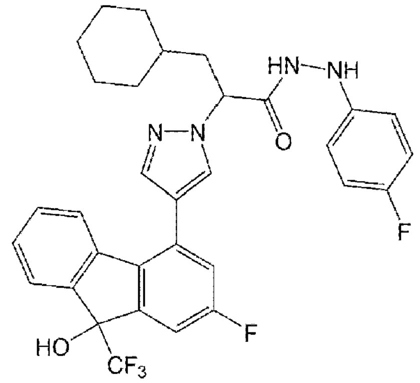 |
| 47 | 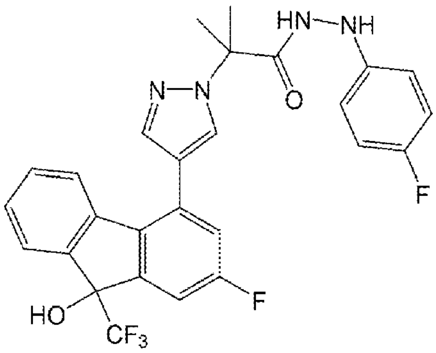 | 48 | 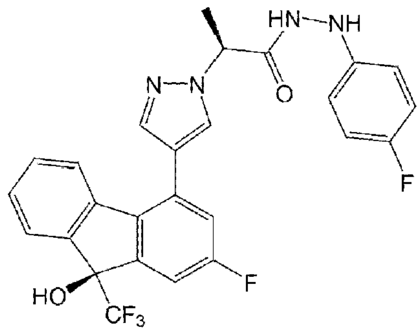 |
| 49 | 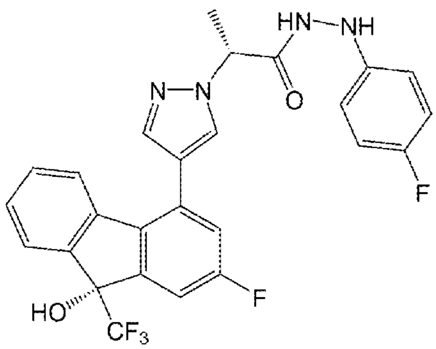 | 50 | 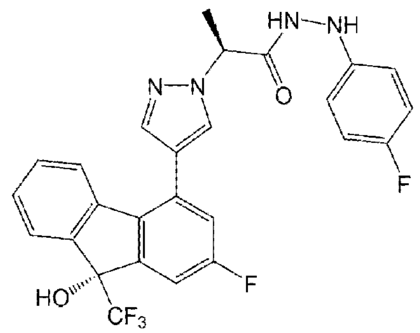 |
| 51 | 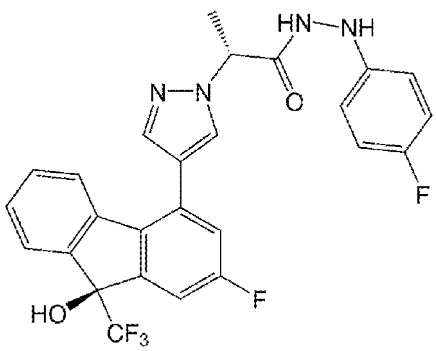 | 52 | 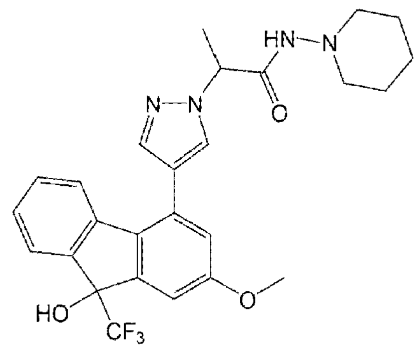 |

(continued)

| Compound | Structural formula | Compound | Structural formula |
|---|---|---|---|
| 53 | 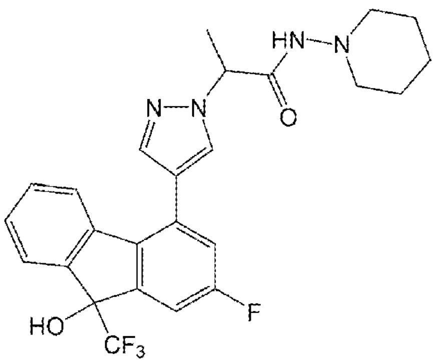 | 54 | 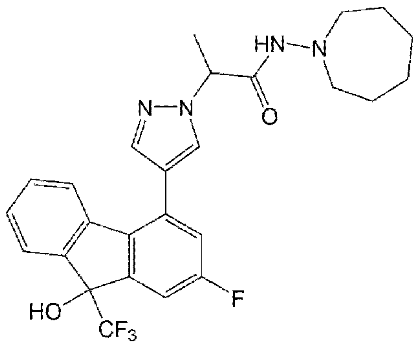 |

The compound of the present disclosure may exist in the form of a pharmaceutically acceptable salt. As the salt, an acid addition salt formed by a pharmaceutically acceptable free acid is useful. As used herein, the term "pharmaceutically acceptable salt", having a concentration that is relatively nontoxic to and has a harmless effect on a patient, means any organic or inorganic addition salt of the compound in which side effects caused by this salt do not degrade the beneficial efficacy of the compound represented by Chemical Formula 1.

Acid addition salts may be prepared using a common method of, for example, by dissolving a compound in an excess of an aqueous acid solution, and then precipitating the salt using a water-miscible organic solvent, e.g., methanol, ethanol, acetone, or acetonitrile. Equimolar amounts of the compound and an acid or alcohol (e.g., glycol monomethyl ether) in water may be heated, and subsequently, the resulting mixture may be evaporated or dried, or the precipitated salt may be suction-filtered.

At this time, organic and inorganic acids may be used as the free acid. As the inorganic acids, hydrochloric acid, phosphoric acid, sulfuric acid, nitric acid, tartaric acid, etc. may be used. As the organic acids, methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, etc. may be used, but are not limited thereto.

Furthermore, a pharmaceutically acceptable metal salt may be prepared using a base. Alkali metal salts or alkaline earth metal salts may be obtained, for example, by dissolving a compound in an excess of alkali metal hydroxide or alkali earth metal hydroxide solution, filtering the undissolved compound salt, and then evaporating and drying the filtrate. At this time, particularly preparing sodium, potassium or calcium salts are pharmaceutically suitable as the metal salt, but are not limited thereto. Additionally, the corresponding silver salt may be obtained by reacting an alkali metal or alkaline earth metal salt with a suitable silver salt (e.g., silver nitrate).

Unless otherwise indicated, the pharmaceutically acceptable salt of the compound of the present disclosure may comprise salts of acidic or basic groups that may be present in the compound of Chemical Formula 1. For example, sodium, calcium, and potassium salts of a hydroxy group may be comprised as pharmaceutically acceptable salts. Other pharmaceutically acceptable salts of an amino group may comprise hydrobromide, sulfate, hydrogen sulfate, phosphate, hydrogen phosphate, dihydrogen phosphate, acetate, succinate, citrate, tartrate, lactate, mandelate, methanesulfonate (mesylate) and p-toluenesulfonate (tosylate) salts, etc. These salts may be prepared using a method of preparing a salt known in the art.

As for the salt of the fluorene derivative compound of the present disclosure, any salt of the fluorene derivative, which is a pharmaceutically acceptable salt exhibiting pharmacological activity equivalent to that of the fluorene derivative compound, may be used without limitation.

Further, the compound represented by Chemical Formula 1 according to the present disclosure may comprise not only pharmaceutically acceptable salts thereof, but also solvates such as hydrates, which may be prepared therefrom, and all possible stereoisomers without limitation. Solvates and stereoisomers of the compound represented by Chemical Formula 1 may be prepared from the compound represented by Chemical Formula 1 using methods known in the art.

Furthermore, the compound represented by Chemical Formula 1 according to the present disclosure may be prepared in a crystalline form or an amorphous form. When prepared in a crystalline form, the compound may be optionally hydrated or solvated. In the present disclosure, not only stoichiometric hydrates of the compound represented by Chemical Formula 1, but also the compounds comprising various amounts of water may be comprised. Solvates of the compound represented by Chemical Formula 1 according to the present disclosure may comprise both stoichiometric solvates and non-stoichiometric solvates.

A second aspect of the present disclosure provides a method of preparing the compound or pharmaceutically acceptable salt thereof of the first aspect, comprising the step of reacting a compound represented by the following Chemical Formula 2 with a compound represented by the following Chemical Formula 3:

[Chemical Formula 2]

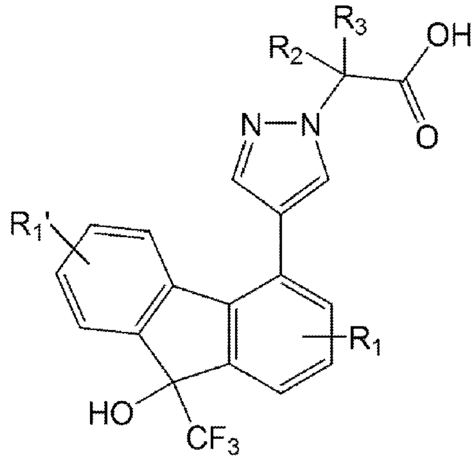

[Chemical Formula 3]

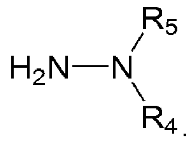

in Chemical Formulae 2 and 3, R1' and $R_1$ to $R_5$ are as defined above.

For example, the reaction may be performed in the presence of 2-(1H-Benzotriazole-1-yl)-1,1,3,3-tetramethylaminium tetrafluoroborate (TBTU) and N,N-Diisopropylethylamine (DIPEA), but is not limited thereto

The compound of Chemical Formula 2 may be prepared by a process comprising one or more reactions of the following Reaction Schemes 1 to 7, but is not limited thereto.

[Reaction Scheme 1]

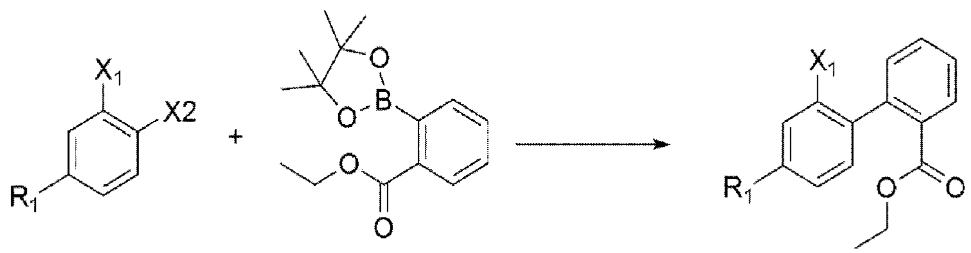

[Reaction Scheme 2]

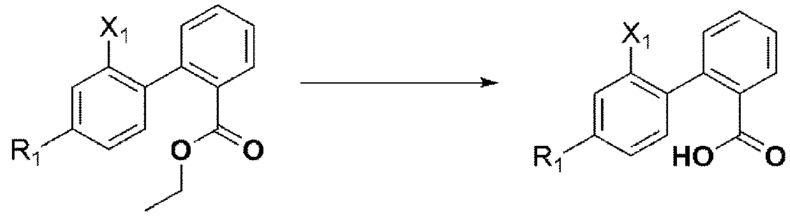

[Reaction Scheme 3]

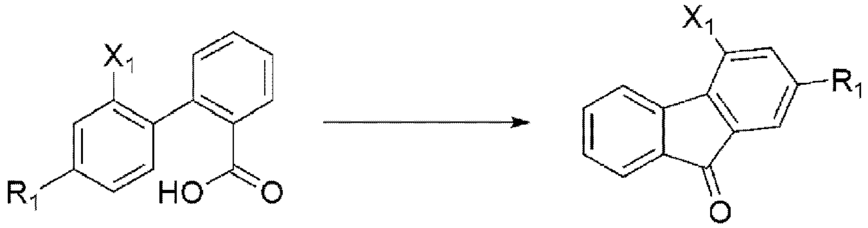

[Reaction Scheme 4]

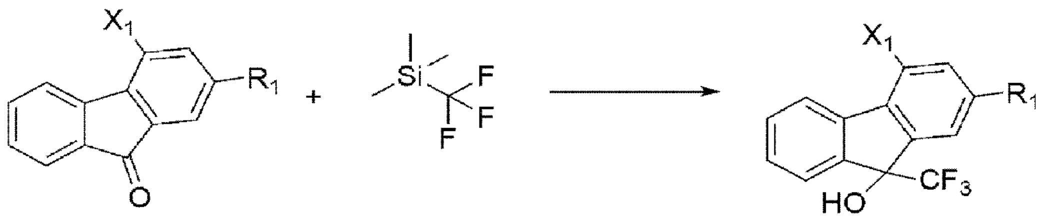

[Reaction Scheme 5]

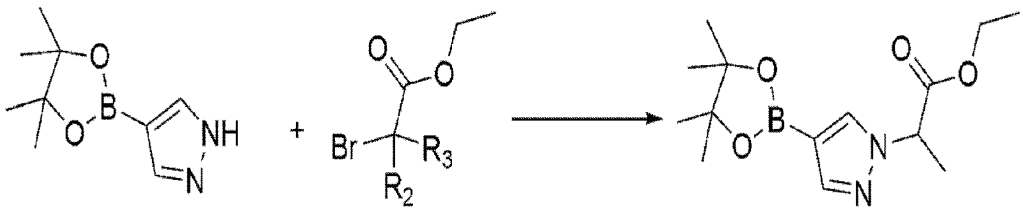

[Reaction Scheme 6]

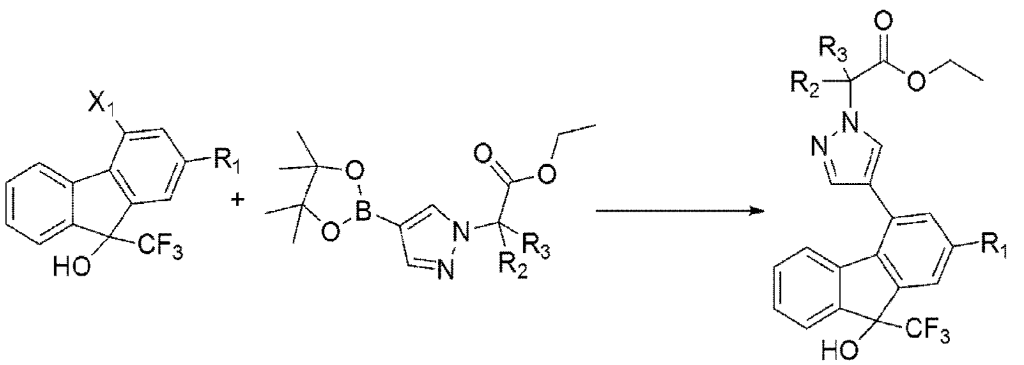

[Reaction Scheme 7]

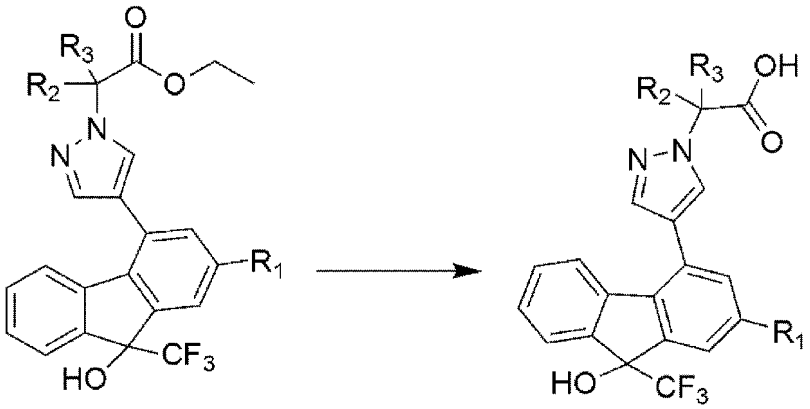

In Reaction Schemes 1 to 7, $R_1$ to $R_3$ are as defined above.

In Reaction Schemes 1 to 7, $X_1$ and $X_2$ may be each independently halogen, and specifically, $X_1$ may be chloro, and $X_2$ may be bromo, but are not limited thereto.

The reaction of Reaction Scheme 1 may be performed by heating to 90°C to 130°C in the presence of $K_3PO_4$ and $PdCl_2(PPh_3)_2$ in a sealed container, but is not limited thereto. At this time, a mixed solvent of toluene and water may be used as a solvent, but is not limited thereto.

The reaction of Reaction Scheme 2 may be performed by adding a base, for example, sodium hydroxide, and heating to 60°C to 100°C, but is not limited thereto. At this time, a lower alcohol, for example, ethanol, may be used as a solvent, but is not limited thereto.

The reaction of Reaction Scheme 3 may be performed by mixing with Eaton's reagent and heating to 60°C to 100°C, but is not limited thereto.

The reaction of Reaction Scheme 4 may be performed by cooling a mixture of the fluorene derivative among reactants and an excess of cesium fluoride and $K_2CO_3$ to 0°C, maintaining the temperature, adding another reactant, trimethyl(trifluoromethyl)silane dropwise, slowly bringing the mixture to room temperature, and stirring, but is not limited thereto. At this time, an organic solvent, DMF may be used as a solvent, but is not limited thereto.

The reaction of Reaction Scheme 5 may be performed by heating to 70°C to 100°C in the presence of cesium carbonate, but is not limited thereto. At this time, an organic solvent, acetonitrile may be used as a solvent, but is not limited thereto.

The reaction of Reaction Scheme 6 may be performed by mixing $Pd(OAc)_2$ and Sphos in a sealed container, and heating to 80°C to 120°C, but is not limited thereto. At this time, a mixed solvent of toluene and water may be used as a solvent, but is not limited thereto.

The reaction of Reaction Scheme 7 may be performed by adding a base, for example, sodium hydroxide, and allowing to react at room temperature, but is not limited thereto. At this time, a mixed solvent of THF and water may be used as a solvent, but is not limited thereto.

In the preparation method of the present disclosure, in order to increase purity and/or yield, the product after each reaction may be used as it is in the subsequent reaction, or additional steps of washing, separating, and/or purifying the product may be further performed, but are not limited thereto.

A third aspect of the present disclosure provides a pharmaceutical composition for preventing or treating a disease related to pyruvate dehydrogenase kinase 4 (hereinafter, referred to as PDK4) activity, comprising the compound or pharmaceutically acceptable salt thereof of the first aspect as an active ingredient.

As used herein, the terms "compound of the first aspect" and "pharmaceutically acceptable salt thereof" are as described above.

As used herein, the term "preventing" means any actions that inhibit or delay the occurrence, spread, and recurrence of PDK4 activity-related diseases through administration of the composition of the present disclosure, and the term "treating" means any actions that improve or beneficially change the symptoms of the diseases through administration of the composition of the present disclosure.

The pharmaceutical composition of the present disclosure may inhibit the PDK4 activity, thereby preventing or treating a disease related thereto.

As used herein, the term "pyruvate dehydrogenase kinase 4 (PDK4)" is a mitochondrial protein present in the matrix of mitochondria, and is one of four pyruvate dehydrogenase kinase isozymes. PDK4 is overexpressed in insulin-deficient or insulin-insensitive cells, and reduces metabolism, thereby preventing oxidation of pyruvate formed from glycolysis, and as a result, glucose in the blood is not consumed efficiently, which may induce hyperglycaemia.

Accordingly, PDK4 may be a target for type 2 diabetes treatment.

In this regard, the disease related to PDK4 activity, which may be prevented or treated with the pharmaceutical composition of the present disclosure, may be a metabolic disease, an inflammatory disease, or a cancer disease.

Specifically, the metabolic disease may be obesity, type 1 diabetes, type 2 diabetes, diabetic nephropathy, diabetic neuropathy, metabolic syndrome, hyperlipidemia, hyperlactatemia, arteriosclerosis, angina, acute kidney ischemic reperfusion injury, ischemic heart disease, myocardial infarction, heart failure, chemotherapy-induced cardiomyopathy, viral myocarditis, peripheral vascular disease, intermittent claudication, stroke, mitochondriopathy, pulmonary hypertension, fatty liver, hypertension, or Cushing's syndrome.

More specifically, the fatty liver may comprise alcoholic fatty liver diseases, non-alcoholic fatty liver diseases (NAFLDs), and non-alcoholic steatohepatitis (NASH).

Specifically, the inflammatory disease may be acute or chronic pancreatitis, inflammatory bowel disease, sepsis, graft-versus-host disease, or Charcot-Marie-Tooth disease.

Specifically, the cancer disease may be colorectal cancer, thyroid cancer, brain cancer, liver cancer, prostate cancer, breast cancer, pancreatic cancer, lung cancer, bladder cancer, or ovarian cancer.

However, the disease that may be prevented or treated using the pharmaceutical composition of the present disclosure is not limited thereto, and any disease for which prophylactic or therapeutic effect may be exhibited by inhibiting PDK4 activity may be comprised in the scope of the present disclosure.

Preferably, the pharmaceutical composition according to the present disclosure may comprise, as an active ingredient, the compound represented by Chemical Formula 1, a tautomer thereof, a stereoisomer thereof, a mixture of stereoisomers thereof, or a pharmaceutically acceptable salt thereof, in an amount of 0.1% by weight to 75% by weight, more preferably, in an amount of 1% by weight to 50% by weight, based on the total weight of the composition.

The composition of the present disclosure may further comprise a pharmaceutically acceptable carrier, diluent, or excipient, and may be formulated and used in various forms according to common methods according to each purpose for use, such as oral formulations such as powders, granules, tablets, capsules, suspensions, emulsions, syrups, aerosols, etc., and injectable formulation of sterile injection solutions, etc. The composition of the present disclosure may be administered through various routes comprising oral administration or intravenous, intraperitoneal, subcutaneous, rectal, topical administration, etc. Examples of suitable carriers, excipients, or diluents that may be comprised in such compositions may comprise lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, and mineral oil, etc. In addition, the composition of the present disclosure may further comprise a filler, an anti-coagulant, a lubricant, a wetting agent, a flavoring agent, an emulsifier, a preservative, etc.

Solid preparations for oral administration may comprise tablets, pills, powders, granules, capsules, etc. Such solid preparations may be formulated by mixing the composition with at least one or more excipients, such as starch, calcium carbonate, sucrose, lactose, gelatin, etc. Additionally, lubricants such as magnesium stearate and talc may be used, in addition to simple excipients.

Oral liquid preparations may be exemplified by suspensions, internal solutions, emulsions, syrups, etc., and may comprise various excipients, such as wetting agents, sweeteners, aromatics, preservatives, etc., in addition to simple diluents commonly used, such as water and liquid paraffin.

Preparations for parenteral administration may comprise sterilized aqueous solutions, non-aqueous solvents, suspensions, emulsions, freeze-drying preparations, and suppositories. Propylene glycol, polyethylene glycol, vegetable oils such as olive oil and injectable esters such as ethyl oleate may be used as non-aqueous solvents and suspensions. Witepsol, Macrogol, Tween 61, cacao butter, laurin butter, glycerogelatin, etc., may be used as a base for the suppository. Meanwhile, traditional additives such as solubilizers, isotonic agents, suspending agents, emulsifiers, stabilizers, preservatives, etc., may be comprised in the injectable formulation.

In this regard, the composition of the present disclosure may be administered in a pharmaceutically effective amount. As used herein, the term "pharmaceutically effective amount" means an amount that is sufficient to treat a disease at a reasonable benefit/risk ratio applicable to medical treatment and does not cause side effects. The effective dose level may be determined according to factors depending on the patient's health condition, the type of disease, severity, activity of the drug, sensitivity to the drug, method of administration, time of administration, route of administration and excretion rate, duration of treatment, drugs used in combination or concurrently and other factors well known in the medical field. The composition of the present disclosure may be administered as an individual therapeutic agent or in combination with other therapeutic agents, may be administered sequentially or simultaneously with existing therapeutic agents, and may be administered singly or multiple doses. Considering all of the above factors, it is important to administer an amount that may obtain the maximum effect with the minimum amount without side effects, which may be easily determined by those skilled in the art.

For example, as a dosage may increase or decrease depending on the route of administration, severity of disease, sex, weight, age, etc., the dosage does not limit the scope of the present disclosure in any way.

Specifically, the effective amount of the compound in the composition of the present disclosure may vary depending on a patient's age, sex, and body weight. In general, 1 to 100 mg, preferably, 5 mg to 60 mg per kg of body weight may be administered daily, every other day or divided into 1 to 3 times per day. However, as a dosage may increase or decrease depending on the route of administration, severity of disease, sex, body weight, age, etc., the dosage does not limit the scope of the present disclosure in any way.

A third aspect of the present disclosure provides a method of treating a disease related to PDK4 activity, comprising the step of administering the pharmaceutical composition of the second aspect to a subject in need thereof.

As used herein, the term "pharmaceutical composition of the second aspect" and "disease related to PDK4 activity" are as described above.

As used herein, the term "subject" means any animals, comprising monkeys, cows, horses, sheep, pigs, chickens, turkeys, quails, cats, dogs, mice, rats, rabbits, or guinea pigs, as well as humans who have developed or may develop the disease related to PDK4 activity. The diseases may be effectively prevented or treated by administering the pharmaceutical composition of the present disclosure to a subject. The pharmaceutical composition of the present disclosure may be administered in combination with existing therapeutic agents.

As used herein, the term "administering" means providing a predetermined substance to a patient by any suitable method. The administration route of the composition of the present disclosure may be administered through any general route as long as it may reach a target tissue. The composition of the present disclosure may be administered via intraperitoneal administration, intravenous administration, intramuscular administration, subcutaneous administration, intradermal administration, oral administration, topical administration, intranasal administration, intrapulmonary administration or intrarectal administration, but is not limited thereto. In addition, the pharmaceutical composition of the present disclosure may be administered by any device capable of transporting an active substance to a target cell. Preferred administration modes and preparations may be intravenous injections, subcutaneous injections, intradermal injections, intramuscular injections, drop injections, etc. Injections may be prepared using aqueous solvents such as physiological saline, Ringer's solution, etc., or non-aqueous solvents such as vegetable oil, higher fatty acid esters (e.g., ethyl oleate, etc.), alcohols (e.g., ethanol, benzyl alcohol, propylene glycol, glycerin, etc.), and may comprise a pharmaceutical carrier such as stabilizers for preventing deterioration (e.g., ascorbic acid, sodium hydrogensulfite, sodium pyrosulfite, BHA, tocopherol, EDTA, etc.), emulsifiers, buffers to control pH, preservatives to inhibit microbial growth (e.g., phenylmercuric nitrate, thimerosal, benzalkonium chloride, phenol, cresol, benzyl alcohol, etc.).

The term "therapeutically effective amount" used in combination with an active ingredient in the present disclosure means an effective amount of the fluorene derivative compound, a stereoisomer thereof, a mixture of stereoisomers thereof, or a pharmaceutically acceptable salt thereof for preventing or treating a target disease.

Depending on the type of diseases to be prevented or treated, the pharmaceutical composition of the present disclosure may further comprise known drugs used for preventing or treating each disease, in addition to the fluorene derivative compound, a stereoisomer thereof, a mixture of stereoisomers thereof, or a pharmaceutically acceptable salt thereof as an active ingredient. For example, the composition of the present disclosure may further comprise known drugs in addition to the fluorene derivative compound, a stereoisomer thereof, a mixture of stereoisomers thereof, or a pharmaceutically acceptable salt thereof as an active ingredient, when the composition is used for preventing or treating cancer diseases, and may be used in combination with other known therapies for treating these diseases.

## [Mode for Carrying Out the Invention]

Hereinafter, the present disclosure will be described in more detail with reference to exemplary embodiments. However, these exemplary embodiments are only for illustrating the present disclosure in more detail, and the scope of the present disclosure is not intended to be limited by these exemplary embodiments.

### Example 1: Synthesis of 2-(4-(2-fluoro-9-hydroxy-9-(trifluoromethyl)-9H-fluoren-4-yl)-1H-pyrazol-1-yl)-N'-(4-fluorophenyl)propanehydrazide(compound 1)

### Step 1-1: Preparation of ethyl 2'-chloro-4'-fluorobiphenyl-2-carboxylate

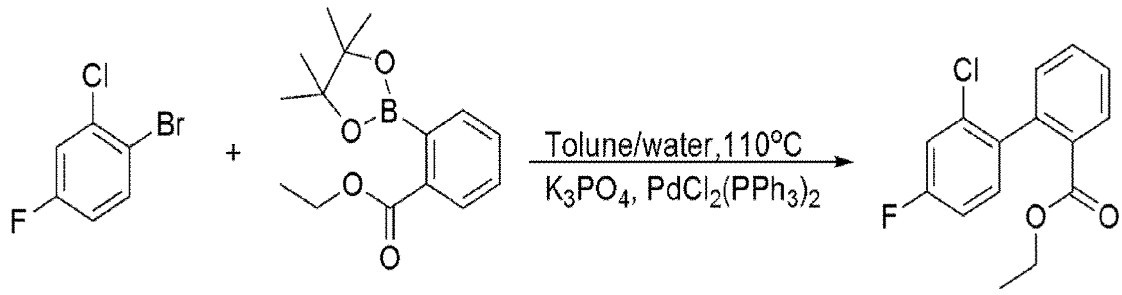


A mixture of 1-bromo-2-chloro-4-fluorobenzene (1 g, 4.775 mmol), ethyl 2-(4,4,5,5-tetramethyl-1,3,2-dioxaborolan-2-yl)benzoate (1.58 g, 5.73 mmol), $K_3PO_4$ (2.03 g, 9.55 mmol), and $PdCl_2(PPh_3)_2$ (67.03 mg, 0.095 mmol) in 10 mL of toluene:water (1:1) was heated at 110°C for 3 hours in a sealed tube. The solution was diluted with brine (200 mL) and extracted with ethyl acetate (EtOAC). The organic layer was dried over anhydrous $Na_2SO_4$ and concentrated under vacuum. The product was purified by column chromatography using ethyl acetate and hexane as solvents to obtain the title compound (1.1 g, 82%).

**Step 1-2: Preparation of 2'-chloro-4'-fluorobiphenyl-2-carboxylic acid**

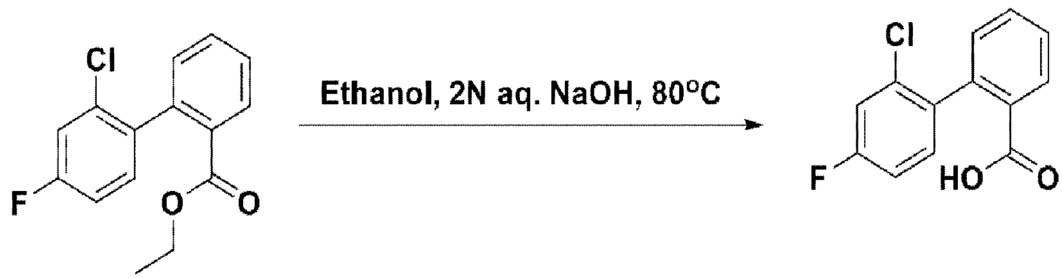


To a solution of ethyl 2'-chloro-4'-fluorobiphenyl-2-carboxylate (1.1 g, 9.67 mmol) dissolved in ethanol (150 mL), a 2 N aqueous NaOH solution (4.74 mL) was added. The reaction mixture was stirred at 80°C for 24 hours. Ethanol was removed under vacuum, and the resulting solution was acidified to pH 3 with 1 N hydrochloric acid. Water was further added (100 mL) and the aqueous solution was extracted with EtOAc (3 × 200 mL). The combined organic layer was washed with brine, dried over $Na_2SO_4$, and concentrated. The obtained crude product was used in the next step without further purification (870 mg, 87%).

**Step 1-3: Preparation of 4-chloro-2-fluoro-9H-fluoren-9-one**

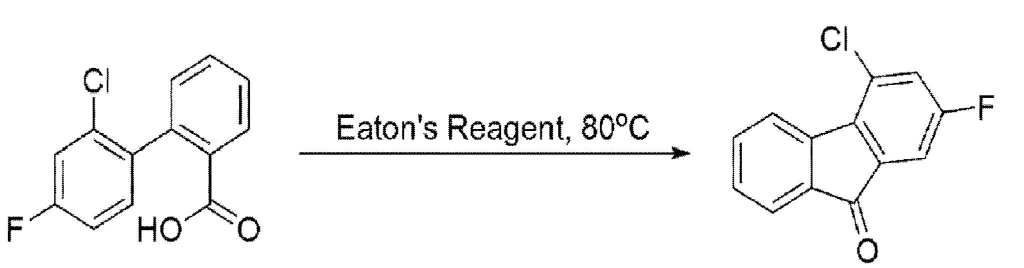


Ethyl 2'-chloro-4'-fluorobiphenyl-2-carboxylic acid (870 mg, 3.47 mmol) was added to Eaton's reagent (5 mL), and the mixture was stirred at 80°C for 2 hours. The reaction mixture was then cooled to 0°C. The solution was diluted with brine (50 mL) and extracted with ethyl acetate. The organic layer was dried over anhydrous $Na_2SO_4$ and concentrated under vacuum. The product was purified by column chromatography using ethyl acetate and hexane as solvents to obtain the title compound (626 mg, 77%).

**Step 1-4: Preparation of 4-chloro-2-fluoro-9-(trifluoromethyl)-9H-fluoren-9-ol**

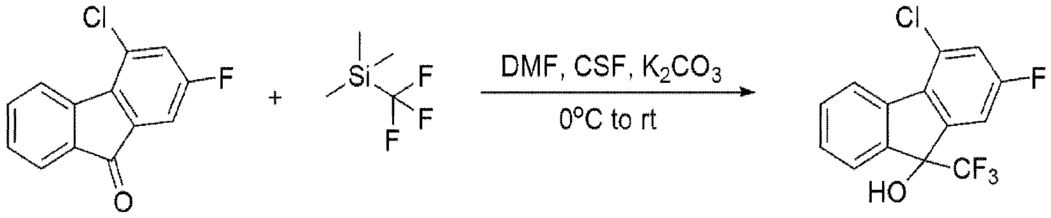


A mixture of 4-chloro-2-fluoro-9H-fluorene-9-one (2.9 g, 12.47 mmol), cesium fluoride (4.74 g, 31.17 mmol, 2.5 eq), and $K_2CO_3$ (5.62 g, 40.69 mmol, 2.5 eq) in DMF (12 mL) was cooled to 0°C under inert atmosphere. Trimethyl(trifluoromethyl)silane (4.6 mL, 31.17 mmol, 2.5 eq) was added dropwise while maintaining the temperature lower than 0°C. The resulting mixture was slowly warmed to room temperature and stirred for 24 hours. The reaction mixture was diluted with water (100 mL) and extracted with ethyl acetate (3 × 100 mL). The combined organic layer was washed with brine (100 mL), dried over $Na_2SO_4$, filtered, and volatiles evaporated under vacuum. The crude product was purified by column chromatography using ethyl acetate and hexane as solvents to obtain the title compound (3 g, 79%).

**Step 1-5: Preparation of ethyl 2-(4-(4,4,5,5-tetramethyl-1,3,2-dioxaborolan-2-yl)-1H-pyrazol-1-yl) propanoate**

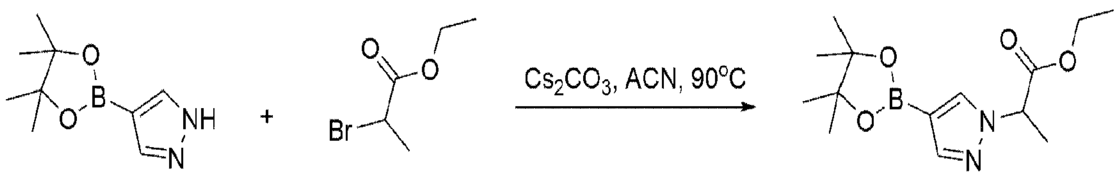


A mixture of 4-(4,4,5,5-tetramethyl-1,3,2-dioxaborolan-2-yl)-1H-pyrazole (0.40 g, 2.1 mmol), ethyl 2-bromopropanoate (290 μL, 2.3 mmol), and cesium carbonate (1.5 g, 4.6 mmol) in acetonitrile (8 mL) was stirred at 90°C for 4 hours. After cooling, the mixture was worked up with an aqueous $Na_2CO_3$ solution, extracted with ethyl acetate (3 × 50 mL), and washed with brine. The recovered organic layers were combined, dried over $Na_2SO_4$, filtered, and concentrated under reduced pressure. The residue was purified by column chromatography using ethyl acetate and hexane as solvents to obtain the title compound (420 mg, 61%).

**Step 1-6: Preparation of ethyl 2-(4-(2-fluoro-9-hydroxy-9-(trifluoromethyl)-9H-fluoren-4-yl)-1H-pyrazol-1-yl)propanoate**

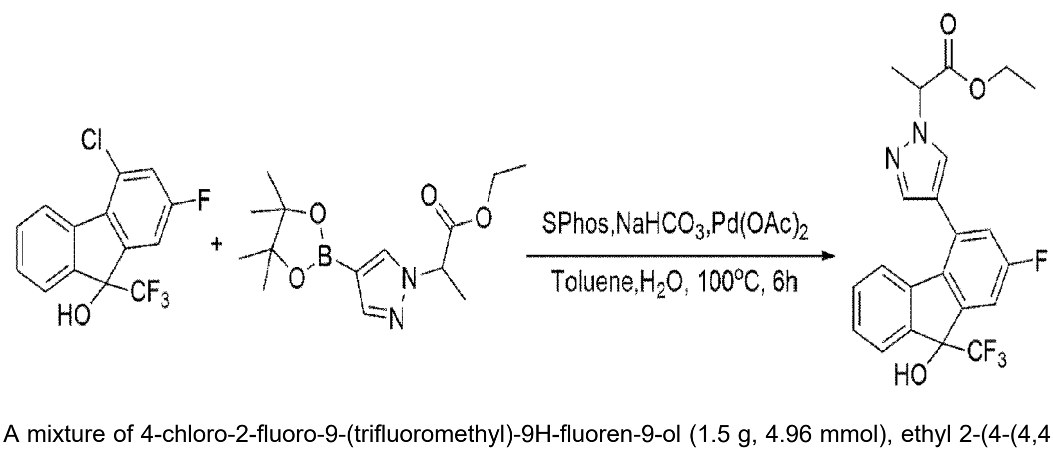


A mixture of 4-chloro-2-fluoro-9-(trifluoromethyl)-9H-fluoren-9-ol (1.5 g, 4.96 mmol), ethyl 2-(4-(4,4,5,5-tetramethyl-1,3,2-dioxaborolan-2-yl)-1H-pyrazol-1-yl)propanoate (2.9 g, 9.9 mmol, 2 eq), $NaHCO_3$ (1.25 g, 14.87 mmol),

Pd(OAc)$_2$ (111.27 mg, 0.496 mmol), and Sphos (610.4 mg, 1.487 mmol) in 30 mL of toluene:water (1:1) was heated at 100°C for 6 hours in a sealed tube. The solution was diluted with brine (200 mL) and extracted with ethyl acetate. The combined organic layer dried over anhydrous $Na_2SO_4$ and concentrated. The product was purified by column chromatography using ethyl acetate and hexane as solvents and used in the next step.

**Step 1-7: Preparation of 2-(4-(2-fluoro-9-hydroxy-9-(trifluoromethyl)-9H-fluoren-4-yl)-1H-pyrazol-1-yl)propanoic acid**

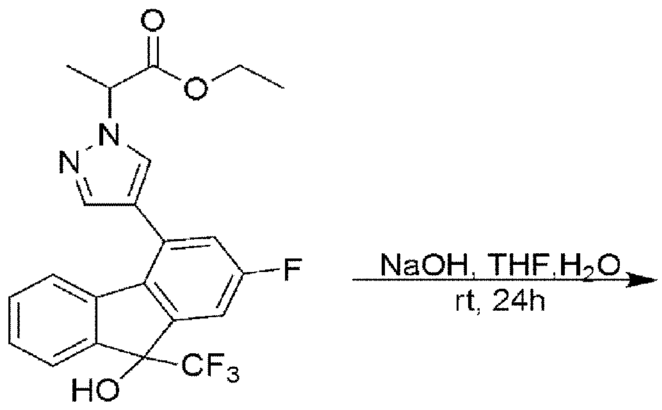


To a solution of ethyl 2-(4-(2-fluoro-9-hydroxy-9-(trifluoromethyl)-9H-fluoren-4-yl)-1H-pyrazol-1-yl)propanoate (2 g, 4.6 mmol) in THF/water (3:1, 100 mL), NaOH (736.67 g, 18.42 mmol) was added. The reaction mixture was stirred at room temperature for 24 hours. THF was removed under vacuum, and the resulting solution was acidified to pH 5 with 1 N hydrochloric acid. Water was further added (50 mL) and the aqueous solution was extracted with EtOAc (3 × 100 mL). The combined organic layer washed with brine, dried over $Na_2SO_4$, and concentrated. The crude product was purified by column chromatography using DCM and methanol as solvents to obtain the title compound (1.4 g, 74.8%).

**Step 1-8: Preparation of 2-(4-(2-fluoro-9-hydroxy-9-(trifluoromethyl)-9H-fluoren-4-yl)-1H-pyrazol-1-yl)-N'-(4-fluorophenyl)propanehydrazide**

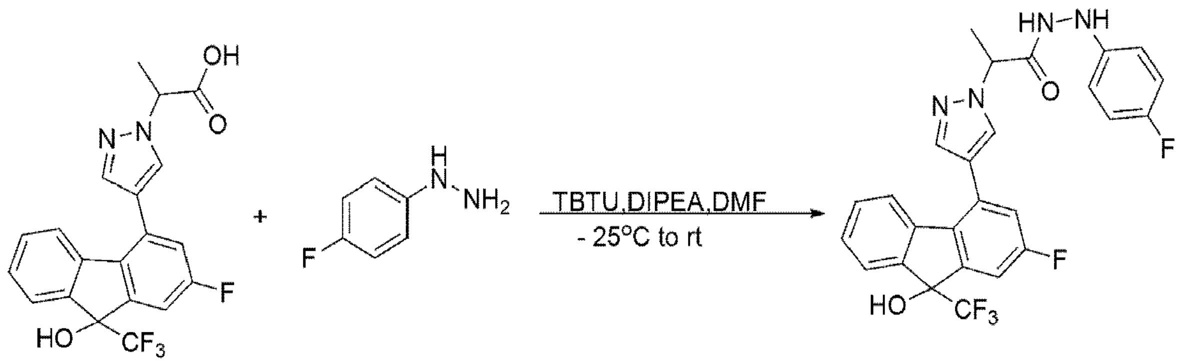


To a solution of 2-(4-(2-fluoro-9-hydroxy-9-(trifluoromethyl)-9H-fluoren-4-yl)-1H-pyrazol-1-yl)propanoic acid (500 mg, 1.23 mmol), (4-fluorophenyl)hydrazine (220 mg, 1.35 mmol), and TBTU (474.2 mg, 1.48 mmol) in DMF (3 mL), which was cooled to -25°C, N,N-diisopropylethylamine (DIPEA, 0.752 mL, 4.31 mmol) was added. The mixture was stirred at room temperature overnight. The reaction mixture was then poured into brine (50 mL) and extracted with EtOAc (3 × 50 mL). The combined organic layer washed successively with a 1 M aqueous $KHSO_4$ solution (50 mL), an aqueous $NaHCO_3$ solution (50 mL), and a brine solution (50 mL). After drying over $Na_2SO_4$, the solvents were evaporated under reduced pressure. The crude product was purified by column chromatography using ethyl acetate and hexane as solvents to obtain the title compound as a yellow solid (444 mg, 70%).

1H NMR (400 MHz, CDCl3): δ 8.47 (s, 1H), 7.73 (d, J = 1.83 Hz, 1H), 7.70 (s, 1H), 7.68 (d, J = 1.37 Hz, 2H), 7.42 (d,

J = 6.41 Hz, 1H), 7.32 - 7.24 (m, 1H), 7.22 - 7.14 ( m, 2H), 6.99 (dd, J = 9.62, 2.29 Hz, 1H), 6.90 (t, J = 8.70 Hz, 2H), 6.69 (dd, J = 8.42 , 4.12 Hz, 2H), 6.04 (s, 1H), 5.08 (q, J = 3.66 Hz, 1H), 1.89 (d, J = 6.87 Hz, 3H).

**Example 2: Preparation of N'-(4-fluorophenyl)-2-(4-(9-hydroxy-2-methoxy-9-(trifluoromethyl)-9H-fluoren-4-yl)-1H-pyrazol-1-yl)propanehydrazide**

The title compound was obtained in a similar manner as in Example 1, except that 1-bromo-2-chloro-4-methoxybenzene was used instead of 1-bromo-2-chloro-4-fluorobenzene in the step 1-1 of Example 1.

1H NMR (400 MHz, CDCl3): δ 8.49 (s, 1H), 7.75 (d, J = 3.66 Hz, 1H), 7.70 - 7.63 (m, 2H), 7.30 - 7.10 (m, 5H), 6.90 (t, J = 8.54 Hz, 2H), 6.78 (d, J = 2.44 Hz, 1H), 6.71 (dd, J = 8.20, 4.27 Hz, 2H), 6.05 (s, 1H), 5.09 (q, J = 7.17, 3.66 Hz, 1H), 3.87 (s, 3H), 1.91 (d, J = 7.32 Hz, 3H).

**Example 3: Preparation of N'-(2-fluoro-4-(methylsulfonyl)phenyl)-2-(4-(2-fluoro-9-hydroxy-9-(trifluoromethyl)-9H-fluoren-4-yl)-1H-pyrazol-1-yl)propanehydrazide**

The title compound was obtained in a similar manner as in Example 1, except that (2-fluoro-4-(methylsulfonyl) phenyl)hydrazine) was used instead of (4-fluorophenyl)hydrazine in the step 1-8 of Example 1.

1H NMR (400 MHz, DMSO-d6): δ 10.45 (s, 1H), 8.78 (s, 1H), 8.21 (d, J = 9.39 Hz, 1H), 7.76 (d, J = 2.75 Hz, 1H), 7.66 - 7.59 (m, 2H), 7.56 - 7.48 (m, 1H), 7.41 (d, J = 6.87 Hz, 2H), 7.39 - 7.26 (m, 2H), 7.25 - 7.16 (m, 2H), 6.95 - 6.85 (m, 1H), 5.23 (q, J = 7.10 Hz, 1H), 3.09 (s, 3H), 1.75 (dd, J = 6.98 , 2.29 Hz, 3H).

**Example 4: Preparation of 2-(4-(2-fluoro-9-hydroxy-9-(trifluoromethyl)-9H-fluoren-4-yl)-1H-pyrazol-1-yl)-N'-phenylpropanehydrazide**

The title compound was obtained in a similar manner as in Example 1, except that phenylhydrazine was used instead of (4-fluorophenyl)hydrazine in the step 1-8 of Example 1.

1H NMR (400 MHz, CDCl3): δ 8.82 (d, J = 6.10 Hz, 1H), 8.41 - 8.35 (m, 2H), 7.94 (s, 1H), 7.74 - 7.62 (m, 2H), 7.42 (t, J = 6.87 Hz, 1H), 7.30 - 7.10 (m, 5H), 6.99 - 6.91 (m, 1H), 6.88 (t, J = 7.63 Hz, 1H), 6.72 (d, J = 8.39 Hz, 1H), 6.21 (s, 1H), 5.12 (q, J = 6.87 Hz, 1H), 1.86 (d, J = 6.87 Hz, 3H).

**Example 5: Preparation of 2-(4-(2-fluoro-9-hydroxy-9-(trifluoromethyl)-9H-fluoren-4-yl)-1H-pyrazol-1-yl)-N'-(naphthalen-1-yl)propanehydrazide**

The title compound was obtained in a similar manner as in Example 1, except that naphthalen-1-ylhydrazine was used instead of (4-fluorophenyl)hydrazine in the step 1-8 of Example 1.

1H NMR (400 MHz, CDCl3): δ 8.76 (d, J = 3.81 Hz, 1H), 7.83 (t, J = 6.87 Hz, 1H), 7.78 (d, J = 7.63 Hz, 1H), 7.70 - 7.53 (m, 3H), 7.52 - 7.33 (m, 4H), 7.26 - 7.00 (m, 5H), 6.94 - 6.80 (m, 2H), 6.65 (d, J = 7.63 Hz, 1H), 5.09 (q, J = 7.63 Hz, 1H), 1.83 (d, J = 6.87 Hz, 3H).

**Example 6: Preparation of N'-(2-chlorophenyl)-2-(4-(2-fluoro-9-hydroxy-9-(trifluoromethyl)-9H-fluoren-4-yl)-1H-pyrazol-1-yl)propanehydrazide**

The title compound was obtained in a similar manner as in Example 1, except that (2-chlorophenyl)hydrazine was used instead of (4-fluorophenyl)hydrazine in the step 1-8 of Example 1.

1H NMR (400 MHz, DMSO-d6): δ 10.31 (s, 1H), 8.19 (d, J = 9.62 Hz, 1H), 7.94 (s, 1H), 7.74 (s, 1H), 7.67 (s, 1H), 7.63 (d, J = 5.50 Hz, 1H), 7.48 - 7.08 (m, 6H), 6.81 - 6.70 (m, 3H), 5.26 (q, J = 6.41 Hz, 1H), 1.78 (d, J = 6.41 Hz, 3H).

**Example 7: Preparation of N'-(4-chlorophenyl)-2-(4-(2-fluoro-9-hydroxy-9-(trifluoromethyl)-9H-fluoren-4-yl)-1H-pyrazol-1-yl)propanehydrazide**

The title compound was obtained in a similar manner as in Example 1, except that (4-chlorophenyl)hydrazine was used instead of (4-fluorophenyl)hydrazine in the step 1-8 of Example 1.

1H NMR (400 MHz, DMSO-d6): δ 10.20 (s, 1H), 8.18 (d, J = 8.24 Hz, 1H), 8.15 - 8.10 (m, 1H), 7.95 (s, 1H), 7.73 (d, J = 3.21 Hz, 1H), 7.63 (d, J = 6.41 Hz, 1H), 7.46 - 7.12 (m, 8H), 6.72 (d, J = 8.70 Hz, 1H), 5.22 (q, J = 6.41 Hz, 1H), 1.76 (dd, J = 7.33, 3.66 Hz, 3H).

**Example 8: Preparation of 2-(4-(2-fluoro-9-hydroxy-9-(trifluoromethyl)-9H-fluoren-4-yl)-1H-pyrazol-1-yl)-N'-(pyridin-2-yl)propanehydrazide**

The title compound was obtained in a similar manner as in Example 1, except that 2-hydrazinylpyridine was used instead of (4-fluorophenyl)hydrazine in the step 1-8 of Example 1.

1H NMR (400 MHz, DMSO-d6): δ 10.23 (s, 1H), 8.48 (s, 1H), 8.21(d, J = 8.70 Hz, 1H), 8.03 (s, 1H), 7.72 (s, 1H), 7.63 (d, J = 7.10 Hz, 1H), 7.50 (t, J = 8.01 Hz, 1H), 7.40 (d, J = 6.87 Hz, 2H), 7.37 - 7.15 (m, 4H), 6.69 (t, J = 5.95 Hz, 1H), 6.57 (d, J = 8.24 Hz, 1H), 5.25 (q, J = 7.33 Hz, 1H), 1.77 (d, J = 5.50 Hz, 3H).

**Example 9: Preparation of N'-(2-fluoro-4-(methylsulfonyl)phenyl)-2-(4-(9-hydroxy-2-methoxy-9-(trifluoromethyl)-9H-fluoren-4-yl)-1H-pyrazol-1-yl)propanehydrazide**

The title compound was obtained in a similar manner as in Example 1, except that 1-bromo-2-chloro-4-methoxybenzene was used instead of 1-bromo-2-chloro-4-fluorobenzene in the step 1-1 of Example 1, and (2-fluoro-4-(methylsulfonyl)phenyl)hydrazine was used instead of (4-fluorophenyl)hydrazine in the step 1-8.

1H NMR (400 MHz, DMSO-d6): δ 10.45 (s, 1H), 8.78 (s, 1H), 8.15 (d, J = 7.33 Hz, 1H), 7.95 (s, 1H), 7.72 (s, 1H), 7.62 (dd, J = 10.99, 1.83 Hz, 1H), 7.58 (d, J = 5.95 Hz, 1H), 7.55 - 7.49 (m, 1H), 7.31 - 7.21 (m,2H), 7.19 - 7.11 (m,2H), 6.95 - 6.87 (m,1H), 6.85 (d, J = 2.29 Hz, 1H), 5.26 (q, J = 6.87 Hz, 1H), 3.83 (s, 3H), 3.13 (s, 3H), 1.79 (dd, J = 6.87,2.75 Hz, 3H).

**Example 10: Preparation of N'-(4-chlorophenyl)-2-(4-(9-hydroxy-2-methoxy-9-(trifluoromethyl)-9H-fluoren-4-yl)-1H-pyrazol-1-yl)propanehydrazide**

The title compound was obtained in a similar manner as in Example 1, except that 1-bromo-2-chloro-4-methoxybenzene was used instead of 1-bromo-2-chloro-4-fluorobenzene in the step 1-1 of Example 1, and (4-chlorophenyl)hydrazine was used instead of (4-fluorophenyl)hydrazine in the step 1-8.

1H NMR (400 MHz, DMSO-d6): δ 10.19 (s, 1H), 9.64 (s, 1H), 8.11 (d, J = 5.04 Hz, 2H), 7.69 (s, 1H), 7.58 (d, J = 5.95 Hz, 1H), 7.31 - 7.12 (m, 5H), 6.85 (d, J = 2.29 Hz, 1H), 6.77 - 6.75 (m, 2H), 5.21 (q, J = 6.87 Hz, 1H), 3.83 (s, 3H), 1.72 (dd, J = 7.10, 3.21 Hz, 3H).

**Example 11: Preparation of 2-(4-(9-hydroxy-2-methoxy-9-(trifluoromethyl)-9H-fluoren-4-yl)-1H-pyrazol-1-yl)-N'-(naphthalen-1-yl)propanehydrazide**

The title compound was obtained in a similar manner as in Example 1, except that 1-bromo-2-chloro-4-methoxybenzene was used instead of 1-bromo-2-chloro-4-fluorobenzene in the step 1-1 of Example 1, and naphthalen-1-ylhydrazine was used instead of (4-fluorophenyl)hydrazine in the step 1-8.

1H NMR (400 MHz, DMSO-d6): δ 10.28 (s, 1H), 8.41 (s, 1H), 8.15 (d, J = 8.24 Hz, 2H), 7.83 - 7.75 (m, 1H), 7.69 (s, 1H), 7.54 (d, J = 5.04 Hz, 1H), 7.50 - 7.36 (m, 2H), 7.32 - 7.05 (m, 7H), 6.82 (d, J = 2.29 Hz, 1H), 6.72 - 6.62 (m, 1H), 5.30 (q, J = 6.87 Hz, 1H), 3.80 (s, 3H), 1.79 (dd, J = 6.87 , 2.75 Hz, 3H).

**Example 12: Preparation of N'-(2-chlorophenyl)-2-(4-(9-hydroxy-2-methoxy-9-(trifluoromethyl)-9H-fluoren-4-yl)-1H-pyrazol-1-yl)propanehydrazide**

The title compound was obtained in a similar manner as in Example 1, except that 1-bromo-2-chloro-4-methoxybenzene was used instead of 1-bromo-2-chloro-4-fluorobenzene in the step 1-1 of Example 1, and (2-chlorophenyl)hydrazine was used instead of (4-fluorophenyl)hydrazine in the step 1-8.

1H NMR (400 MHz, DMSO-d6): δ 10.30 (s, 1H), 9.77 (s, 1H), 8.13 (d, J = 6.87 Hz, 1H), 7.95 (s, 1H), 7.68 (d, J = 15.11 Hz, 1H), 7.58 (d, J = 6.41 Hz, 1H), 7.36 (s, 1H), 7.33 - 7.09 (m, 5H), 6.85 (d, J = 2.29 Hz, 1H), 6.82 - 6.70 (m, 2H), 5.25 (q, J = 6.87 Hz, 1H), 3.83 (s, 3H), 1.74 (dd, J = 7.10, 3.18 Hz, 3H).

**Example 13: Preparation of 2-(4-(2-fluoro-9-hydroxy-9-(trifluoromethyl)-9H-fluoren-4-yl)-1H-pyrazol-1-yl)-N'-(2-fluorophenyl)propanehydrazide**

The title compound was obtained in a similar manner as in Example 1, except that (2-fluorophenyl)hydrazine was used instead of (4-fluorophenyl)hydrazine in the step 1-8 of Example 1.

1H NMR (400 MHz, CDCl3): δ 8.51 (s, 1H), 8.00 (s, 1H), 7.83 - 7.77 (m, 1H), 7.75 - 7.65 (m, 2H), 7.44 (d, J = 7.63 Hz, 1H), 7.34 - 7.16 (m, 3H), 7.08 - 6.92 (m,3H), 6.90 - 6.80 (m, 1H), 6.73 (t, J = 6.87 Hz, 1H), 6.26 (s, 1H), 5.13 (q, J = 6.87 Hz, 1H), 1.94 (d, J = 7.63 Hz, 3H).

**Example 14: Preparation of 6-(2-(2-(4-(2-fluoro-9-hydroxy-9-(trifluoromethyl)-9H-fluoren-4-yl)-1H-pyrazol-1-yl) propanoyl)hydrazinyl)nicotinic acid**

The title compound was obtained in a similar manner as in Example 1, except that 6-hydrazinylnicotinic acid was used instead of (4-fluorophenyl)hydrazine in the step 1-8 of Example 1.
LCMS [M+H] 542.1.

**Example 15: Preparation of tert-butyl 6-(2-(2-(4-(9-hydroxy-2-methoxy-9-(trifluoromethyl)-9H-fluoren-4-yl)-1H-pyrazol-1-yl)propanoyl)hydrazinyl)nicotinate**

The title compound was obtained in a similar manner as in Example 1, except that 1-bromo-2-chloro-4-methoxybenzene was used instead of 1-bromo-2-chloro-4-fluorobenzene in the step 1-1 of Example 1, and tert-butyl 6-hydrazinylnicotinate was used instead of (4-fluorophenyl)hydrazine in the step 1-8.
LCMS [M+H] 610.2.

**Example 16: Preparation of 6-(2-(2-(4-(9-hydroxy-2-methoxy-9-(trifluoromethyl)-9H-fluoren-4-yl)-1H-pyrazol-1-yl)propanoyl)hydrazinyl)nicotinic acid**

The title compound was obtained in a similar manner as in Example 1, except that 1-bromo-2-chloro-4-methoxybenzene was used instead of 1-bromo-2-chloro-4-fluorobenzene in the step 1-1 of Example 1, and 6-hydrazinylnicotinic acid was used instead of (4-fluorophenyl)hydrazine in the step 1-8.
LCMS [M+H] 554.14.

**Example 17: Preparation of 2-(4-(9-hydroxy-2-methoxy-9-(trifluoromethyl)-9H-fluoren-4-yl)-1H-pyrazol-1-yl)-N'-(pyridin-2-yl)propanehydrazide**

The title compound was obtained in a similar manner as in Example 1, except that 1-bromo-2-chloro-4-methoxybenzene was used instead of 1-bromo-2-chloro-4-fluorobenzene in the step 1-1 of Example 1, and 2-hydrazinylpyridine was used instead of (4-fluorophenyl)hydrazine in the step 1-8.
LCMS [M+H] 510.2.

**Example 18: Preparation of 2-(4-(2-fluoro-9-hydroxy-9-(trifluoromethyl)-9H-fluoren-4-yl)-1H-pyrazol-1-yl)-N'-(pyridin-2-yl)acetohydrazide**

The title compound was obtained in a similar manner as in Example 1, except that ethyl 2-bromoacetate was used instead of ethyl 2-bromopropanoate in the step 1-5 of Example 1, and 2-hydrazinylpyridine was used instead of (4-fluorophenyl)hydrazine in the step 1-8.
LCMS [M+H] 484.1.

**Example 19: Preparation of tert-butyl 6-(2-(2-(4-(2-fluoro-9-hydroxy-9-(trifluoromethyl)-9H-fluoren-4-yl)-1H-pyrazol-1-yl)acetyl)hydrazinyl)nicotinate**

The title compound was obtained in a similar manner as in Example 1, except that ethyl 2-bromoacetate was used instead of ethyl 2-bromopropanoate in the step 1-5 of Example 1, and tert-butyl 6-hydrazinylnicotinate was used instead of (4-fluorophenyl)hydrazine in the step 1-8.
LCMS [M+H] 584.2.

**Example 20: Preparation of 6-(2-(2-(4-(2-fluoro-9-hydroxy-9-(trifluoromethyl)-9H-fluoren-4-yl)-1H-pyrazol-1-yl) acetyl)hydrazinyl)nicotinic acid**

The title compound was obtained in a similar manner as in Example 1, except that ethyl 2-bromoacetate was used instead of ethyl 2-bromopropanoate in the step 1-5 of Example 1, and 6-hydrazinylnicotinic acid was used instead of (4-fluorophenyl)hydrazine in the step 1-8.
LCMS [M+H] 528.1.

**Example 21: Preparation of 2-(4-(2-fluoro-9-hydroxy-9-(trifluoromethyl)-9H-fluoren-4-yl)-1H-pyrazol-1-yl)-N'-(4-fluorophenyl)acetohydrazide**

The title compound was obtained in a similar manner as in Example 1, except that ethyl 2-bromoacetate was used instead of ethyl 2-bromopropanoate in the step 1-5 of Example 1.
LCMS [M+H] 501.1.

**Example 22: Preparation of 2-(4-(2-fluoro-9-hydroxy-9-(trifluoromethyl)-9H-fluoren-4-yl)-1H-pyrazol-1-yl)-N'-(4-(methylsulfonyl)phenyl)propanehydrazide**

The title compound was obtained in a similar manner as in Example 1, except that (4-(methylsulfonyl)phenyl) hydrazine was used instead of (4-fluorophenyl)hydrazine in the step 1-8 of Example 1.
LCMS [M+H] 575.1.

**Example 23: Preparation of 2-(4-(9-hydroxy-2-methoxy-9-(trifluoromethyl)-9H-fluoren-4-yl)-1H-pyrazol-1-yl)-N'-(4-(methylsulfonyl)phenyl)propanehydrazide**

The title compound was obtained in a similar manner as in Example 1, except that 1-bromo-2-chloro-4-methoxybenzene was used instead of 1-bromo-2-chloro-4-fluorobenzene in the step 1-1 of Example 1, and (4-(methylsulfonyl)phenyl)hydrazine was used instead of (4-fluorophenyl)hydrazine in the step 1-8.
LCMS [M+H] 587.2.

**Example 24: Preparation of 2-(4-(2-fluoro-9-hydroxy-9-(trifluoromethyl)-9H-fluoren-4-yl)-1H-pyrazol-1-yl)-N'-(pyrazin-2-yl)propanehydrazide**

The title compound was obtained in a similar manner as in Example 1, except that 2-hydrazinylpyrazine was used instead of (4-fluorophenyl)hydrazine in the step 1-8 of Example 1.
LCMS [M+H]499.4.

**Example 25: Preparation of 2-(4-(9-hydroxy-2-methoxy-9-(trifluoromethyl)-9H-fluoren-4-yl)-1H-pyrazol-1-yl)-N'-(pyrazin-2-yl)propanehydrazide**

The title compound was obtained in a similar manner as in Example 1, except that 1-bromo-2-chloro-4-methoxybenzene was used instead of 1-bromo-2-chloro-4-fluorobenzene in the step 1-1 of Example 1, and 2-hydrazinylpyrazine was used instead of (4-fluorophenyl)hydrazine in the step 1-8.
LCMS [M+H]511.4.

**Example 26: Preparation of 2-(4-(2-fluoro-9-hydroxy-9-(trifluoromethyl)-9H-fluoren-4-yl)-1H-pyrazol-1-yl)-N'-(5-(trifluoromethyl)pyridin-2-yl)propanehydrazide**

The title compound was obtained in a similar manner as in Example 1, except that 2-hydrazinyl-5-(trifluoromethyl)pyrazine was used instead of (4-fluorophenyl)hydrazine in the step 1-8 of Example 1.
LCMS [M+H]566.4.

**Example 27: Preparation of 2-(4-(9-hydroxy-2-methoxy-9-(trifluoromethyl)-9H-fluoren-4-yl)-1H-pyrazol-1-yl)-N'-(5-(trifluoromethyl)pyridin-2-yl)propanehydrazide**

The title compound was obtained in a similar manner as in Example 1, except that 1-bromo-2-chloro-4-methoxybenzene was used instead of 1-bromo-2-chloro-4-fluorobenzene in the step 1-1 of Example 1, and 2-hydrazinyl-5-(trifluoromethyl)pyrazine was used instead of (4-fluorophenyl)hydrazine in the step 1-8.
LCMS [M+H]578.5.

**Example 28: Preparation of 2-(4-(2-fluoro-9-hydroxy-9-(trifluoromethyl)-9H-fluoren-4-yl)-1H-pyrazol-1-yl)-N'-(pyrimidin-2-yl)propanehydrazide**

The title compound was obtained in a similar manner as in Example 1, except that 2-hydrazinylpyrimidine was used instead of (4-fluorophenyl)hydrazine in the step 1-8 of Example 1.
LCMS [M+H]499.4.

**Example 29: Preparation of 2-(4-(9-hydroxy-2-methoxy-9-(trifluoromethyl)-9H-fluoren-4-yl)-1H-pyrazol-1-yl)-N'-(pyrimidin-2-yl)propanehydrazide**

The title compound was obtained in a similar manner as in Example 1, except that 1-bromo-2-chloro-4-methoxybenzene was used instead of 1-bromo-2-chloro-4-fluorobenzene in the step 1-1 of Example 1, and 2-hydrazinylpyrimidine was used instead of (4-fluorophenyl)hydrazine in the step 1-8.

LCMS [M+H]511.4.

**Example 30: Preparation of 2-(4-(2-fluoro-9-hydroxy-9-(trifluoromethyl)-9H-fluoren-4-yl)-1H-pyrazol-1-yl)-N'-(pyridin-3-yl)propanehydrazide**

The title compound was obtained in a similar manner as in Example 1, except that 3-hydrazinylpyridine was used instead of (4-fluorophenyl)hydrazine in the step 1-8 of Example 1.

LCMS [M+H]498.4.

**Example 31: Preparation of 2-(4-(9-hydroxy-2-methoxy-9-(trifluoromethyl)-9H-fluoren-4-yl)-1H-pyrazol-1-yl)-N'-(pyridin-3-yl)propanehydrazide**

The title compound was obtained in a similar manner as in Example 1, except that 1-bromo-2-chloro-4-methoxybenzene was used instead of 1-bromo-2-chloro-4-fluorobenzene in the step 1-1 of Example 1, and 3-hydrazinylpyridine was used instead of (4-fluorophenyl)hydrazine in the step 1-8.

LCMS [M+H]510.3.

**Example 32: Preparation of N'-(3-chloro-5-(trifluoromethyl)pyridin-2-yl)-2-(4-(2-fluoro-9-hydroxy-9-(trifluoromethyl)-9H-fluoren-4-yl)-1H-pyrazol-1-yl)propanehydrazide**

The title compound was obtained in a similar manner as in Example 1, except that 3-chloro-2-hydrazinyl-5-(trifluoromethyl)pyridine was used instead of (4-fluorophenyl)hydrazine in the step 1-8 of Example 1.

LCMS [M+H]600.7.

**Example 33: Preparation of N'-(3-chloro-5-(trifluoromethyl)pyridin-2-yl)-2-(4-(9-hydroxy-2-methoxy-9-(trifluoromethyl)-9H-fluoren-4-yl)-1H-pyrazol-1-yl)propanehydrazide**

The title compound was obtained in a similar manner as in Example 1, except that 1-bromo-2-chloro-4-methoxybenzene was used instead of 1-bromo-2-chloro-4-fluorobenzene in the step 1-1 of Example 1, and 3-chloro-2-hydrazinyl-5-(trifluoromethyl)pyridine was used instead of (4-fluorophenyl)hydrazine in the step 1-8.

LCMS [M+H]612.8.

**Example 34: Preparation of 2-(4-(2-fluoro-9-hydroxy-9-(trifluoromethyl)-9H-fluoren-4-yl)-1H-pyrazol-1-yl)-N'-(pyridin-4-yl)propanehydrazide**

The title compound was obtained in a similar manner as in Example 1, except that 4-hydrazinylpyridine was used instead of (4-fluorophenyl)hydrazine in the step 1-8 of Example 1.

LCMS [M+H]498.4.

**Example 35: Preparation of 2-(4-(2-fluoro-9-hydroxy-9-(trifluoromethyl)-9H-fluoren-4-yl)-1H-pyrazol-1-yl)-N'-(quinazolin-4-yl)propanehydrazide**

The title compound was obtained in a similar manner as in Example 1, except that 4-hydrazinylquinazoline was used instead of (4-fluorophenyl)hydrazine in the step 1-8 of Example 1.

LCMS [M+H]549.2.

**Example 36: Preparation of 2-(4-(2-fluoro-9-hydroxy-9-(trifluoromethyl)-9H-fluoren-4-yl)-1H-pyrazol-1-yl)-N'-(quinolin-4-yl)propanehydrazide**

The title compound was obtained in a similar manner as in Example 1, except that 4-hydrazinylquinoline was used instead of (4-fluorophenyl)hydrazine in the step 1-8 of Example 1.

LCMS [M+H]548.4.

**Example 37: Preparation of 2-(4-(2-fluoro-9-hydroxy-9-(trifluoromethyl)-9H-fluoren-4-yl)-1H-pyrazol-1-yl)-N'-(quinoxalin-2-yl)propanehydrazide**

The title compound was obtained in a similar manner as in Example 1, except that 2-hydrazinylquinoxaline was used instead of (4-fluorophenyl)hydrazine in the step 1-8 of Example 1.
LCMS [M+H]549.5.

**Example 38: Preparation of 2-(4-(2-fluoro-9-hydroxy-9-(trifluoromethyl)-9H-fluoren-4-yl)-1H-pyrazol-1-yl)-N'-(2-oxo-1,2-dihydroquinolin-3-yl)propanehydrazide**

The title compound was obtained in a similar manner as in Example 1, except that 3-hydrazinylquinolin-2(1H)-one was used instead of (4-fluorophenyl)hydrazine in the step 1-8 of Example 1.
LCMS [M+H] 564.5.

**Example 39: Preparation of 2-(4-(2-fluoro-9-hydroxy-9-(trifluoromethyl)-9H-fluoren-4-yl)-1H-pyrazol-1-yl)-N'-(quinolin-2-yl)propanehydrazide**

The title compound was obtained in a similar manner as in Example 1, except that 2-hydrazinylquinoline was used instead of (4-fluorophenyl)hydrazine in the step 1-8 of Example 1.
LCMS [M+H]548.4.

**Example 40: Preparation of 2-(4-(2-fluoro-9-hydroxy-9-(trifluoromethyl)-9H-fluoren-4-yl)-1H-pyrazol-1-yl)-N'-(1-methyl-2-oxo-1,2-dihydroquinolin-4-yl)propanehydrazide**

The title compound was obtained in a similar manner as in Example 1, except that 4-hydrazinyl-1-methylquinolin-2(1H)-one was used instead of (4-fluorophenyl)hydrazine in the step 1-8 of Example 1.
LCMS [M+H]578.3.

**Example 41: Preparation of 2-(4-(2-fluoro-9-hydroxy-9-(trifluoromethyl)-9H-fluoren-4-yl)-1H-pyrazol-1-yl)-N'-(imidazo[1,2-b]pyridazin-6-yl)propanehydrazide**

The title compound was obtained in a similar manner as in Example 1, except that 6-hydrazinylimidazo[1,2-b] pyridazine was used instead of (4-fluorophenyl)hydrazine in the step 1-8 of Example 1.
LCMS [M+H]538.4.

**Example 42: Preparation of N'-(benzo[d]thiazol-2-yl)-2-(4-(2-fluoro-9-hydroxy-9-(trifluoromethyl)-9H-fluoren-4-yl)-1H-pyrazol-1-yl)propanehydrazide**

LCMS [M+H]554.4.

**Example 43: Preparation** of **2-(4-(2-fluoro-9-hydroxy-9-(trifluoromethyl)-9H-fluoren-4-yl)-1H-pyrazol-1-yl)-N'-(4-fluorophenyl)butanehydrazide**

The title compound was obtained in a similar manner as in Example 1, except that ethyl 2-bromobutanoate was used instead of ethyl 2-bromopropanoate in the step 1-5 of Example 1.
LCMS [M+H]529.4.

**Example 44: Preparation of 2-(4-(2-fluoro-9-hydroxy-9-(trifluoromethyl)-9H-fluoren-4-yl)-1H-pyrazol-1-yl)-N'-(4-fluorophenyl)-3-methylbutanehydrazide**

The title compound was obtained in a similar manner as in Example 1, except that ethyl 2-bromo-3-methylbutanoate was used instead of ethyl 2-bromopropanoate in the step 1-5 of Example 1.
LCMS [M+H]543.5.

**Example 45: Preparation of 2-(4-(2-fluoro-9-hydroxy-9-(trifluoromethyl)-9H-fluoren-4-yl)-1H-pyrazol-1-yl)-N'-(4-fluorophenyl)-4-methylpentanehydrazide**

The title compound was obtained in a similar manner as in Example 1, except that ethyl 2-bromo-4-methylpentanoate was used instead of ethyl 2-bromopropanoate in the step 1-5 of Example 1.
LCMS [M+H]557.5.

**Example 46: Preparation of 3-cyclohexyl-2-(4-(2-fluoro-9-hydroxy-9-(trifluoromethyl)-9H-fluoren-4-yl)-1H-pyrazol-1-yl)-N'-(4-fluorophenyl)propanehydrazide**

The title compound was obtained in a similar manner as in Example 1, except that ethyl 2-bromo-3-cyclohexylpropanoate was used instead of ethyl 2-bromopropanoate in the step 1-5 of Example 1.
LCMS [M+H]597.6.

**Example 47: Preparation of 2-(4-(2-fluoro-9-hydroxy-9-(trifluoromethyl)-9H-fluoren-4-yl)-1H-pyrazol-1-yl)-N'-(4-fluorophenyl)-2-methylpropanehydrazide**

The title compound was obtained in a similar manner as in Example 1, except that ethyl 2-bromo-2-methylpropanoate was used instead of ethyl 2-bromopropanoate in the step 1-5 of Example 1.
LCMS [M+H]529.4.

**Example 48: Preparation of (S)-2-(4-((S)-2-fluoro-9-hydroxy-9-(trifluoromethyl)-9H-fluoren-4-yl)-1H-pyrazol-1-yl)-N'-(4-fluorophenyl)propanehydrazide**

LCMS [M+H]515.46.

**Example 49: Preparation of (R)-2-(4-((R)-2-fluoro-9-hydroxy-9-(trifluoromethyl)-9H-fluoren-4-yl)-1H-pyrazol-1-yl)-N'-(4-fluorophenyl)propanehydrazide**

LCMS [M+H]515.46.

**Example 50: Preparation of (S)-2-(4-((R)-2-fluoro-9-hydroxy-9-(trifluoromethyl)-9H-fluoren-4-yl)-1H-pyrazol-1-yl)-N'-(4-fluorophenyl)propanehydrazide**

LCMS [M+H]515.46.

**Example 51: Preparation of (R)-2-(4-((S)-2-fluoro-9-hydroxy-9-(trifluoromethyl)-9H-fluoren-4-yl)-1H-pyrazol-1-yl)-N'-(4-fluorophenyl)propanehydrazide**

LCMS [M+H]515.46.

**Example 52: Preparation of 2-(4-(9-hydroxy-2-methoxy-9-(trifluoromethyl)-9H-fluoren-4-yl)-1H-pyrazol-1-yl)-N-(piperidin-1-yl)propanamide**

The title compound was obtained in a similar manner as in Example 1, except that 1-bromo-2-chloro-4-methoxybenzene was used instead of 1-bromo-2-chloro-4-fluorobenzene in the step 1-1 of Example 1, and piperidin-1-amine was used instead of (4-fluorophenyl)hydrazine in the step 1-8.
LCMS [M+H]501.5.

**Example 53: Preparation of 2-(4-(2-fluoro-9-hydroxy-9-(trifluoromethyl)-9H-fluoren-4-yl)-1H-pyrazol-1-yl)-N-(piperidin-1-yl)propanamide**

The title compound was obtained in a similar manner as in Example 1, except that piperidin-1-amine was used instead of (4-fluorophenyl)hydrazine in the step 1-8 of Example 1.
LCMS [M+H]489.4.

**Example 54: Preparation of N-(azepan-1-yl)-2-(4-(2-fluoro-9-hydroxy-9-(trifluoromethyl)-9H-fluoren-4-yl)-1H-pyrazol-1-yl)propanamide**

The title compound was obtained in a similar manner as in Example 1, except that azepan-1-amine was used instead of (4-fluorophenyl)hydrazine in the step 1-8 of Example 1.
LCMS [M+H]503.5

**Experimental Example 1: Verification of therapeutic effect in diabetes animal model**

A vehicle (10% DMSO + 0.5% methylcellulose) and the compound of Example 1 (Compound 1) were administered to 6-week-old male KKAy diabetes mice (n = 6 per group) at a dose of 0.5 mg/kg once a day by oral gavage. At 10 weeks of administration, an intraperitoneal glucose tolerance test (IPGTT) was performed by intraperitoneally administering 1 g/kg of glucose, and the results are shown in FIG. 1.

As shown in FIG. 1, it was confirmed that the blood glucose increase was significantly suppressed in the group administered 0.5 mg/kg of Compound 1, as compared to the vehicle administration group (*p<0.01 versus B6/J Control. **p<0.01, ***p<0.05 versus KKAy Control).

**Experimental Example 2: Verification of therapeutic effect in acute kidney ischemic reperfusion (IR) injury animal model**

Vehicle and Compound 1 (4 μg/kg or 8 μg/kg) were administered to 8-week-old male C57BL6J mice via the tail vein 24 hours before, 1 hour before, and 12 hours after ischemic reperfusion (IR) surgery. The surgery was performed by blocking the artery leading to the kidney to induce ischemia for 37 minutes. The blocked artery was released, and 24 hours later, the mice were anesthetized and autopsied. A part of the isolated kidney was stained with H&E and TUNEL, and the results are shown in FIG. 2. Other part was used to synthesize cDNA, and a kidney damage marker, NGAL was identified by real-time PCR. And serum was separated from the blood, and creatinine, which is a kidney damage marker in the serum, was examined, and the results are shown in FIG. 3.

As shown in FIG. 2, H&E staining of mouse kidneys showed renal tubular tissue damage in the vehicle-treated IR group after IR surgery, and a significant reduction in the renal tubular tissue damage was observed in the Compound 1-treated IR groups (4 μg/kg and 8 μg/kg). TUNEL staining to examine apoptosis of the tissue also showed a significant reduction in the apoptosis in the Compound 1-treated IR groups (4 μg/kg and 8 μg/kg), as compared to the vehicle-treated IR group.

As shown in FIG. 3, RNA expression of NGAL, which is a mouse renal tissue damage marker, was markedly increased in the vehicle-treated IR group, and this increase was significantly reduced in the Compound 1-treated IR groups (4 μg/kg and 8 μg/kg). The amount of serum creatinine, which is another renal tissue damage marker, was also considerably increased in the vehicle-treated IR group, and this increase was significantly reduced in the Compound 1-treated IR groups (4 μg/kg and 8 μg/kg) (*p<0.01 versus Sham, **p<0.01 versus IR+Vehicle).

**Experimental Example 3: Verification of therapeutic effect in myocardial infarction disease cell model**

H9C2 cells, which are a rat cardioblastoma cell line, were seeded at a density of $3 \times 10^3$ cells/well in a 96-well plate and cultured for 24 hours. The next day, the medium was replaced with FBS-free DMEM and cultured for an additional 24 hours. After replacing the medium with FBS-free DMEM again, 1 μM of Compound 1 was treated and cultured in a hypoxia chamber (1% oxygen, 5% $CO_2$) for 2 hours (hypoxia). After replacing the medium with DMEM with 10% FBS, 1 μM of Compound 1 was treated and cultured in a $CO_2$ incubator for 1 hour (reoxygenation). Apoptosis, mitochondrial superoxide levels, and intracellular calcium levels were measured, and the results are shown in FIGS. 4 and 5, respectively.

As shown in FIG. 4, TUNEL staining showed that the increased apoptosis due to hypoxia-reoxygenation (H/R) was markedly reduced in the cell group treated with 1 μM of Compound 1 (top of FIG. 4). MitoSOX staining (5 μM, 10 min), which is a fluorescent dye treated to measure mitochondrial superoxide, also showed that the mitochondrial superoxide increased due to hypoxia-reoxygenation was remarkably reduced in the cell group treated with 1 μM of Compound 1 (bottom of FIG. 4).

In addition, as shown in FIG. 5, Rhod2 staining (5 μM, 1 h), which is a fluorescent dye treated to measure the amount of intracellular calcium causing cell damage, also showed that the amount of intracellular calcium increased due to hypoxia-reoxygenation was remarkably reduced in the cell group treated with 1 μM of Compound 1.

**Experimental Example 4: Verification of therapeutic effect in inflammatory bowel disease animal model**

Colitis was induced in 8-week-old male C57BL6J JmcSlc mice by feeding 3.5% dextran sodium sulfate (DSS) ad libitum for 9 days, and then replacing the same with normal drinking water. The vehicle and Compound 1 (0.5 mg/kg or 1 mg/kg) were orally administered to the experimental animal model prepared as described above daily from 2 days before DSS drinking until the end of drinking. In order to determine the degree of colonic inflammation, the degree of longitudinal contraction of the isolated colon was measured, and the histological scores were confirmed through H&E staining, which is shown in FIG. 6. Furthermore, total RNA was isolated from the isolated colon, cDNA was synthesized, and the expression levels of representative cytokines (IFNγ, IL-1β, IL-4, IL-6, IL-10, IL-12β, IL-17a, and TNFα) were measured and compared

using qPCR, and the results are shown in FIG. 7.

As shown in FIG. 6, damage to the colonic epithelial cells (yellow arrows in FIG. 6A) was observed in the DSS-treated mice, and the recovery was slight in the group administered 0.5 mg/kg of Compound 1, but remarkable epithelial cell recovery (red arrows in FIG. 6A) was observed in the group administered 1 mg/kg of Compound 1. The tissue score also showed the lowest score in the group administered 1 mg/kg of Compound 1 in a similar pattern (FIG. 6B). Furthermore, when the longitudinal contraction accompanying colonic inflammation was observed with the naked eye, it was observed that the contraction was alleviated both in the group administered 0.5 mg/kg of Compound 1 and in the group administered 1 mg/kg of Compound 1, as compared to the vehicle administration group, and the effect increased as the administration amount increased (FIGS. 6C and 6D).

As shown in FIG. 7, the mRNA expressions of inflammatory cytokines IFNγ, IL-6, IL-12b, IL-17a and TNFα were significantly reduced in the Compound 1 administration group, as compared to the vehicle administration group, although there was a difference in the degree. Meanwhile, the mRNA expression of IL-4, which is a tissue wound healing cytokine, increased in the Compound 1 administration group, as compared to the vehicle administration group (*p<0.05, **p<0.01, ***p<0.001 versus Vehicle).

**Experimental Example 5: Verification of therapeutic effect in acute pancreatitis animal model**

Acute pancreatitis was induced by intraperitoneal administration of cerulein (50 μg/kg) dissolved in saline to 8-week-old male mice. Compound 1 was administered at a concentration of 5 μg/kg or 10 μg/kg into the tail vein 24 hours and 1 hour before cerulein administration, and cerulein was intraperitoneally administered six times at 1-hour intervals. 18 hours after the first cerulein administration, the mice were anesthetized, the serum was separated, and the amylase and lipase activities in the serum were measured, which are shown in FIG. 8.

As shown in FIG. 8, the serum amylase and lipase activities increased by acute pancreatitis were decreased by administration of Compound 1 in a dose-dependent manner (0.01 versus Control. **p<0.01 versus Cerulein).

**Experimental Example 6: Verification of therapeutic effect in sepsis animal model**

50 mg/kg of lipopolysaccharide (LPS) administration dose corresponds to the 'half lethal dose' that causes death of one half of the population within 24 hours. LPS at a dose of 50 mg/kg was intraperitoneally injected with 1× PBS as a vehicle control, and when they showed signs of pathological condition such as immobility, difficulty breathing, or difficulty maintaining posture, they were euthanized with $CO_2$ gas, and that point was recorded as the humane endpoint. The animal experiment was conducted with six 8-week-old male C57BL6J mice per group. Each mouse was intraperitoneally administered 15 mg/kg of Compound 1 or pretreated with 100 μL of 10% DMSO (vehicle control). 30 minutes later, 50 mg/kg lipopolysaccharide was intraperitoneally injected into each mouse, and the viability was examined for 80 hours, and the results are shown in FIG. 9 and Table 2.

[Table 2]

| | Viability (%) | | | | | | | | | | | | |
|---|---|---|---|---|---|---|---|---|---|---|---|---|---|
| Time (h) | 0 | 20 | 24 | 28 | 32 | **36** | 40 | 44 | 48 | 50 | 60 | 70 | 80 |
| Compound 1 | 100 | 100 | 83.3 | 83.3 | 83.3 | **66.7** | 66.7 | 66.7 | 66.7 | 66.7 | 50 | 50 | 50 |
| Vehicle control group | 100 | 100 | 50 | 50 | 33.3 | **0** | 0 | 0 | 0 | 0 | 0 | 0 | 0 |

As shown in FIG. 9 and Table 2, when Compound 1 was administered before LPS injection, 66.7% survived 36 hours after LPS injection, whereas all of the control group (vehicle control group) that was injected only with LPS without pretreatment with the drug died, confirming that administration of Compound 1 had the effect of improving the viability in a sepsis-induced animal model (*p<0.01 versus LPS+V.C, V.C: Vehicle).

Furthermore, to verify the anti-inflammatory effect at a cellular level, bone marrow cells of C57BL6 mice were treated with 20 ng/mL of GM-CSF and IL-4, and 6 days later, bone marrow-derived dendritic cells (BMDCs) were collected. The BMDCs were prepared at $1\times10^6$ cells/mL and stabilized for one day, and then subsequent experiments were conducted. The above dendritic cells were treated with 500 ng/mL of LPS for 3 hours to prime pro-IL1 β, and then treated with Compound 1 (0 μM, 0.01 μM, 0.1 μM, 1 μM, and 10 μM) for 15 minutes. Next, 5 mM of ATP was treated, which induces inflammasome formation by converting pro-IL1β to IL-1β through pro-IL1β cleavage, and 30 minutes later, the concentration of IL-1β in the supernatant was measured by ELISA, and the results are shown in FIG. 10 and Table 3.

[Table 3]

| | IL-1β (pg/mL) | | | | | | |
|---|---|---|---|---|---|---|---|
| Compound 1 (μM) | 0 | 0 | 0 | 0.01 | 0.1 | 1 | 10 |
| | 15.83775 | 14.93956 | 1205.011 | 1131.823 | 1009.825 | 907.2636 | 647.6982 |

As shown in FIG. 10 and Table 3, Compound 1 treatment group significantly inhibited IL-1β expression by about 83.802% at a concentration of 0.1 μM and about 53.7504% at a concentration of 10 μM, as compared to the vehicle control group (***p<0.01 versus con, *p<0.05, **p<0.01 versus LPS+ATP).

**Experimental Example 7: Verification of therapeutic effect in graft-versus-host disease (GvHD) animal model**

5-week-old BALB/c male mice were acclimatized for 1 week and irradiated with 800 cGy of total body irradiation. Then, $5 \times 10^6$ bone marrow cells derived from C57BL/6J and $1 \times 10^7$ spleen cells were intravenously injected to establish a GvHD *in vivo* model (positive control). A syngeneic control group was established in the same manner as above, but BALB/c-derived bone marrow cells were selected as the injected bone marrow cells. Compound 1 Daily was established by daily oral gavage of1 mg/kg of Compound 1 to the GvHD *in vivo* model. Compound 1 Initial only was established by orally administering 1 mg/kg of Compound 1 to the GvHD *in vivo* model only for 6 days after model induction. The therapeutic effect of Compound 1 was verified by measuring the change in body weight every 3 days for each control and experimental group established as above, and the results are shown in FIG. 11 and Table 4. On day 14, the production of $CD4^+IFNg^+$ T cells, which is a major factor inducing GvHD, was measured in the spleens isolated from the mice, and the results are shown in FIG. 12 and Table 5.

[Table 4]

| Measurement date | Syngeneic | Positive | Daily compound 1 | Compound 1 Initial only |
|---|---|---|---|---|
| D0 | 0 | 0 | 0 | 0 |
| D3 | -14.34132 | -12.50228 | -14.77473 | -11.14954 |
| D6 | -18.23346 | -21.62782222 | -22.94925 | -19.00776 |
| D9 | -12.11754 | -19.48265714 | -19.06935714 | -14.6667 |
| D12 | -8.443326 | -20.24557143 | -16.1959 | -11.98814 |

[Table 5]

| | Syngeneic | Positive | Daily compound 1 | Compound 1 Initial only |
|---|---|---|---|---|
| Mean value | 3.16 | 18.06666667 | 10.27666667 | 13.04333333 |

As shown in FIG. 11 and Table 4, the body weight of the GvHD *in vivo* model was significantly recovered in Daily compound 1 administration group and Compound 1 initial only administration group, as compared to the control group. In addition, as shown in FIG. 12 and Table 5, the production of allogeneic $CD4^+IFNg^+$ T cells was significantly reduced in Daily compound 1 administration group and Compound 1 initial only administration group, as compared to the control group (**p<0.01, ***0.001 versus Positive).

**Experimental Example 8: Verification of therapeutic effect in Charcot-Marie-Tooth disease animal model**

To verify the functional loss due to mutation of MFN2 present in the outer mitochondrial membrane, which is the main cause of Charcot-Marie-Tooth disease, the mitochondrial oxygen consumption rate (OCR) was measured. In detail, $Mfn2^{+/+}$ MEF cells (normal cells), which are mouse embryonic fibroblasts expressing Mfn2, and $Mfn2^{-/-}$ MEF cells not expressing Mfn2 were cultured at a density of $1 \times 10^4$ for 24 hours in a Seahorse XF24 culture dish using a DMEM medium supplemented with 10% FBS and penicillin/streptomycin. The cells were treated with DMSO as a vehicle and Compound 1 (1 μM) and cultured for another 24 hours. The mitochondrial oxygen consumption rate was measured using a Seahorse XFe96 analyzer (Agilent Technologies), and the results are shown in FIG. 13.

As shown in FIG. 13, the mitochondrial oxygen consumption rate in $Mfn2^{-/-}$ MEF cells not expressing Mfn2 rapidly decreased, as compared to normal $Mfn2^{+/+}$ MEF cells. In contrast, the oxygen consumption rate of $Mfn2^{-/-}$ MEF cells treated with Compound 1 was markedly restored to a level comparable to that of normal cells, indicating that Compound 1

may improve the lost function of mitochondria (*p<0.01 versus Mfn2$^{-/-}$+DMSO, **p<0.01 versus Mfn2$^{+/+}$+DMSO).

**Experimental Example 9: Verification of therapeutic effect in Cushing's syndrome cell model**

NCI-H295R cells (ATCC #CRL-2128), which are a human adrenocortical carcinoma cell line, were seeded at a density of 2.5 × $10^5$ cells/well in a 24-well plate. The cells were cultured for 48 hours, and then they were pretreated with Compound 1 (0.1 μM, 0.5 μM, and 1 μM) for 12 hours. Furthermore, to increase cortisol synthesis, the cells were treated with 8-bromoadenosine 3',4'-cyclic monophosphate (8-Br-cAMP) at a concentration of 0.5 mM, and then re-treated with Compound 1, and allowed to react for 24 hours. After the reaction, the cell culture medium was collected, and the amount of cortisol produced in the cells was measured using an ELISA kit, and the results are shown in FIG. 14.

As shown in FIG. 14, cortisol production in NCI-H295R cells, which was remarkably increased by the addition of cAMP, was significantly inhibited by the administration of Compound 1, and this inhibition showed a concentration-dependent tendency (*p<0.01 versus non-treatment, **0.05, ***p<0.01 versus cAMP).

**Experimental Example 10: *In vitro* PDH S293 phosphorylation inhibitory effect**

HEK293 cells, which are human embryonic kidney cells, were seeded at a density of 2 × $10^6$ in a 60 mm dish, and cultured for 24 hours. After replacing the medium with a medium comprising 0.5% FBS, the compounds prepared according to Examples 1 to 54 were treated at various concentrations. 5 hours later, the cells were scraped off and collected into a 15 mL conical tube and centrifuged at 1500 rpm for 10 minutes. The supernatant was discarded, the resultant was washed with 1× PBS, and transferred to a 1.5 mL e-tube, followed by centrifugation at 10,000 rpm for 5 minutes. The supernatant was discarded again, a lysis buffer was added, and the cells were lysed on ice for 30 minutes. The cells were vortexed every 10 minutes to completely lyse, and centrifuged again at 10,000 rpm for 15 minutes at 4°C, and the supernatant was collected. The protein in the supernatant was quantified using BCA technique, and the sample buffer was added to 20 μg of protein and allowed to react at 70°C for 5 minutes. The expression of PDHE1α S232, S293, S300, and total PDHE1α proteins was detected by Western blot, and the resulting PDH S293 phosphorylation inhibition is shown in Table 6. As shown in Table 6, many compounds showed 50% or more of PHDE1α S293 expression inhibition rates, and some compounds showed high inhibition rates of 80% or more.

[Table 6]

| Example | PDH S293 phosphorylation inhibition | Example | PDH S293 phosphorylation inhibition | Example | PDH S293 phosphorylation inhibition | Example | PDH S293 phosphorylation inhibition |
|---|---|---|---|---|---|---|---|
| 1 | +++ | 2 | ++ | 3 | ++ | 4 | +++ |
| 5 | ++ | 6 | ++ | 7 | ++ | 8 | +++ |
| 9 | +++ | 10 | ++ | 11 | ++ | 12 | ++ |
| 13 | ++ | 14 | ++ | 15 | ++ | 16 | ++ |
| 17 | ++ | 18 | +++ | 19 | ++ | 20 | ++ |
| 21 | ++ | 22 | ++ | 23 | ++ | 24 | ++ |
| 25 | ++ | 26 | ++ | 27 | ++ | 28 | ++ |
| 29 | ++ | 30 | ++ | 31 | ++ | 32 | ++ |
| 33 | ++ | 34 | ++ | 35 | ++ | 36 | ++ |
| 37 | ++ | 38 | ++ | 39 | ++ | 40 | ++ |
| 41 | ++ | 42 | ++ | 43 | ++ | 44 | ++ |
| 45 | ++ | 46 | ++ | 47 | ++ | 48 | ++ |
| 49 | ++ | 50 | ++ | 51 | ++ | 52 | ++ |
| 53 | ++ | 54 | ++ | | | | |
| +++: 80% or more of PHDE1α S293 expression inhibition rate at 1 μM<br>++: 50% or more of PHDE1α S293 expression inhibition rate at 1 μM<br>+: less than 50% of PHDE1α S293 expression inhibition rate at 1 μM | | | | | | | |

**Experimental Example 11: Growth inhibitory effects on normal cells and various cancer cells**

In order to verify the growth inhibitory effect of Compound 1 on various normal and cancer cell lines, 25 types of cell lines listed in Table 7 below were tested. In detail, each cell was seeded at a density of $2 \times 10^4$ cells/well in a 96-well plate and cultured for 24 hours, and then the medium was replaced with a medium comprising 0.5% (for cancer cells) or 10% (for normal cells) FBS, and Compound 1 was treated at various concentrations. 24 hours later, 10 μL of Cell Counting Kit-8 (Dojindo Molecular Technologies) was added, and 1 hour later, absorbance at 480 nm was measured using a microplate reader (TECAN SPARK), and the results are shown in FIGS. 15 to 21.

[Table 7]

| Cell lines | Biological sources |
|---|---|
| BJ | Human normal fibroblast cell |
| CHO | hamster epithelial ovary |
| Raw264.7 | Murine macrophage cell |
| L929 | Mouse fibroblast cell |
| PC3 | Prostate cancer |
| DU145 | Prostate cancer |
| LNCaP | Prostate cancer |
| HCT116 | Colorectal cancer |
| RKO | Colorectal cancer |
| SW480 | Colorectal cancer |
| CT26 | Colorectal cancer |
| C42B | Prostate cancer |
| A549 | Lung cancer |
| H460 | Lung cancer |
| Huh7 | Liver cancer |
| 4T1 | Breast cancer |
| MDA-MB231 | Breast cancer |
| A2780 | Ovary cancer |
| SKOV3 | Ovary cancer |
| U87MG | Glioma |
| HTB-9 | Bladder cancer |
| T24 | Bladder cancer |
| CAL62 | Anaplastic thyroid cancer |
| BHT-101 | Anaplastic thyroid cancer |
| BHP103scp | Poorly differentiated thyroid cancer |

As shown in FIG. 15, when treated with Compound 1, normal cells showed 70% or more of viability at a concentration of up to less than 40 μM. In contrast, as shown in FIGS. 16 to 20, Compound 1 decreased the viability of cancer cells in a concentration-dependent manner. Specifically, the viability of colorectal cancer, thyroid cancer, prostate cancer, lung cancer, breast cancer, bladder cancer, liver cancer, brain cancer, and ovarian cancer cells began to rapidly decrease to 70% or less when treated with Compound 1 at concentrations of 5 μM to 15 μM, 1 μM to 10 μM, 10 μM to 15 μM, 3 μM to 7 μM, 1 μM to 15 μM, less than 1.2 μM, 10 μM, and 1 μM to 10 μM or more, respectively. This indicates that the compound of the present disclosure does not exhibit toxicity on normal cells at concentrations of up to less than 40 μM, whereas it inhibits cell viability to 70% or less at concentrations much lower than the above concentration for various cancer cells, suggesting that the compound may be usefully applied in the treatment of these cancer diseases.

**Experimental Example 12: Evaluation of anticancer efficacy in HCT-116 colorectal cancer tumor model**

A colorectal cancer model was established by subcutaneously transplanting HCT-116 colorectal cancer cell line into nude mice. The colorectal cancer model was divided into the following three groups: Group 1 was administered a vehicle as a control group, Group 2 was administered 0.5 mg/kg of Compound 1, and Group 3 was administered 1.0 mg/kg of Compound 1. The drug was orally administered once a day for a total of 21 times. To evaluate the therapeutic efficacy, the tumor size was measured before drug administration and on days 3, 7, 10, 14, 17, 21, and 24 after drug administration. The tumor size was calculated as follows:

$$Tumor\ size\ (mm^3) = (A \times B^2)/2$$

wherein A and B represent the tumor long axis and short axis, respectively.

In addition, the body weight was measured before drug administration and on days 7, 14, and 21 after drug administration. The calculated tumor volume and the measured body weight are shown in FIG. 21. The vehicle administration group showed a continuous and accelerated tumor growth trend until day 24. In contrast, Compound 1 administration group showed a remarkably delayed and reduced tumor growth trend, as compared to the vehicle administration group, and showed a statistically significant concentration-dependent tumor growth inhibitory effect (*$p < 0.05$ versus the vehicle administration group). In detail, the tumor volume measured on day 24, which was the last day of observation, showed 41% and 49% reduced tumor growth in 0.5 mg/kg and 1.0 mg/kg Compound 1 administration groups, respectively, as compared to the vehicle administration group. Meanwhile, no significant body weight change was observed in either the vehicle control group or the Compound 1 administration group.

**Experimental Example 13: Evaluation of anticancer efficacy in CT26 colorectal cancer tumor model**

A colorectal cancer model was established by subcutaneously transplanting CT26 colorectal cancer cell line into nude mice. The colorectal cancer model was divided into two groups: vehicle administration group and Compound 1 administration group. At this time, Compound 1 was administered at a dose of 1.0 mg/kg, which showed a higher tumor growth inhibitory effect in Experimental Example 13. Similar to Experimental Example 13, tumor size was calculated before drug administration and on days 3, 7, 10, 14, 18, 22, and 24 after drug administration, and body weight was measured before drug administration and on days 7, 14 and 22 after drug administration, which are shown in FIG. 22. In the CT26 colorectal cancer tumor model, tumor growth was statistically significantly delayed and inhibited in the Compound 1 administration group, as compared to the vehicle administration group (*$p < 0.05$ versus the vehicle administration group), and the tumor volume measured on day 24 showed a 55% decrease in tumor growth in the 1.0 mg/kg compound 1 administration group, as compared to the vehicle administration group. Neither the vehicle administration group nor the compound 1 administration group showed any change in the body weight in the CT26 colorectal cancer model, as in the HCT-116 colorectal cancer model.

Additionally, tumors were extracted from the colorectal cancer model, fixed with 4% PFA solution, and subjected to TUNEL assay to determine the degree of apoptosis induction, and the results are shown in FIG. 22. As shown in the middle of FIG. 22, 28% apoptosis was induced in the vehicle administration group, and 72% apoptosis was induced in the 1.0 mg/kg compound 1 administration group, and statistical significance between these values was also verified (*$p<0.01$ versus the vehicle administration group).

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A compound represented by the following Chemical Formula 1 or a pharmaceutically acceptable salt thereof:

[Chemical Formula 1]

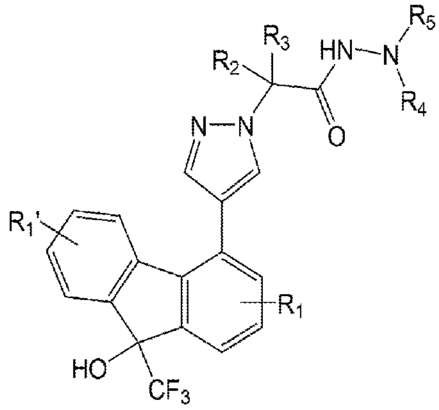

in Chemical Formula 1,

$R_1$ and $R_1$' are each independently hydrogen, $C_{1-4}$ alkoxy, or halogen;
$R_2$ and $R_3$ are each independently hydrogen, $C_{3-10}$ cycloalkyl- $C_{1-4}$ alkyl or $C_{1-4}$ alkyl;
$R_4$ is hydrogen;
$R_5$ is $C_{6-10}$ aryl, 5-10 membered heteroaryl, or 5-10 membered heterocyclyl which is unsubstituted or substituted with one or more substituents selected from the group consisting of oxo, halogen, $C_{1-4}$ haloalkyl, $C_{0-4}$ alkylsulfonyl, carboxy, and $C_{1-4}$ alkoxycarbonyl; or
$R_4$ and $R_5$ are connected to each other to form 5- to 10-membered heterocyclyl together with a nitrogen atom to which they are attached.

**2.** The compound or pharmaceutically acceptable salt thereof of claim 1, wherein the compound is a compound represented by the following Chemical Formula 1-1:

[Chemical Formula 1-1]

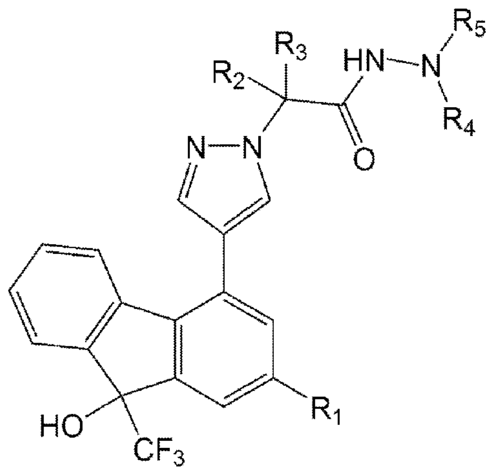

.

**3.** The compound or pharmaceutically acceptable salt thereof of claim 1 or 2, wherein $R_1$ and $R_1$' are each independently methoxy or fluoro.

**4.** The compound or pharmaceutically acceptable salt thereof of claim 1 or 2, wherein $R_2$ is hydrogen or methyl, and $R_3$ is hydrogen, methyl, ethyl, isopropyl, isobutyl, or cyclohexylmethyl.

**5.** The compound or pharmaceutically acceptable salt thereof of claim 1 or 2, wherein $R_4$ is hydrogen,

$R_5$ is phenyl, naphthyl, pyridinyl, pyrimidinyl, pyrazinyl, quinoxalinyl, quinolinyl, quinazolinyl, or dihydroquinolinyl which is unsubstituted or substituted with one or more substituents selected from the group consisting of oxo,

fluoro, chloro, trifluoromethyl, methylsulfonyl, carboxy, and tert-butoxycarbonyl; or $R_4$ and $R_5$ are connected to each other to form piperidinyl or azepanyl together with a nitrogen atom to which they are attached.

**6.** The compound or pharmaceutically acceptable salt thereof of claim 1 or 2, wherein the compound is

1. 2-(4-(2-fluoro-9-hydroxy-9-(trifluoromethyl)-9H-fluoren-4-yl)-1H-pyrazol-1-yl)-N'-(4-fluorophenyl)propanehydrazide,
2. N'-(4-fluorophenyl)-2-(4-(9-hydroxy-2-methoxy-9-(trifluoromethyl)-9H-fluoren-4-yl)-1H-pyrazol-1-yl)propanehydrazide,
3. N'-(2-fluoro-4-(methylsulfonyl)phenyl)-2-(4-(2-fluoro-9-hydroxy-9-(trifluoromethyl)-9H-fluoren-4-yl)-1H-pyrazol-1-yl)propanehydrazide,
4. 2-(4-(2-fluoro-9-hydroxy-9-(trifluoromethyl)-9H-fluoren-4-yl)-1H-pyrazol-1-yl)-N'-phenylpropanehydrazide,
5. 2-(4-(2-fluoro-9-hydroxy-9-(trifluoromethyl)-9H-fluoren-4-yl)-1H-pyrazol-1-yl)-N'-(naphthalen-1-yl)propanehydrazide,
6. N'-(2-chlorophenyl)-2-(4-(2-fluoro-9-hydroxy-9-(trifluoromethyl)-9H-fluoren-4-yl)-1H-pyrazol-1-yl)propanehydrazide,
7. N'-(4-chlorophenyl)-2-(4-(2-fluoro-9-hydroxy-9-(trifluoromethyl)-9H-fluoren-4-yl)-1H-pyrazol-1-yl)propanehydrazide,
8. 2-(4-(2-fluoro-9-hydroxy-9-(trifluoromethyl)-9H-fluoren-4-yl)-1H-pyrazol-1-yl)-N'-(pyridin-2-yl)propanehydrazide,
9. N'-(2-fluoro-4-(methylsulfonyl)phenyl)-2-(4-(9-hydroxy-2-methoxy-9-(trifluoromethyl)-9H-fluoren-4-yl)-1H-pyrazol-1-yl)propanehydrazide,
10. N'-(4-chlorophenyl)-2-(4-(9-hydroxy-2-methoxy-9-(trifluoromethyl)-9H-fluoren-4-yl)-1H-pyrazol-1-yl)propanehydrazide,
11. 2-(4-(9-hydroxy-2-methoxy-9-(trifluoromethyl)-9H-fluoren-4-yl)-1H-pyrazol-1-yl)-N'-(naphthalen-1-yl)propanehydrazide,
12. N'-(2-chlorophenyl)-2-(4-(9-hydroxy-2-methoxy-9-(trifluoromethyl)-9H-fluoren-4-yl)-1H-pyrazol-1-yl)propanehydrazide,
13. 2-(4-(2-fluoro-9-hydroxy-9-(trifluoromethyl)-9H-fluoren-4-yl)-1H-pyrazol-1-yl)-N'-(2-fluorophenyl)propanehydrazide,
14. 6-(2-(2-(4-(2-fluoro-9-hydroxy-9-(trifluoromethyl)-9H-fluoren-4-yl)-1H-pyrazol-1-yl)propanoyl)hydrazinyl)nicotinic acid,
15. tert-butyl 6-(2-(2-(4-(9-hydroxy-2-methoxy-9-(trifluoromethyl)-9H-fluoren-4-yl)-1H-pyrazol-1-yl)propanoyl) hydrazinyl)nicotinate,
16. 6-(2-(2-(4-(9-hydroxy-2-methoxy-9-(trifluoromethyl)-9H-fluoren-4-yl)-1 H-pyrazol-1-yl)propanoyl)hydrazinyl)nicotinic acid,
17. 2-(4-(9-hydroxy-2-methoxy-9-(trifluoromethyl)-9H-fluoren-4-yl)-1H-pyrazol-1-yl)-N'-(pyridin-2-yl)propanehydrazide,
18. 2-(4-(2-fluoro-9-hydroxy-9-(trifluoromethyl)-9H-fluoren-4-yl)-1H-pyrazol-1-yl)-N'-(pyridin-2-yl)acetohydrazide,
19. tert-butyl 6-(2-(2-(4-(2-fluoro-9-hydroxy-9-(trifluoromethyl)-9H-fluoren-4-yl)-1H-pyrazol-1-yl)acetyl)hydrazinyl)nicotinate,
20. 6-(2-(2-(4-(2-fluoro-9-hydroxy-9-(trifluoromethyl)-9H-fluoren-4-yl)-1H-pyrazol-1-yl)acetyl)hydrazinyl)nicotinic acid,
21. 2-(4-(2-fluoro-9-hydroxy-9-(trifluoromethyl)-9H-fluoren-4-yl)-1H-pyrazol-1-yl)-N'-(4-fluorophenyl)acetohydrazide,
22. 2-(4-(2-fluoro-9-hydroxy-9-(trifluoromethyl)-9H-fluoren-4-yl)-1H-pyrazol-1-yl)-N'-(4-(methylsulfonyl)phenyl) propanehydrazide,
23. 2-(4-(9-hydroxy-2-methoxy-9-(trifluoromethyl)-9H-fluoren-4-yl)-1H-pyrazol-1-yl)-N'-(4-(methylsulfonyl)phenyl)propanehydrazide,
24. 2-(4-(2-fluoro-9-hydroxy-9-(trifluoromethyl)-9H-fluoren-4-yl)-1H-pyrazol-1-yl)-N'-(pyrazin-2-yl)propanehydrazide,
25. 2-(4-(9-hydroxy-2-methoxy-9-(trifluoromethyl)-9H-fluoren-4-yl)-1H-pyrazol-1-yl)-N'-(pyrazin-2-yl)propanehydrazide,
26. 2-(4-(2-fluoro-9-hydroxy-9-(trifluoromethyl)-9H-fluoren-4-yl)-1H-pyrazol-1-yl)-N'-(5-(trifluoromethyl)pyridin-2-yl)propanehydrazide,
27. 2-(4-(9-hydroxy-2-methoxy-9-(trifluoromethyl)-9H-fluoren-4-yl)-1H-pyrazol-1-yl)-N'-(5-(trifluoromethyl)pyridin-2-yl)propanehydrazide,
28. 2-(4-(2-fluoro-9-hydroxy-9-(trifluoromethyl)-9H-fluoren-4-yl)-1H-pyrazol-1-yl)-N'-(pyrimidin-2-yl)propanehydrazide,
29. 2-(4-(9-hydroxy-2-methoxy-9-(trifluoromethyl)-9H-fluoren-4-yl)-1H-pyrazol-1-yl)-N'-(pyrimidin-2-yl)propanehydrazide,
30. 2-(4-(2-fluoro-9-hydroxy-9-(trifluoromethyl)-9H-fluoren-4-yl)-1H-pyrazol-1-yl)-N'-(pyridin-3-yl)propanehydrazide,
31. 2-(4-(9-hydroxy-2-methoxy-9-(trifluoromethyl)-9H-fluoren-4-yl)-1H-pyrazol-1-yl)-N'-(pyridin-3-yl)propanehydrazide,
32. N'-(3-chloro-5-(trifluoromethyl)pyridin-2-yl)-2-(4-(2-fluoro-9-hydroxy-9-(trifluoromethyl)-9H-fluoren-4-yl)-1H-pyrazol-1-yl)propanehydrazide,
33. N'-(3-chloro-5-(trifluoromethyl)pyridin-2-yl)-2-(4-(9-hydroxy-2-methoxy-9-(trifluoromethyl)-9H-fluoren-4-yl)-1H-pyrazol-1-yl)propanehydrazide,
34. 2-(4-(2-fluoro-9-hydroxy-9-(trifluoromethyl)-9H-fluoren-4-yl)-1H-pyrazol-1-yl)-N'-(pyridin-4-yl)propanehydrazide,
35. 2-(4-(2-fluoro-9-hydroxy-9-(trifluoromethyl)-9H-fluoren-4-yl)-1H-pyrazol-1-yl)-N'-(quinazolin-4-yl)propanehydrazide,
36. 2-(4-(2-fluoro-9-hydroxy-9-(trifluoromethyl)-9H-fluoren-4-yl)-1H-pyrazol-1-yl)-N'-(quinolin-4-yl)propanehydrazide,
37. 2-(4-(2-fluoro-9-hydroxy-9-(trifluoromethyl)-9H-fluoren-4-yl)-1H-pyrazol-1-yl)-N'-(quinoxalin-2-yl)propanehydrazide,
38. 2-(4-(2-fluoro-9-hydroxy-9-(trifluoromethyl)-9H-fluoren-4-yl)-1H-pyrazol-1-yl)-N'-(2-oxo-1,2-dihydroquinolin-3-yl)propanehydrazide,
39. 2-(4-(2-fluoro-9-hydroxy-9-(trifluoromethyl)-9H-fluoren-4-yl)-1H-pyrazol-1-yl)-N'-(quinolin-2-yl)propanehydrazide,
40. 2-(4-(2-fluoro-9-hydroxy-9-(trifluoromethyl)-9H-fluoren-4-yl)-1H-pyrazol-1-yl)-N'-(1-methyl-2-oxo-1,2-dihydroquinolin-4-yl)propanehydrazide,
41. 2-(4-(2-fluoro-9-hydroxy-9-(trifluoromethyl)-9H-fluoren-4-yl)-1H-pyrazol-1-yl)-N'-(imidazo[1,2-b]pyridazin-6-yl)propanehydrazide,
42. N'-(benzo[d]thiazol-2-yl)-2-(4-(2-fluoro-9-hydroxy-9-(trifluoromethyl)-9H-fluoren-4-yl)-1H-pyrazol-1-yl)propanehydrazide,
43. 2-(4-(2-fluoro-9-hydroxy-9-(trifluoromethyl)-9H-fluoren-4-yl)-1H-pyrazol-1-yl)-N'-(4-fluorophenyl)butanehydrazide,
44. 2-(4-(2-fluoro-9-hydroxy-9-(trifluoromethyl)-9H-fluoren-4-yl)-1H-pyrazol-1-yl)-N'-(4-fluorophenyl)-3-methylbutanehydrazide,
45. 2-(4-(2-fluoro-9-hydroxy-9-(trifluoromethyl)-9H-fluoren-4-yl)-1H-pyrazol-1-yl)-N'-(4-fluorophenyl)-4-methylpentanehydrazide,
46. 3-cyclohexyl-2-(4-(2-fluoro-9-hydroxy-9-(trifluoromethyl)-9H-fluoren-4-yl)-1H-pyrazol-1-yl)-N'-(4-fluorophenyl)propanehydrazide,
47. 2-(4-(2-fluoro-9-hydroxy-9-(trifluoromethyl)-9H-fluoren-4-yl)-1H-pyrazol-1-yl)-N'-(4-fluorophenyl)-2-methylpropanehydrazide,
48. (S)-2-(4-((S)-2-fluoro-9-hydroxy-9-(trifluoromethyl)-9H-fluoren-4-yl)-1H-pyrazol-1-yl)-N'-(4-fluorophenyl) propanehydrazide,
49. ((R)-2-(4-((R)-2-fluoro-9-hydroxy-9-(trifluoromethyl)-9H-fluoren-4-yl)-1 H-pyrazol-1 -yl)-N'-(4-fluorophenyl) propanehydrazide,
50. (S)-2-(4-((R)-2-fluoro-9-hydroxy-9-(trifluoromethyl)-9H-fluoren-4-yl)-1H-pyrazol-1-yl)-N'-(4-fluorophenyl) propanehydrazide,
51. (R)-2-(4-((S)-2-fluoro-9-hydroxy-9-(trifluoromethyl)-9H-fluoren-4-yl)-1H-pyrazol-1-yl)-N'-(4-fluorophenyl) propanehydrazide,
52. 2-(4-(9-hydroxy-2-methoxy-9-(trifluoromethyl)-9H-fluoren-4-yl)-1H-pyrazol-1-yl)-N-(piperidin-1-yl)propanamide,
53. 2-(4-(2-fluoro-9-hydroxy-9-(trifluoromethyl)-9H-fluoren-4-yl)-1H-pyrazol-1-yl)-N-(piperidin-1-yl)propanamide, or
54. N-(azepan-1-yl)-2-(4-(2-fluoro-9-hydroxy-9-(trifluoromethyl)-9H-fluoren-4-yl)-1H-pyrazol-1-yl)propanamide.

**7.** A method of preparing the compound or pharmaceutically acceptable salt thereof of claim 1 or 2, comprising the step of reacting a compound represented by the following Chemical Formula 2 with a compound represented by the following

Chemical Formula 3:

[Chemical Formula 2]

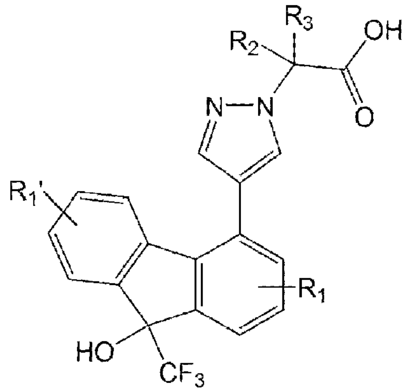

[Chemical Formula 3]

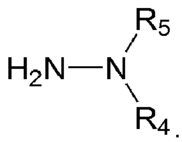

**8.** The method of claim 7, wherein the reaction is performed in the presence of 2-(1H-benzotriazole-1-yl)-1,1,3,3-tetramethylaminium tetrafluoroborate (TBTU) and N,N-diisopropylethylamine (DIPEA).

**9.** A pharmaceutical composition for preventing or treating a disease related to pyruvate dehydrogenase kinase 4 (PDK4) activity, comprising the compound or pharmaceutically acceptable salt thereof of claim 1 or 2 as an active ingredient.

**10.** The pharmaceutical composition of claim 9, wherein the composition inhibits PDK4 activity.

**11.** The pharmaceutical composition of claim 9, wherein the disease related to PDK4 activity is a metabolic disease, an inflammatory disease, or a cancer disease.

**12.** The pharmaceutical composition of claim 11, wherein the metabolic disease is obesity, type 1 diabetes, type 2 diabetes, diabetic nephropathy, diabetic neuropathy, metabolic syndrome, hyperlipidemia, hyperlactatemia, arteriosclerosis, angina, acute kidney ischemic reperfusion injury, ischemic heart disease, myocardial infarction, heart failure, chemotherapy-induced cardiomyopathy, viral myocarditis, peripheral vascular disease, intermittent claudication, stroke, mitochondriopathy, pulmonary hypertension, fatty liver, hypertension, or Cushing's syndrome.

**13.** The pharmaceutical composition of claim 12, wherein the fatty liver comprises alcoholic fatty liver diseases, non-alcoholic fatty liver diseases (NAFLDs), and non-alcoholic steatohepatitis (NASH).

**14.** The pharmaceutical composition of claim 11, wherein the inflammatory disease is acute or chronic pancreatitis, inflammatory bowel disease, sepsis, graft-versus-host disease, or Charcot-Marie-Tooth disease.

**15.** The pharmaceutical composition of claim 11, wherein the cancer disease is colorectal cancer, thyroid cancer, brain cancer, liver cancer, prostate cancer, breast cancer, pancreatic cancer, lung cancer, bladder cancer, or ovarian cancer.

[FIG. 1]

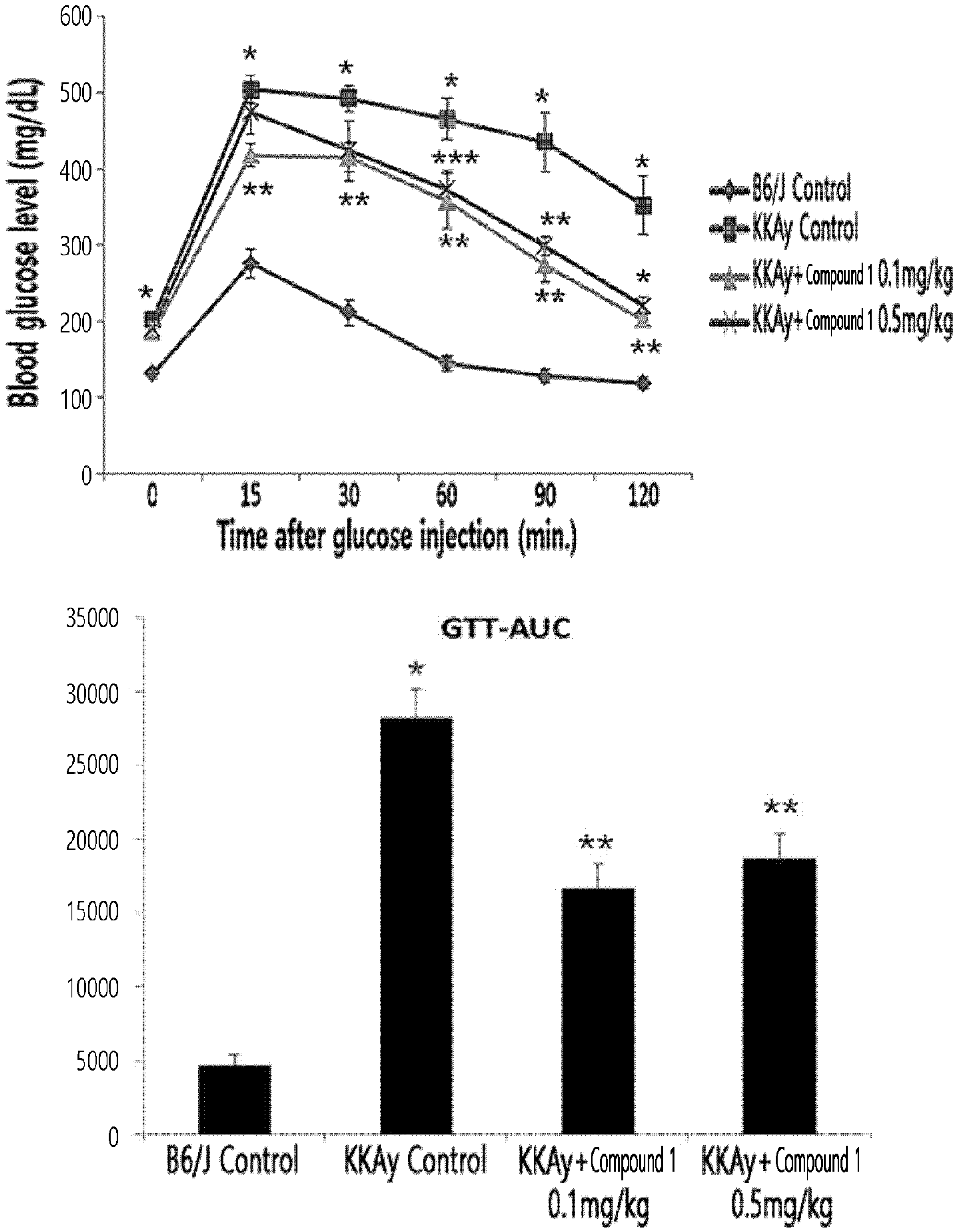
600
500
400
300
200
100
0
Blood glucose level (mg/dL)
0
15
30
60
90
120
Time after glucose injection (min.)
B6/J Control
KKAy Control
KKAy+Compound 1 0.1mg/kg
KKAy+Compound 1 0.5mg/kg
GTT-AUC
35000
30000
25000
20000
15000
10000
5000
0
B6/J Control
KKAy Control
KKAy+Compound 1 0.1mg/kg
KKAy+Compound 1 0.5mg/kg

[FIG. 2]

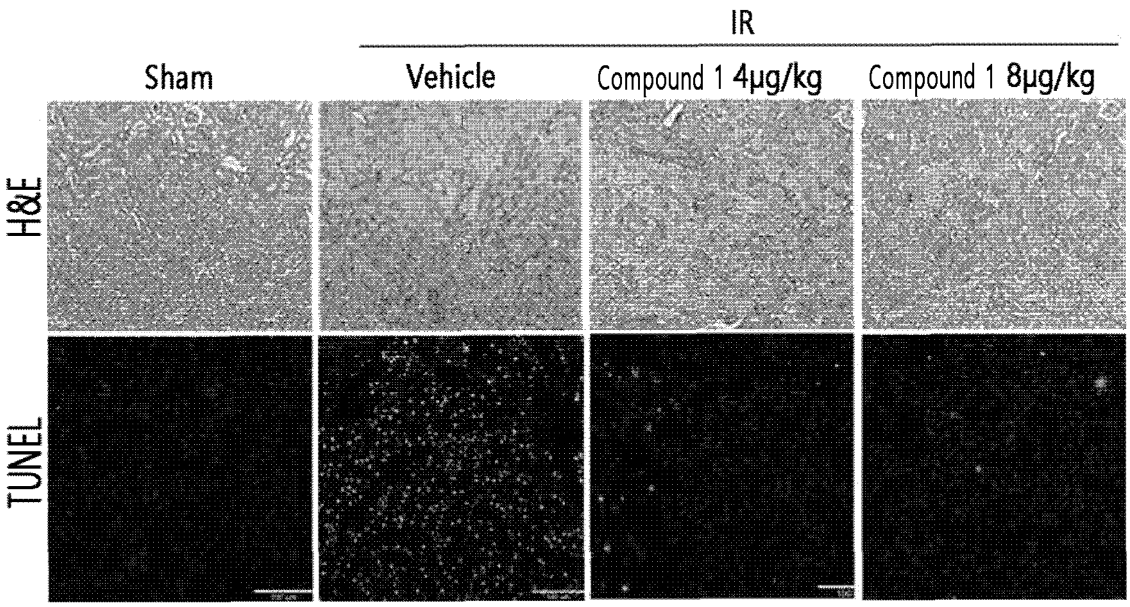
IR
Sham
Vehicle
Compound 1 4μg/kg
Compound 1 8μg/kg
H&E
TUNEL

[FIG. 3]

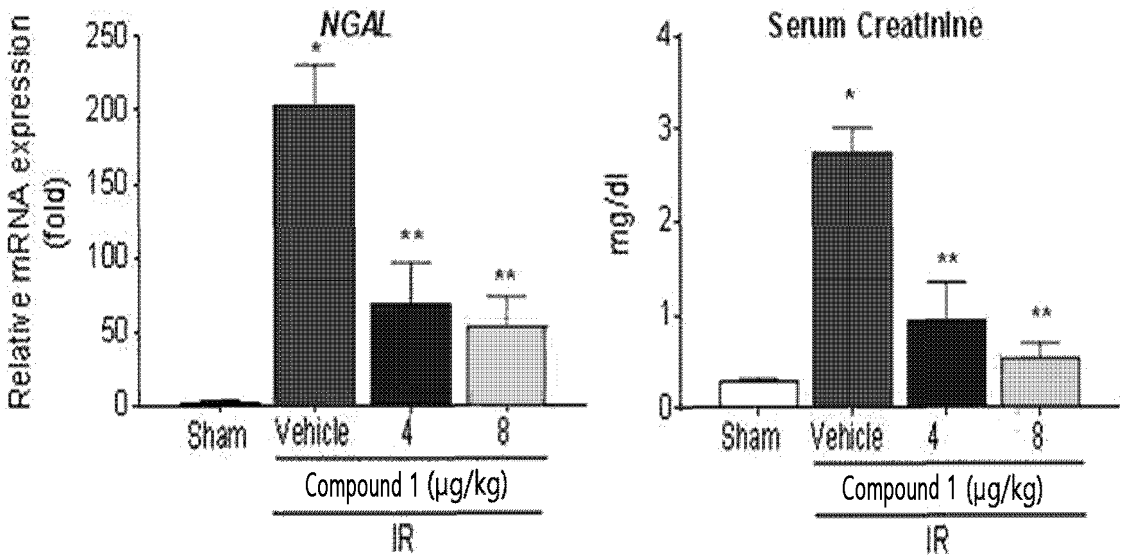
NGAL
Relative mRNA expression (fold)
250
200
150
100
50
0
*
**
**
Sham
Vehicle
4
8
Compound 1 (μg/kg)
IR
Serum Creatinine
mg/dl
4
3
2
1
0
*
**
**
Sham
Vehicle
4
8
Compound 1 (μg/kg)
IR

[FIG. 4]

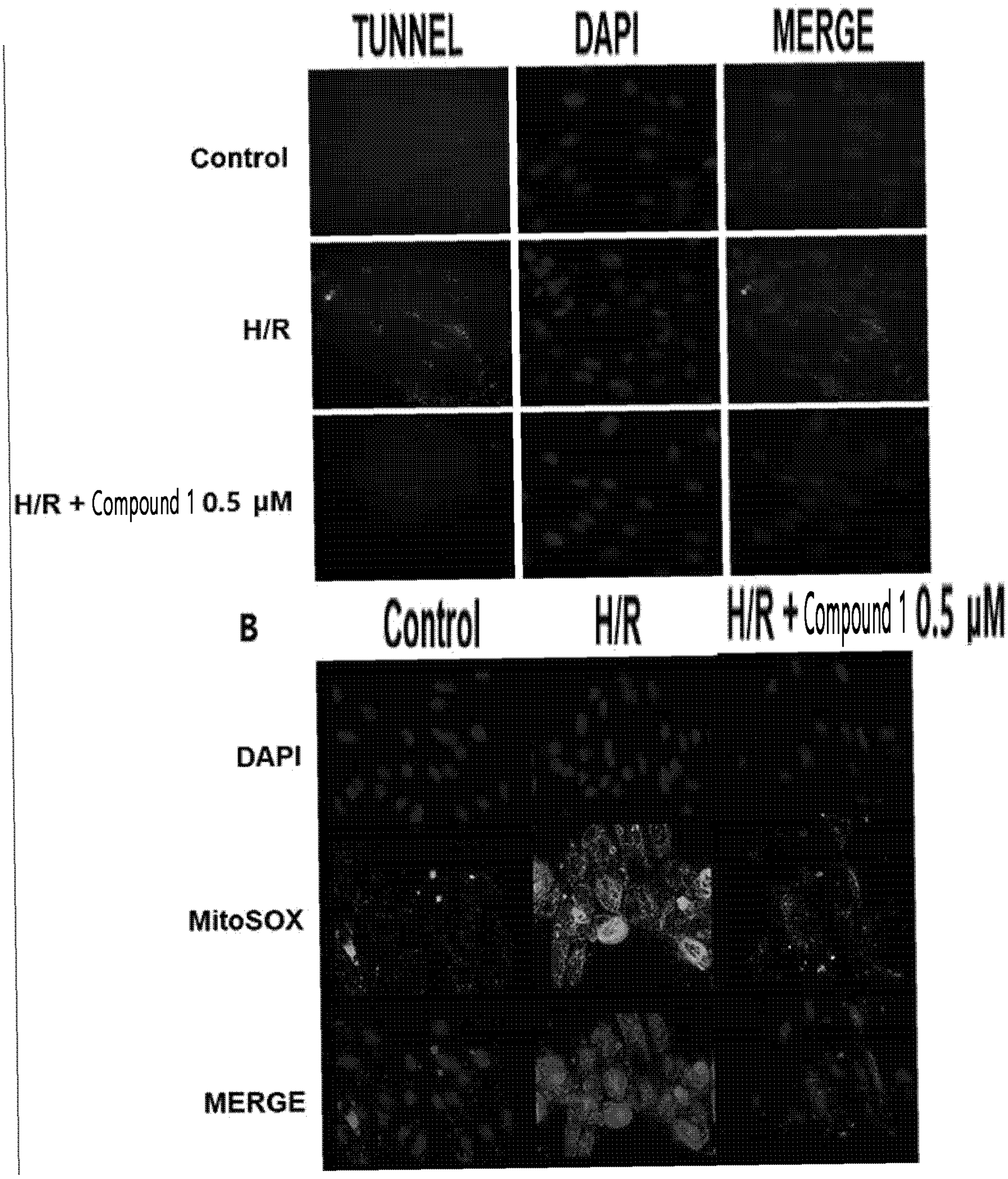
TUNNEL
DAPI
MERGE
Control
H/R
H/R + Compound 1 0.5 μM
B
Control
H/R
H/R + Compound 1 0.5 μM
DAPI
MitoSOX
MERGE

[FIG. 5]

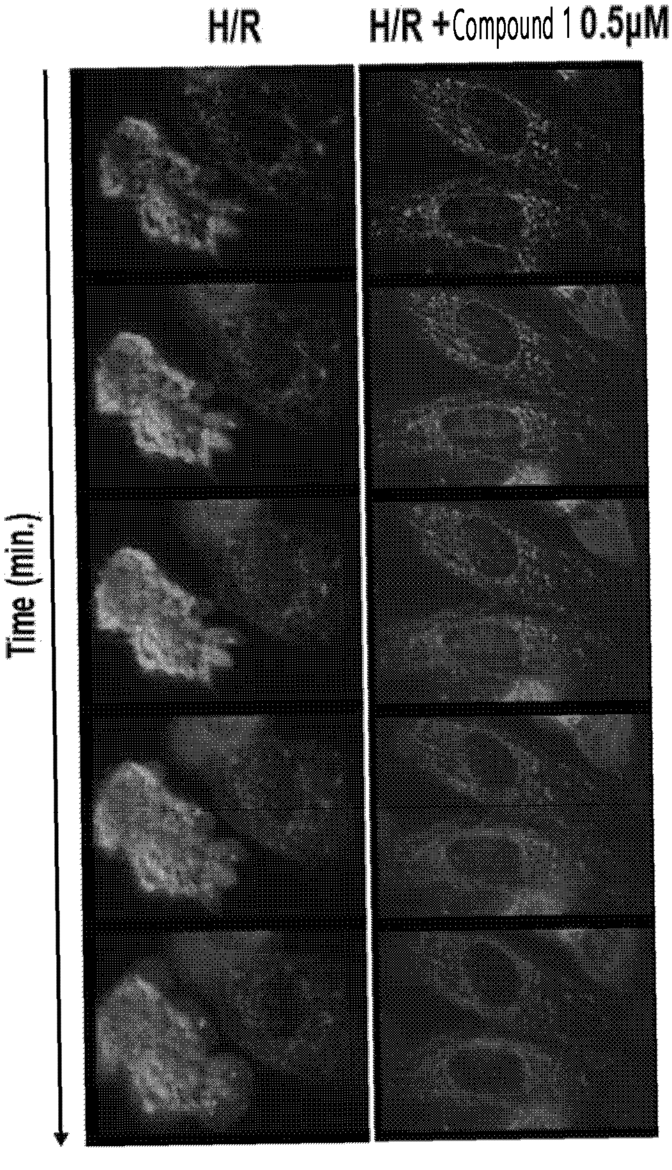

[FIG. 6]

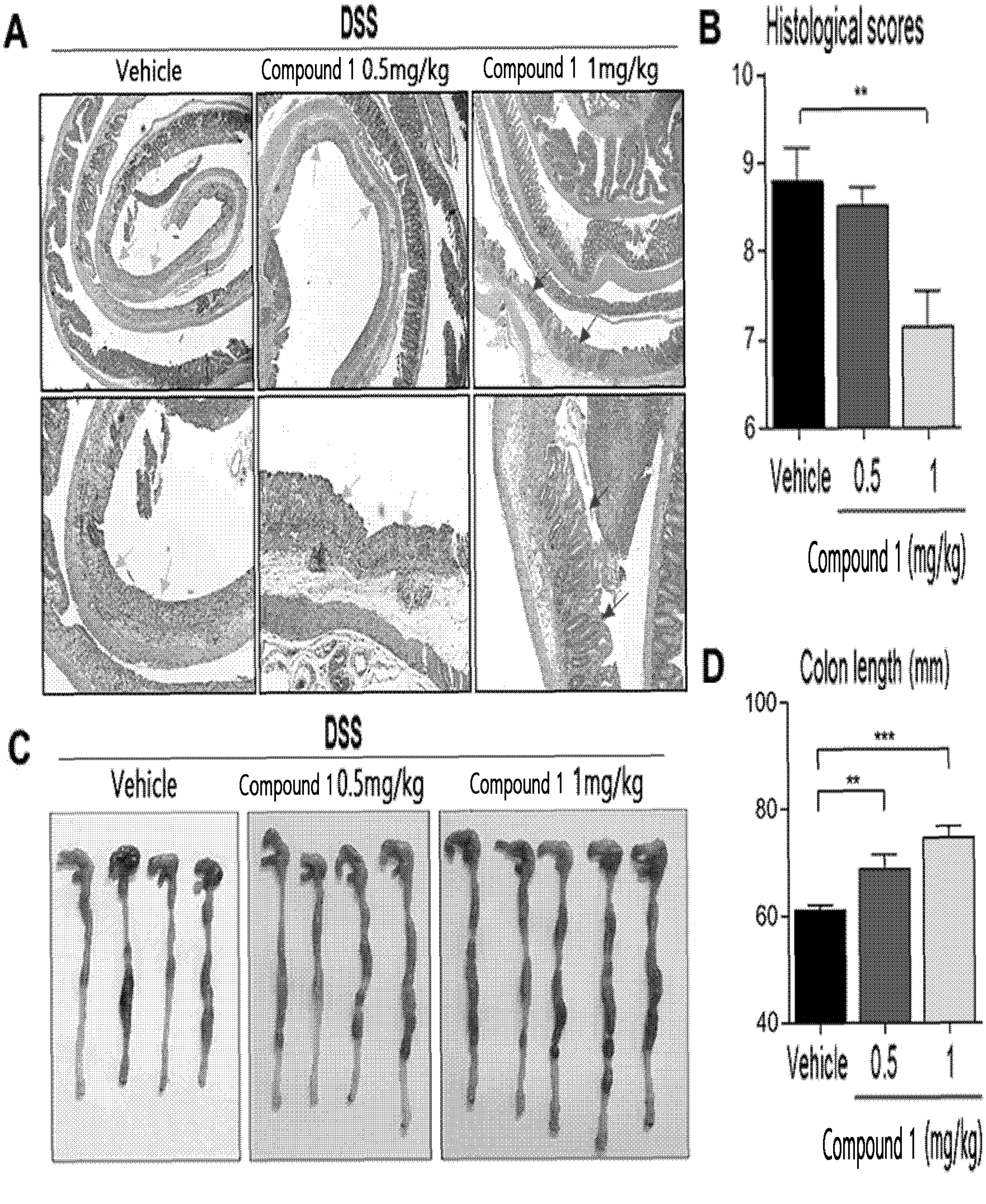
A
DSS
Vehicle
Compound 1 0.5mg/kg
Compound 1 1mg/kg
B
Histological scores
10
9
8
7
6
**
Vehicle
0.5
1
Compound 1 (mg/kg)
C
DSS
Vehicle
Compound 1 0.5mg/kg
Compound 1 1mg/kg
D
Colon length (mm)
100
80
60
40
***
**
Vehicle
0.5
1
Compound 1 (mg/kg)

[FIG. 7]

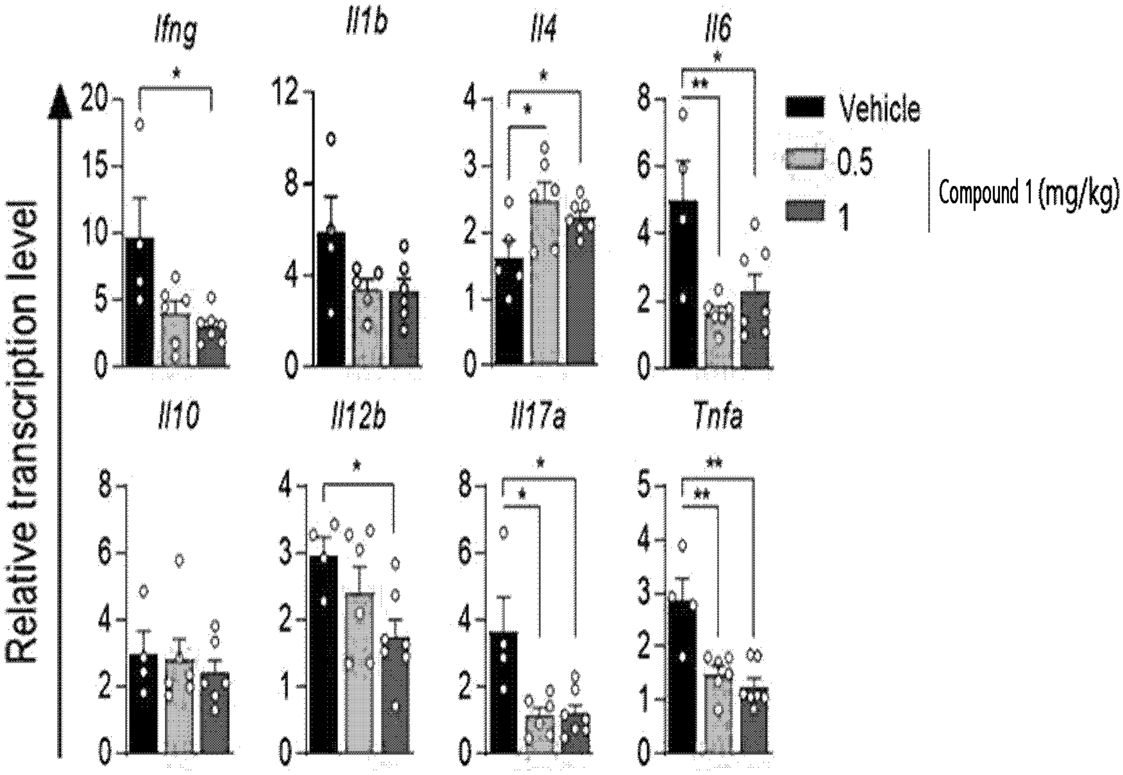
Relative transcription level
Ifng
Il1b
Il4
Il6
Il10
Il12b
Il17a
Tnfa
Vehicle
0.5
1
Compound 1 (mg/kg)

[FIG. 8]

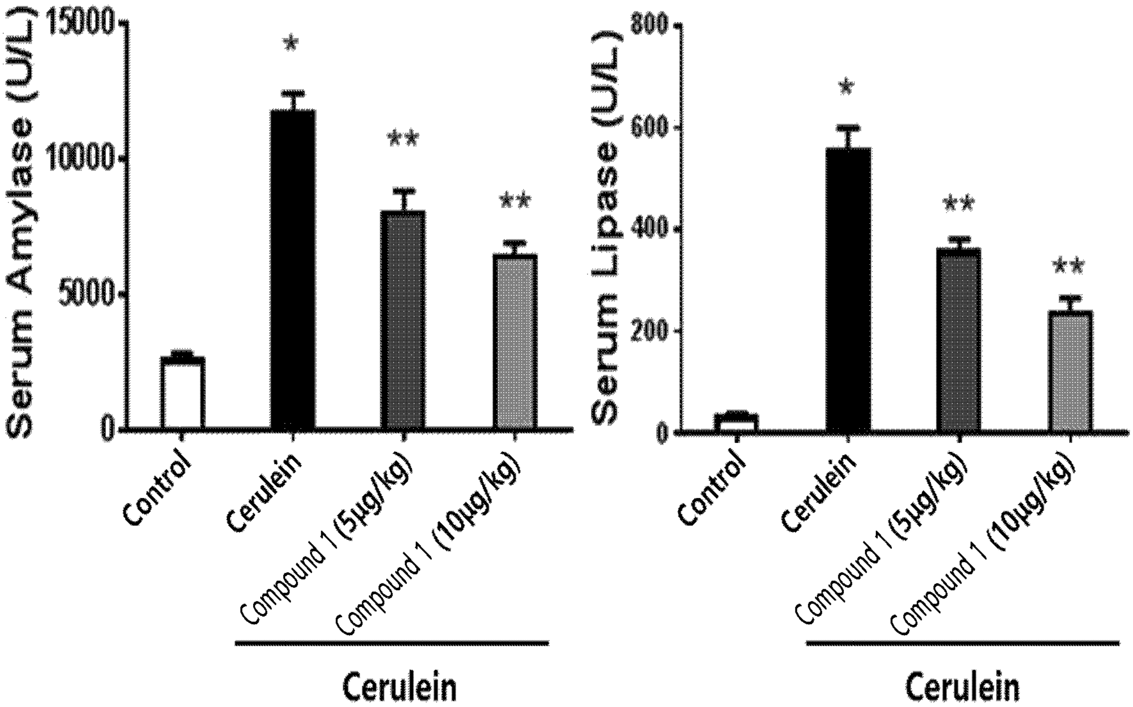
Serum Amylase (U/L)
15000
10000
5000
0
*
**
**
Control
Cerulein
Compound 1 (5μg/kg)
Compound 1 (10μg/kg)
Cerulein
Serum Lipase (U/L)
800
600
400
200
0
*
**
**
Control
Cerulein
Compound 1 (5μg/kg)
Compound 1 (10μg/kg)
Cerulein

[FIG. 9]

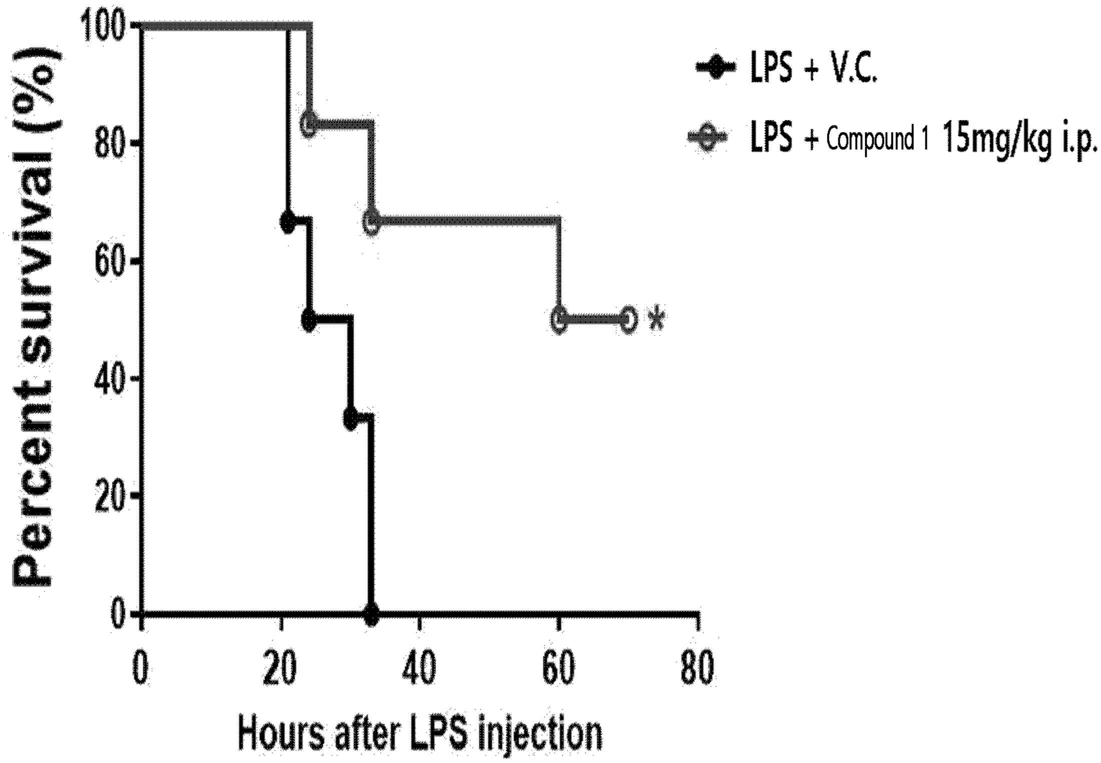
Percent survival (%)
100
80
60
40
20
0
0
20
40
60
80
Hours after LPS injection
LPS + V.C.
LPS + Compound 1 15mg/kg i.p.
*

[FIG. 10]

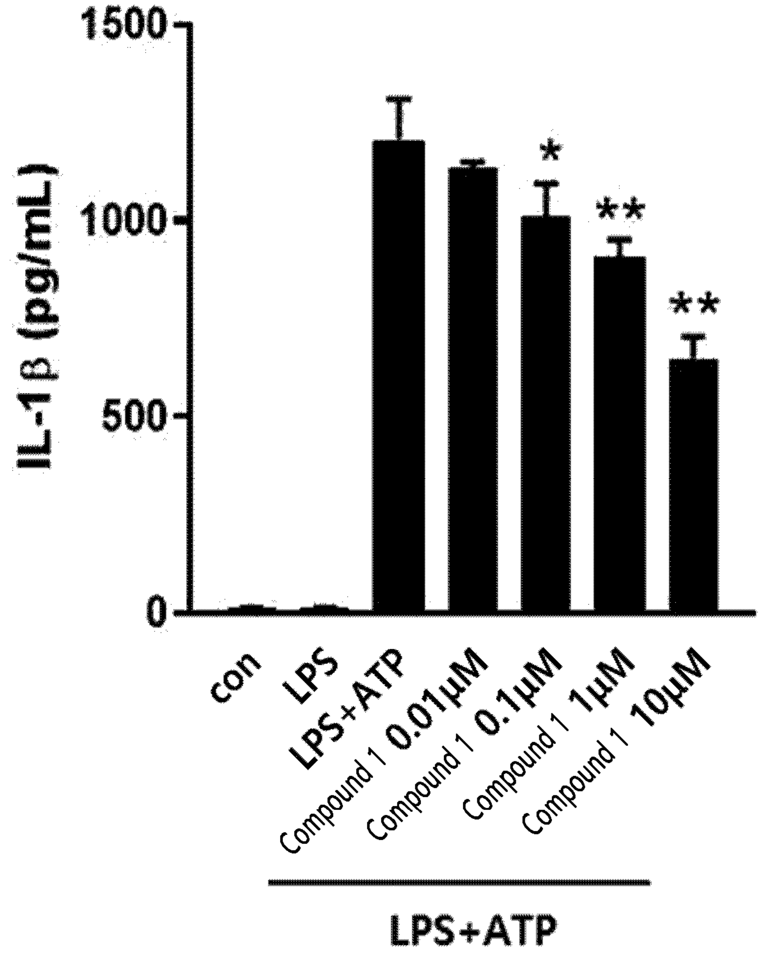
IL-1β (pg/mL)
1500
1000
500
0
*
**
**
con
LPS
LPS+ATP
Compound 1 0.01μM
Compound 1 0.1μM
Compound 1 1μM
Compound 1 10μM
LPS+ATP

[FIG. 11]

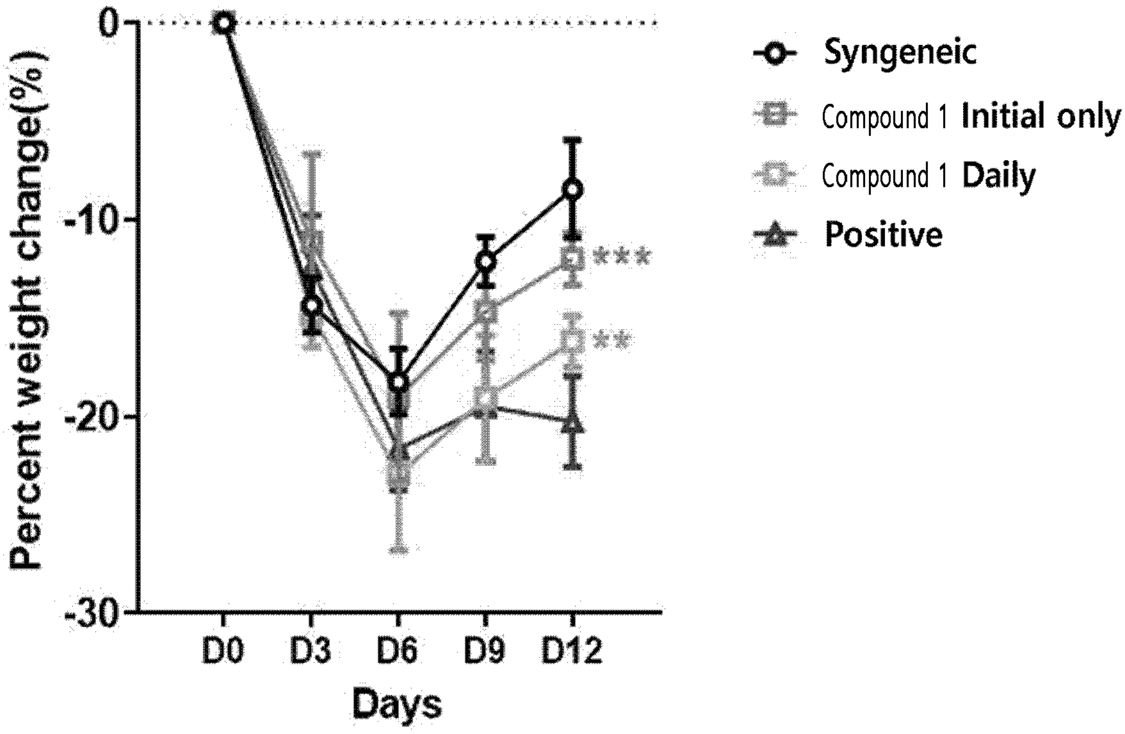
0
-10
-20
-30
Percent weight change(%)
D0
D3
D6
D9
D12
Days
***
**
Syngeneic
Compound 1 Initial only
Compound 1 Daily
Positive

[FIG. 12]

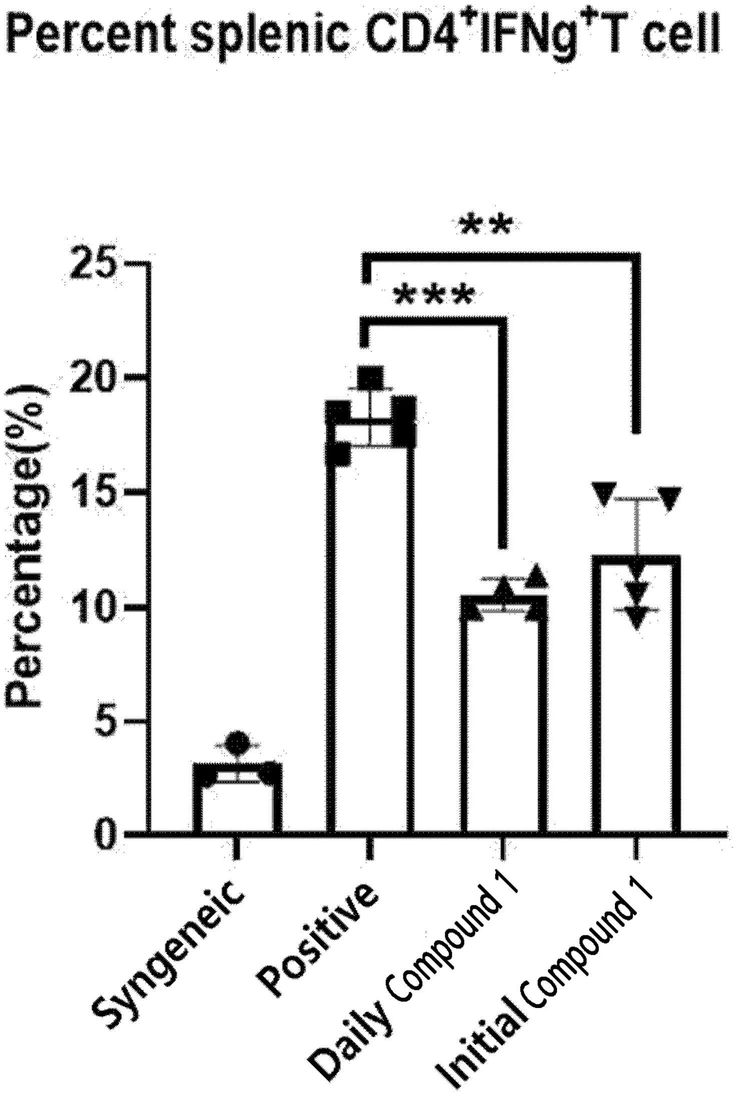
Percent splenic CD4+IFNg+T cell
**
***
25
20
15
10
5
0
Percentage(%)
Syngeneic
Positive
Daily Compound 1
Initial Compound 1

[FIG. 13]

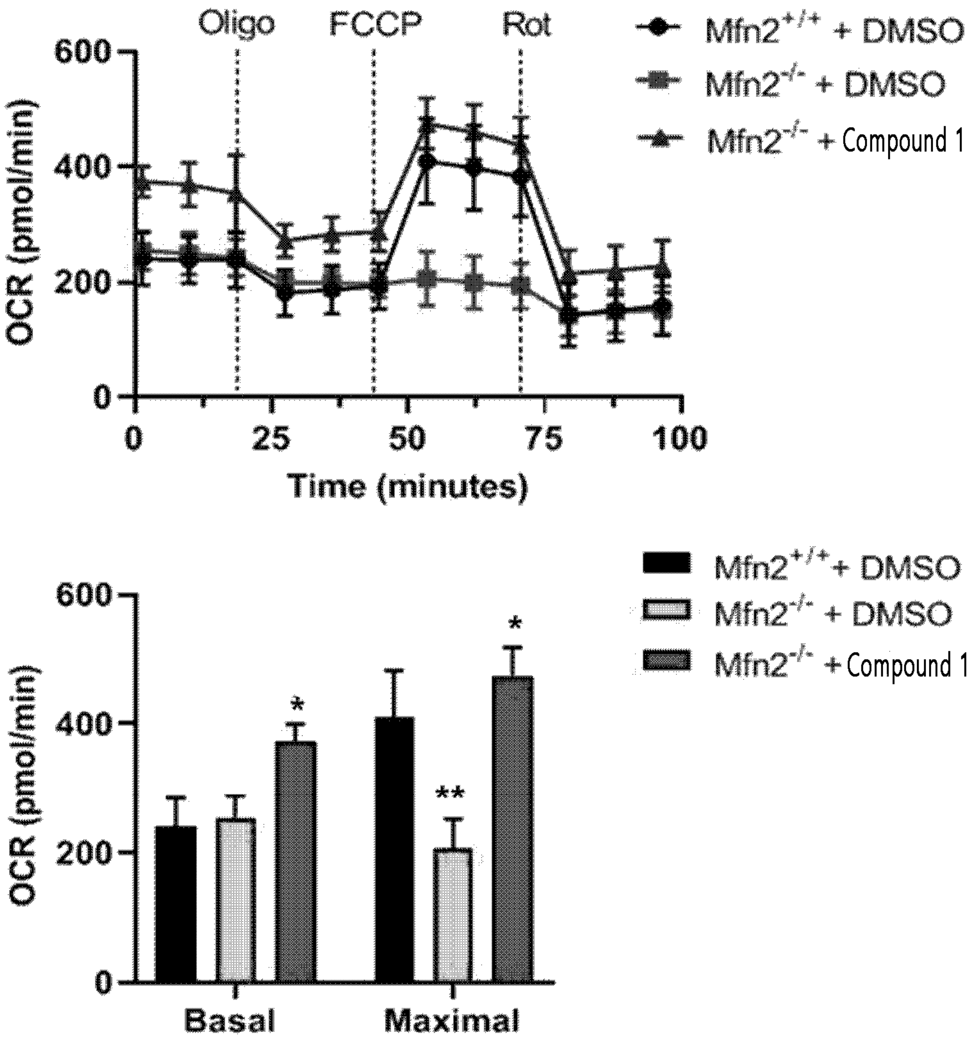
Oligo
FCCP
Rot
Mfn2+/+ + DMSO
Mfn2-/- + DMSO
Mfn2-/- + Compound 1
600
400
200
0
OCR (pmol/min)
0
25
50
75
100
Time (minutes)
Mfn2+/+ + DMSO
Mfn2-/- + DMSO
Mfn2-/- + Compound 1
*
**
*
Basal
Maximal

[FIG. 14]

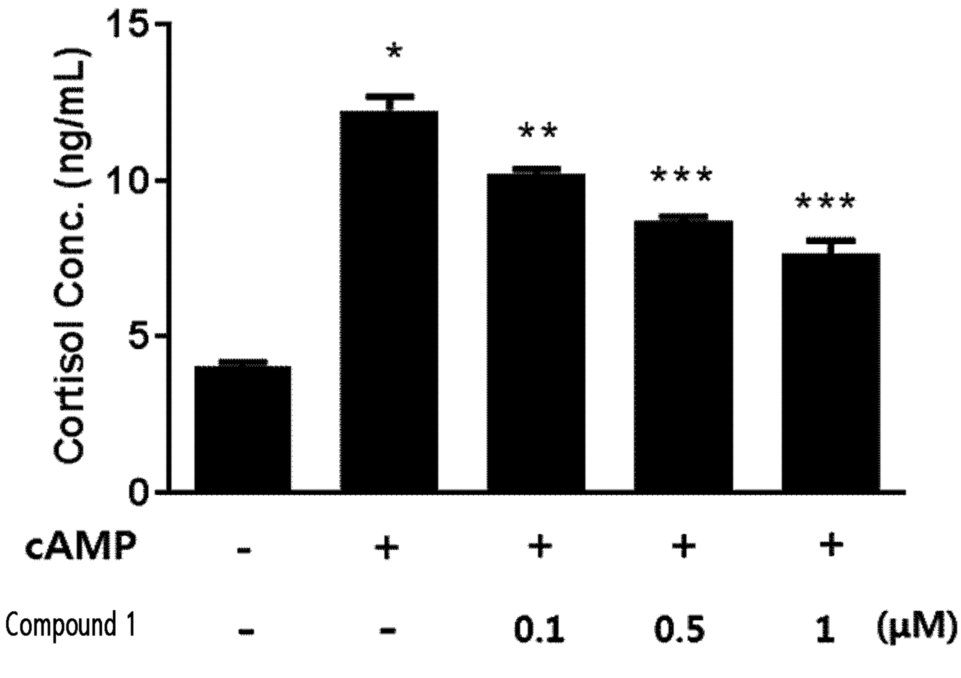

[FIG. 15]

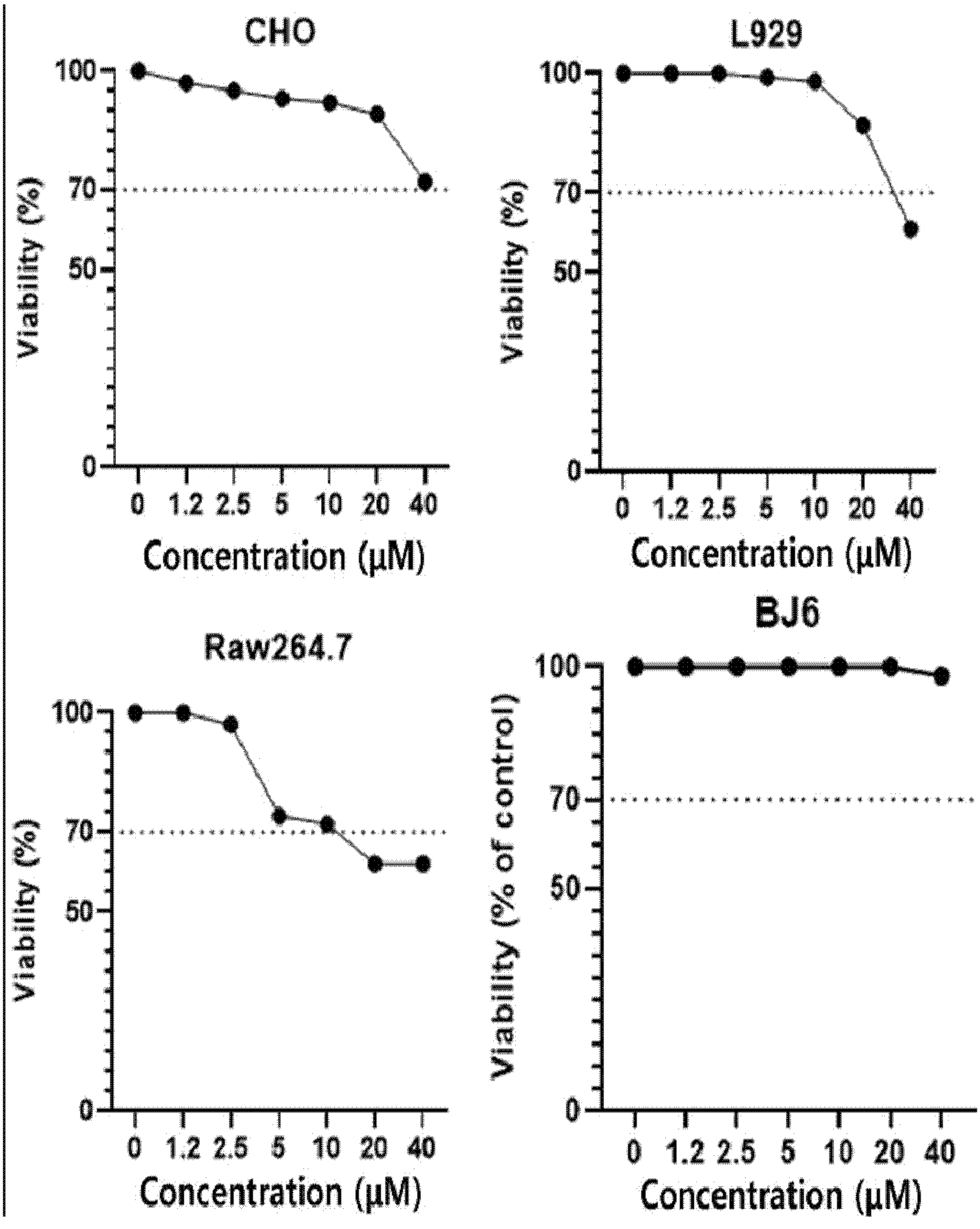
CHO
L929
Raw264.7
BJ6
Viability (%)
Viability (% of control)
100
70
50
0
0 1.2 2.5 5 10 20 40
Concentration (µM)

[FIG. 16]

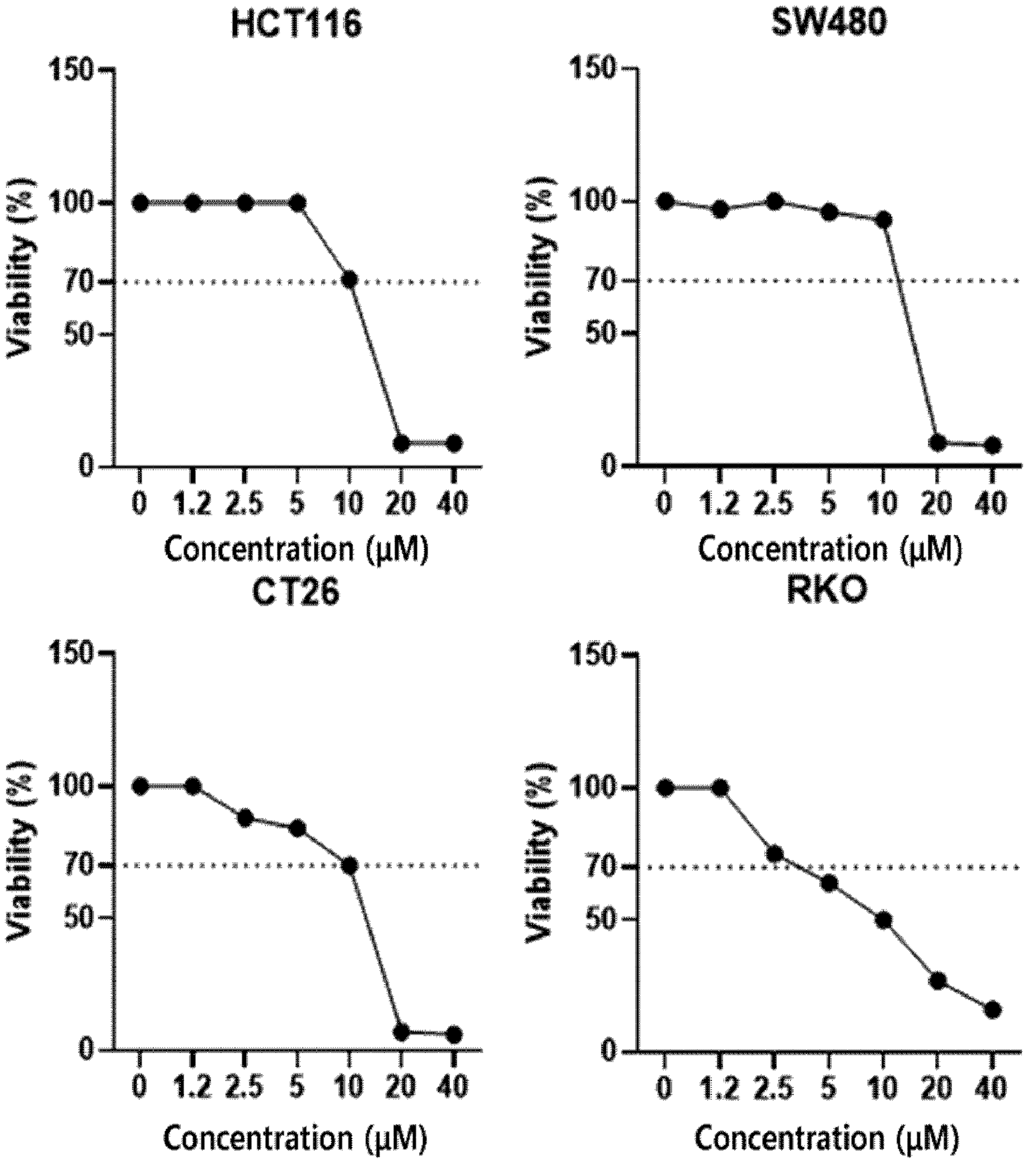
HCT116
SW480
CT26
RKO
Viability (%)
150
100
70
50
0
0 1.2 2.5 5 10 20 40
Concentration (μM)

[FIG. 17]

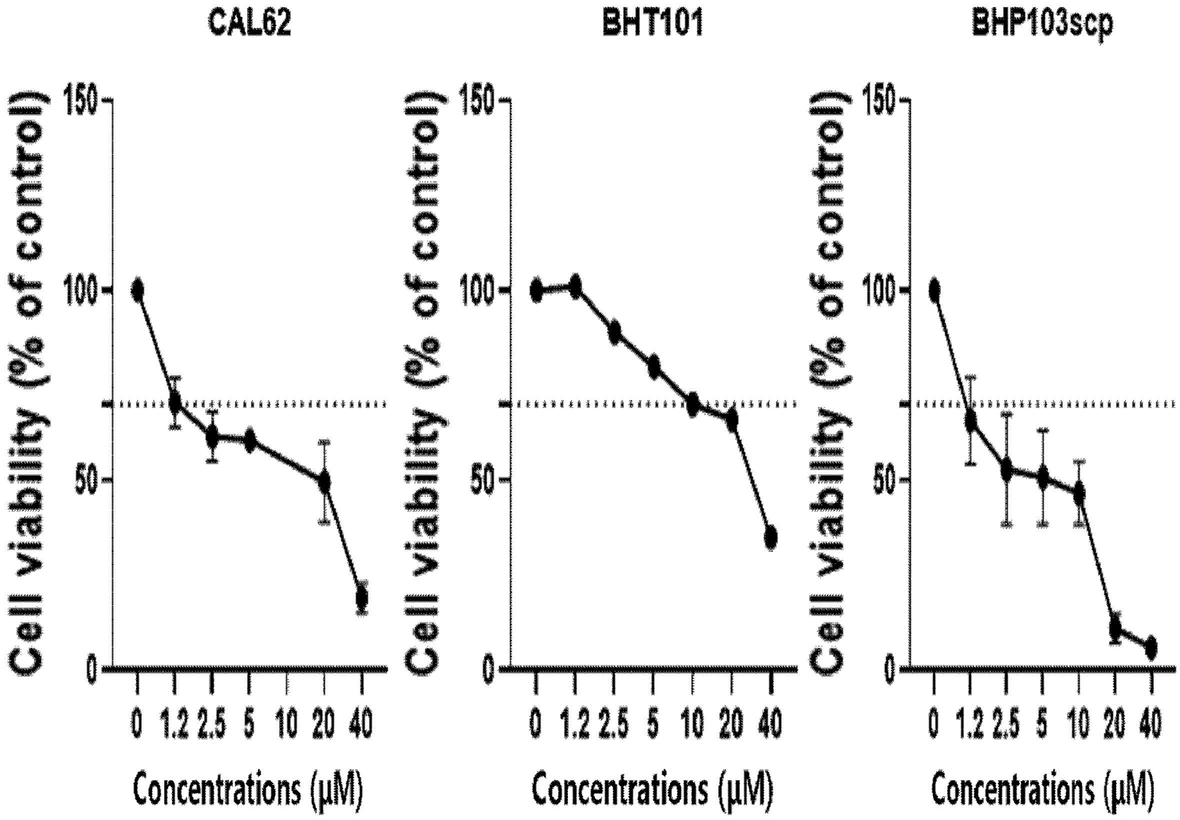
CAL62
BHT101
BHP103scp
Cell viability (% of control)
150
100
50
0
0 1.2 2.5 5 10 20 40
Concentrations (μM)

[FIG. 18]

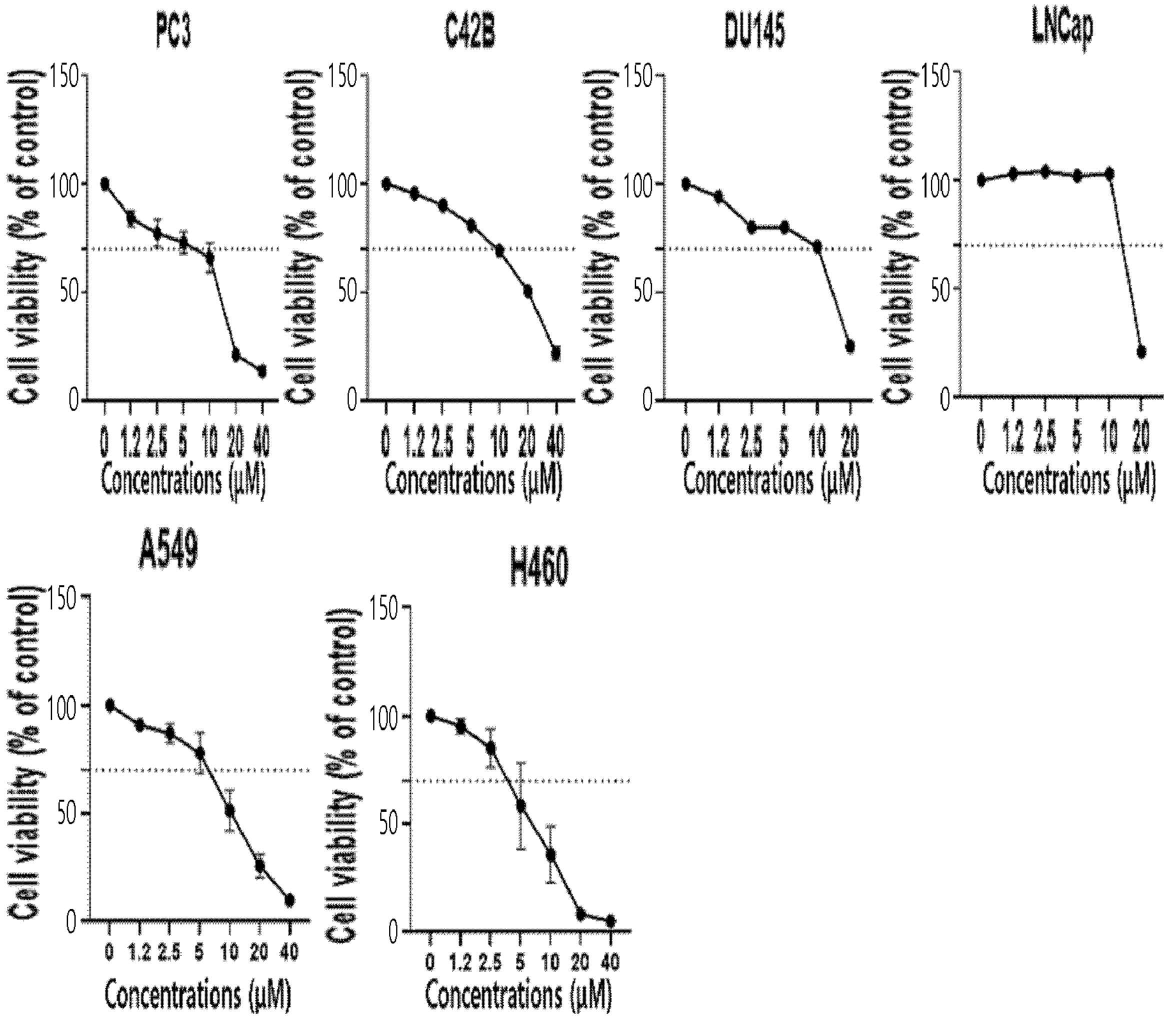
PC3
C42B
DU145
LNCap
A549
H460
Cell viability (% of control)
150
100
50
0
0 1.2 2.5 5 10 20 40
0 1.2 2.5 5 10 20
Concentrations (μM)

[FIG. 19]

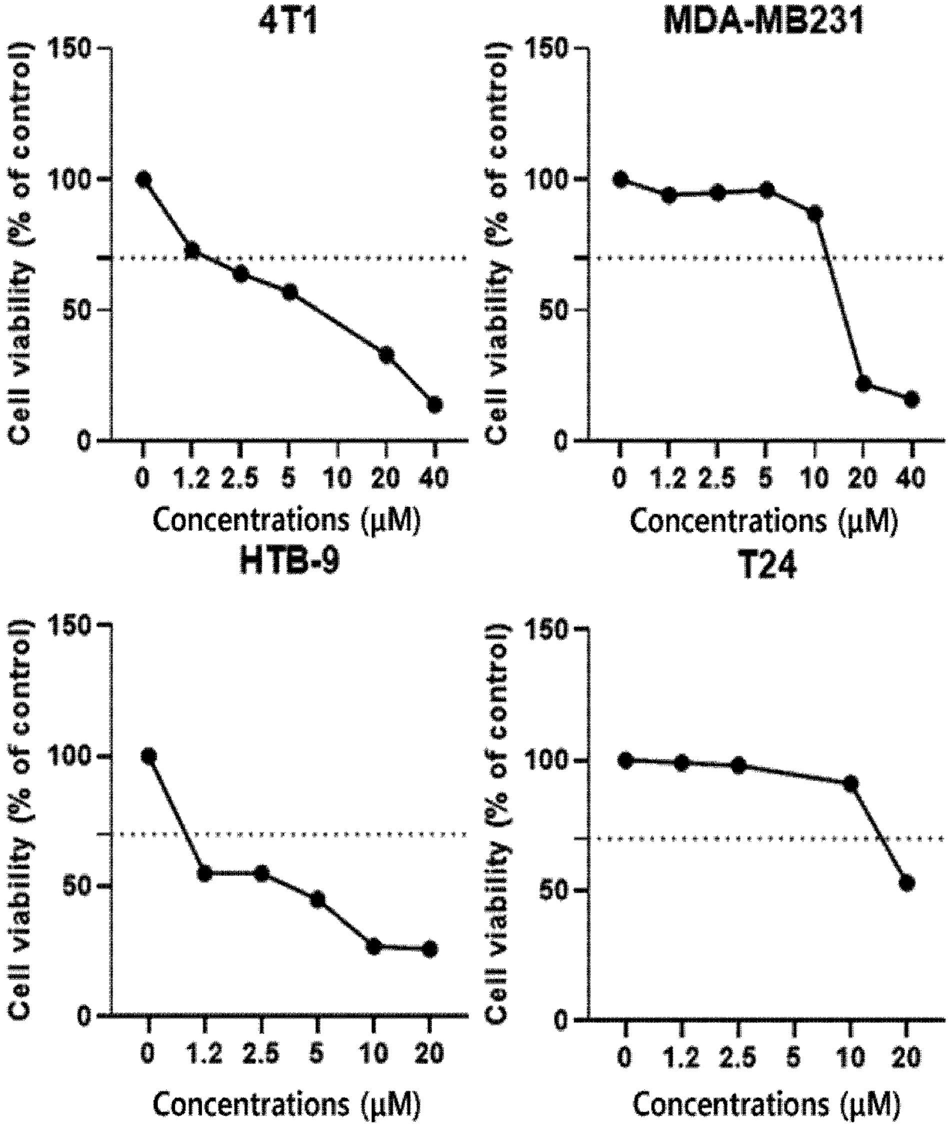
4T1
MDA-MB231
HTB-9
T24
Cell viability (% of control)
150
100
50
0
0 1.2 2.5 5 10 20 40
0 1.2 2.5 5 10 20
Concentrations (µM)

[FIG. 20]

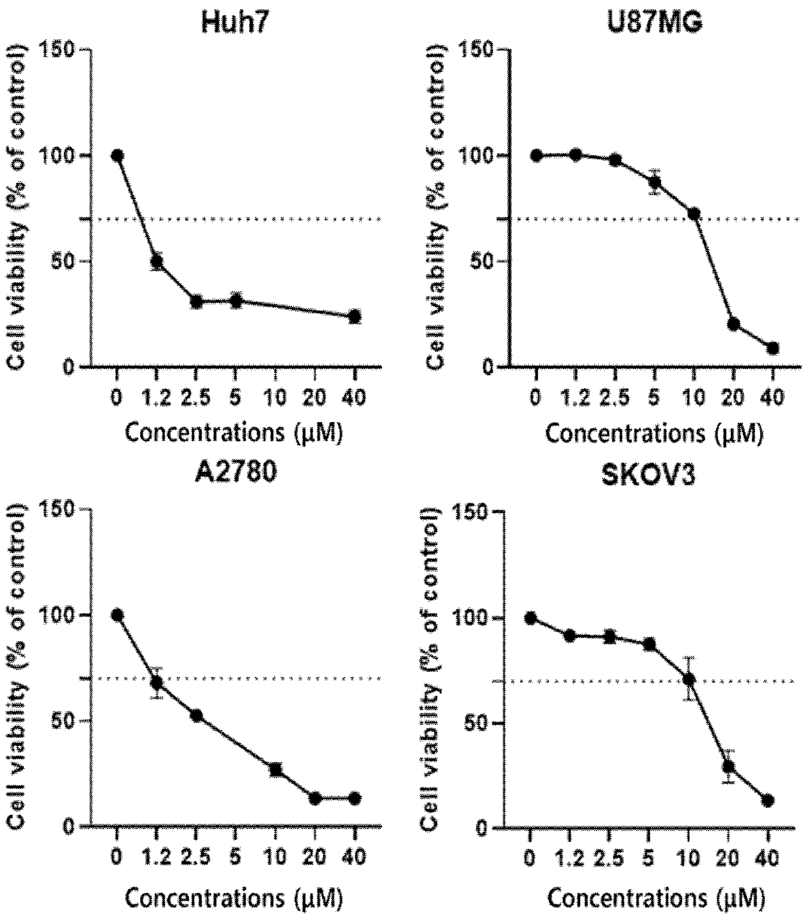
Huh7
U87MG
A2780
SKOV3
Cell viability (% of control)
150
100
50
0
0 1.2 2.5 5 10 20 40
Concentrations (μM)

[FIG. 21]

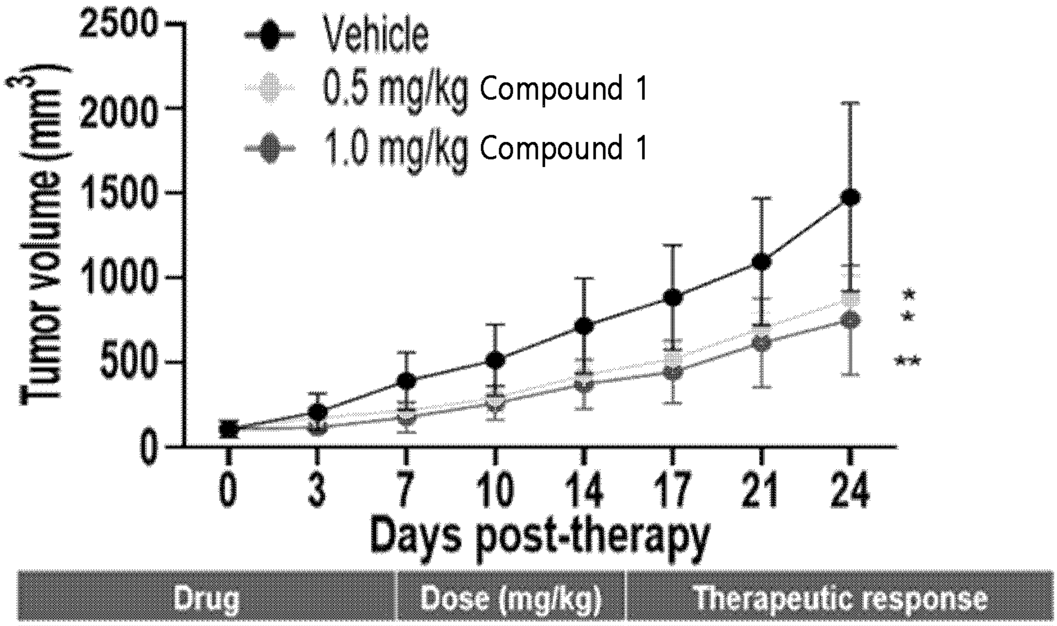


| Drug | Dose (mg/kg) | Therapeutic response |
|---|---|---|
| Vehicle | | |
| Compound 1 | 0.5 | 41% reduction (relative to vehicle) |
| Compound 1 | 1.0 | 49% reduction (relative to vehicle) |

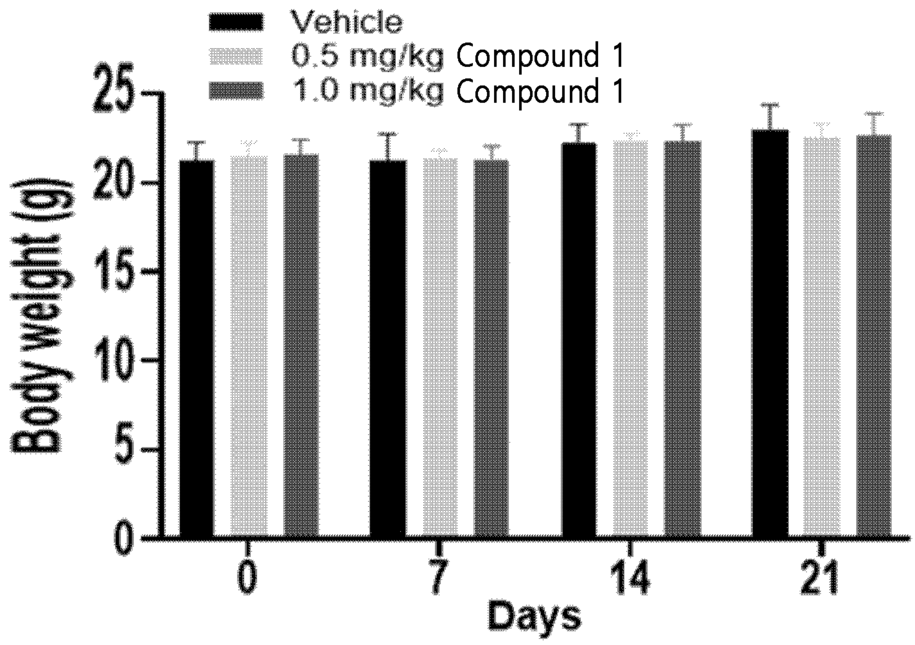

[FIG. 22]

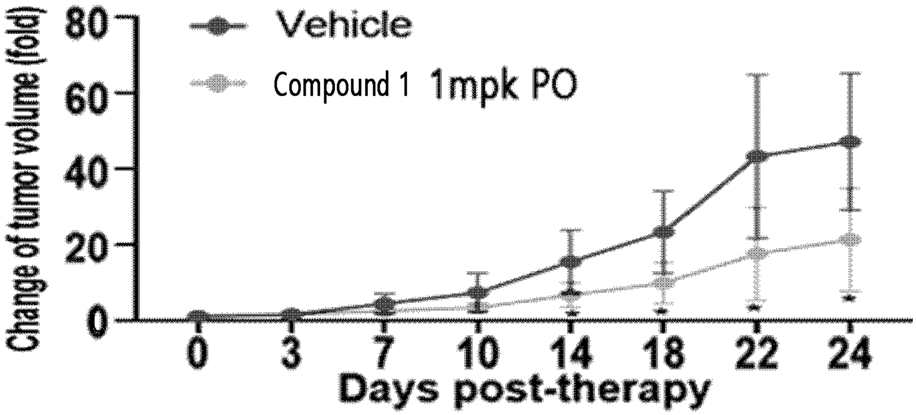


| Drug | Dose (mg/kg) | Therapeutic response |
| --- | --- | --- |
| Vehicle | | |
| Compound 1 | 1.0 | 55% reduction (relative to vehicle) |

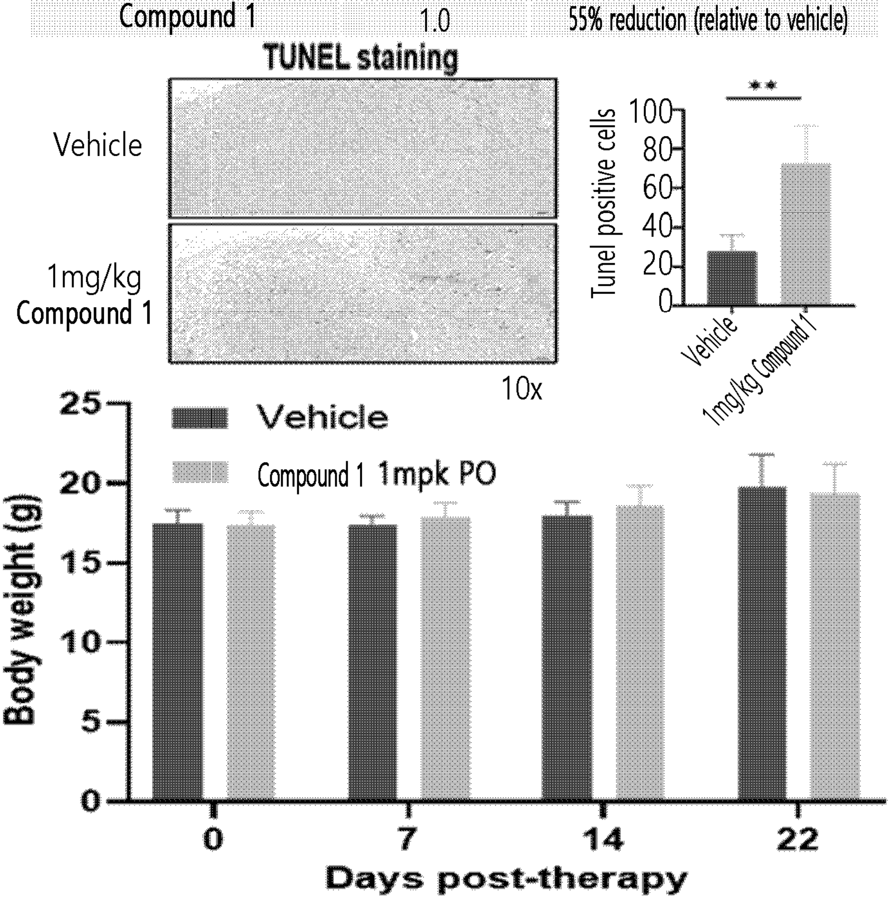

**INTERNATIONAL SEARCH REPORT**

International application No.
**PCT/KR2023/011089**

**A. CLASSIFICATION OF SUBJECT MATTER**

**C07D 231/12**(2006.01)i; **A61K 31/415**(2006.01)i; **A61K 31/4439**(2006.01)i; **A61K 31/497**(2006.01)i; **A61K 31/506**(2006.01)i; **A61K 31/517**(2006.01)i; **A61K 31/4709**(2006.01)i; **A61K 31/5025**(2006.01)i; **A61K 31/427**(2006.01)i; **A61K 31/55**(2006.01)i

According to International Patent Classification (IPC) or to both national classification and IPC

**B. FIELDS SEARCHED**

Minimum documentation searched (classification system followed by classification symbols)

C07D 231/12(2006.01); A61K 31/045(2006.01); A61K 31/415(2006.01); C07C 35/52(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (registry, caplus), Google & keywords: 플루오렌(fluorene), 히드라지닐렌(hydrazynylene), 피루베이트 탈수수효소 키나아제 4(PDK4, pyruvate dehydrogenase kinase 4), 암(cancer), 비만(obesity)

**C. DOCUMENTS CONSIDERED TO BE RELEVANT**

| Category* | Citation of document, with indication, where appropriate, of the relevant passages | Relevant to claim No. |
|---|---|---|
| A | WO 2010-041748 A1 (JAPAN TOBACCO INC.) 15 April 2010 (2010-04-15)<br>See claims 6, 16 and 23. | 1-15 |
| A | WO 2014-142291 A1 (JAPAN TOBACCO INC.) 18 September 2014 (2014-09-18)<br>See claims 1-10. | 1-15 |
| A | WO 2014-142290 A1 (JAPAN TOBACCO INC.) 18 September 2014 (2014-09-18)<br>See claims 1-15. | 1-15 |
| A | WO 2015-002118 A1 (JAPAN TOBACCO INC.) 08 January 2015 (2015-01-08)<br>See claims 1-15. | 1-15 |
| A | WO 2015-002119 A1 (JAPAN TOBACCO INC.) 08 January 2015 (2015-01-08)<br>See claims 1-18. | 1-15 |

☐ Further documents are listed in the continuation of Box C.　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 November 2023** | **21 November 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2023/011089**

| Patent document cited in search report | Publication date (day/month/year) | Patent family member(s) | Publication date (day/month/year) |
|---|---|---|---|
| WO 2010-041748 A1 | 15 April 2010 | AR 074797 A1 | 16 February 2011 |
| | | EP 2345629 A1 | 20 July 2011 |
| | | EP 2345629 A4 | 13 June 2012 |
| | | EP 2345629 B1 | 03 June 2015 |
| | | ES 2543094 T3 | 14 August 2015 |
| | | JP 2011-079801 A | 21 April 2011 |
| | | JP 2014-185159 A | 02 October 2014 |
| | | JP 2016-053055 A | 14 April 2016 |
| | | JP 5550880 B2 | 16 July 2014 |
| | | TW 201018663 A | 16 May 2010 |
| | | US 2010-0240634 A1 | 23 September 2010 |
| | | US 2013-0274240 A1 | 17 October 2013 |
| | | US 2015-0329491 A1 | 19 November 2015 |
| | | US 2019-0177271 A1 | 13 June 2019 |
| | | US 2020-0308109 A1 | 01 October 2020 |
| | | US 8343994 B2 | 01 January 2013 |
| | | US 8871934 B2 | 28 October 2014 |
| WO 2014-142291 A1 | 18 September 2014 | AR 095346 A1 | 07 October 2015 |
| | | JP 2014-198712 A | 23 October 2014 |
| | | TW 201506015 A | 16 February 2015 |
| | | US 2014-0296316 A1 | 02 October 2014 |
| WO 2014-142290 A1 | 18 September 2014 | AR 095345 A1 | 07 October 2015 |
| | | AU 2014-230569 A1 | 08 October 2015 |
| | | AU 2014-230569 A1 | 18 September 2014 |
| | | AU 2014-230569 B2 | 23 November 2017 |
| | | BR 112015022077 A2 | 18 July 2017 |
| | | CA 2904985 A1 | 18 September 2014 |
| | | CA 2904985 C | 20 July 2021 |
| | | CL 2015002608 A1 | 04 March 2016 |
| | | CN 105051015 A | 11 November 2015 |
| | | CN 105051015 B | 25 September 2018 |
| | | CY 1120173 T1 | 12 December 2018 |
| | | DK 2975028 T3 | 12 March 2018 |
| | | EP 2975028 A1 | 20 January 2016 |
| | | EP 2975028 A4 | 17 August 2016 |
| | | EP 2975028 B1 | 21 February 2018 |
| | | EP 3348545 A1 | 18 July 2018 |
| | | EP 3805205 A1 | 14 April 2021 |
| | | ES 2663789 T3 | 17 April 2018 |
| | | HK 1215808 A1 | 15 September 2016 |
| | | HR P20180635 T1 | 01 June 2018 |
| | | HU E036672 T2 | 30 July 2018 |
| | | IL 241355 A | 30 November 2015 |
| | | IL 241355 B | 31 March 2019 |
| | | JP 2015-024984 A | 05 February 2015 |
| | | JP 2018-021061 A | 08 February 2018 |
| | | JP 2018-188449 A | 29 November 2018 |
| | | JP 2019-194197 A | 07 November 2019 |
| | | JP 2020-189855 A | 26 November 2020 |
| | | JP 2022-000453 A | 04 January 2022 |

Form PCT/ISA/210 (patent family annex) (July 2022)

**INTERNATIONAL SEARCH REPORT**
**Information on patent family members**

International application No.
**PCT/KR2023/011089**

| Patent document cited in search report | Publication date (day/month/year) | Patent family member(s) | Publication date (day/month/year) |
|---|---|---|---|
| | | JP 6208603 B2 | 04 October 2017 |
| | | KR 10-2015-0131221 A | 24 November 2015 |
| | | KR 10-2226096 B1 | 09 March 2021 |
| | | LT 2975028 T | 10 April 2018 |
| | | ME 03090 B | 20 January 2019 |
| | | MX 2015012743 A | 19 February 2016 |
| | | MY 182884 A | 05 February 2021 |
| | | NO 2975028 T3 | 21 July 2018 |
| | | NZ 712292 A | 30 October 2020 |
| | | PE 15952015 A1 | 24 November 2015 |
| | | PE 20151595 A1 | 24 November 2015 |
| | | PH 12015501993 A1 | 11 January 2016 |
| | | PH 12015501993 B1 | 11 January 2016 |
| | | PL 2975028 T3 | 31 October 2018 |
| | | PT 2975028 T | 29 March 2018 |
| | | RS 57188 B1 | 31 July 2018 |
| | | RU 2015144182 A | 24 April 2017 |
| | | RU 2015144182 A3 | 06 March 2018 |
| | | RU 2018128304 A | 14 March 2019 |
| | | RU 2018128304 A3 | 15 March 2019 |
| | | RU 2664532 C2 | 20 August 2018 |
| | | SA 515361182 B1 | 01 December 2016 |
| | | SG 11201507327 A | 29 October 2015 |
| | | SI 2975028 T1 | 31 July 2018 |
| | | TW 201501711 A | 16 January 2015 |
| | | TW I633885 B | 01 September 2018 |
| | | US 2014-0296315 A1 | 02 October 2014 |
| | | US 2016-0074364 A1 | 17 March 2016 |
| | | US 2018-0256547 A1 | 13 September 2018 |
| | | US 2020-0163937 A1 | 28 May 2020 |
| | | US 9040717 B2 | 26 May 2015 |
| WO 2015-002118 A1 | 08 January 2015 | JP 2015-028010 A | 12 February 2015 |
| | | TW 201536748 A | 01 October 2015 |
| | | US 2015-0018403 A1 | 15 January 2015 |
| WO 2015-002119 A1 | 08 January 2015 | JP 2015-028009 A | 12 February 2015 |
| | | TW 201536749 A | 01 October 2015 |
| | | US 2015-0025120 A1 | 22 January 2015 |

Form PCT/ISA/210 (patent family annex) (July 2022)